# EUROPEAN PATENT SPECIFICATION

(11) **EP 4 484 563 B1**
(45) Date of publication and mention of the grant of the patent: **05.11.2025**
(21) Application number: 24185827.3
(22) Date of filing: 01.07.2024
(51) Int. Cl.: C12N 15/867, C07K 14/08

(54) **VIRAL VECTOR AND PRODUCER CELL**
VIRALER VEKTOR UND PRODUKTIONSZELLE
VECTEUR VIRAL ET CELLULE PRODUCTRICE

(30) Priority: 30.06.2023 EP 23182836
(43) Date of publication of application: 01.01.2025
(73) Proprietor: EsoBiotec SA, 1435 Mont Saint Guibert (BE)
(72) Inventor: Parone, Philippe, 1435 Mont Saint Guibert (BE); Latere, Jean-Pierre, 1435 Mont Saint Guibert (BE); Doix, Bastien, 1435 Mont Saint Guibert (BE); Galvis, Laura, 1435 Mont Saint Guibert (BE); Webster, Brian, 1435 Mont Saint Guibert (BE); Habib, Joanna Abi, 1435 Mont Saint Guibert (BE); Drappier, Melissa, 1435 Mont Saint Guibert (BE); Fiévet, Alexia, 1435 Mont Saint Guibert (BE); Weerens, Laura, 1435 Mont Saint Guibert (BE); Pecenko, Sylvia, 1435 Mont Saint Guibert (BE)
(74) Representative: Schlich

(56) References cited:
- US-A1- 2015 352 228
- US-A1- 2016 101 174
- US-A1- 2022 333 134
- US-A1- 2023 167 158

## Description

The present invention relates to production and use of viral particles as vectors for genetic modification of specific cell types. The invention also relates to the provision and use of such viral particles for the treatment of disease.

Lentiviruses are a family of retroviruses, which infect by inserting DNA into their host cells' genome. Many such viruses have been the basis of research using viruses in gene therapy, but the lentivirus is unusual in its ability to infect non-dividing cells.

Lentiviral vectors have been used to insert, modify, or delete genes in organisms. In particular, lentiviruses have been used as vectors for gene transfer into target cells, including transduction of CD4⁺, CD8⁺, and/or CD4⁺ regulatory T cells in humans. There are multiple steps involved in the infection and replication of a lentivirus in a host cell. In the first step the virus uses its surface glycoproteins for attachment to the outer surface of a cell. For example, lentiviruses pseudotyped with the vesicular stomatitis virus glycoprotein (VSVG) target a broad range of cell types because of the specificity of VSVG for a receptor (LDLR) which is expressed on the surface of a large number of different cells. Following and targeting and binding of the virus to the target cell, viral material is then injected into the host cell's cytoplasm. Within the cytoplasm, the viral reverse transcriptase enzyme performs reverse transcription of the viral RNA genome to create a viral DNA genome. The viral DNA is then transported into the nucleus of the host cell where it is incorporated into the host cell's genome with the help of the viral enzyme integrase. At this point the systems of the host cell are employed to transcribe the entire viral RNA and express the structural viral proteins, in particular those that form the viral capsid and the envelope. The lentiviral RNA and the viral proteins then assemble, and the newly formed virions leave the host cell when enough are made by budding off or "blebbing" from the exterior membrane of the infected cell.

*Ex vivo* and *in vivo* methods of using lentiviruses for genetic modification have been considered. In the ex *vivo* methodology, cells are extracted from a patient and then cultured. A lentiviral vector carrying a therapeutic transgene is then introduced to the culture to infect the cells and consequently modifies their genome. The modified cells continue to be cultured until they can be infused into a patient. The cells used for *ex vivo* methods may be autologous, i.e. the cells are taken from and returned to the same individual, or heterologous, i.e. the cells are taken from one individual and returned to a different individual.

In contrast, *in vivo* gene therapy involves the injection of viral vectors, such as lentivirus, containing transgenes, into the patient and the consequent modification of the genome.

Cellular immunotherapy for cancer is conceived of as a method of harnessing the immune system of a cancer patient in order to induce their own immune system to attack cancer cells in the patient and thus push the cancer into remission.

One known form of cellular immunotherapy is the production of CAR T cells. Chimeric antigen receptors (CARs) (also known as chimeric immunoreceptors, chimeric T cell receptors or artificial T cell receptors) are receptor proteins that have been engineered to bind specifically to a target antigen. Thus, when the CAR protein is expressed in a T cell that is genetically modified to express the CAR, the T cell targets a specific target antigen. The receptors are chimeric in that they combine both antigen-binding and T cell activating functions into a single receptor.

Thus, CAR T cell therapy uses T cells engineered with CARs to treat cancer by defining the target as a particular antigen displayed by a cancer cell. In this way an individual's immune system can be given the ability to effectively target and destroy cancer cells within the individual.

After CAR T cells are infused into a subject (or patient) they contact their target antigen on a cell's surface. The CAR T cells bind to the target and the T cell comes activated, triggering it to proliferate and become cytotoxic. CAR T cells destroy cells through several mechanisms, including extensive stimulated cell proliferation, increasing the degree to which they are toxic to other living cells (cytotoxicity), and by causing the increased secretion of factors that can affect other cells, such as cytokines, interleukins, and growth factors.

CAR T cells can be derived either autologously, i.e. from a patient's own T cells, or allogeneically, i.e. from the T cells of another, healthy, donor. Once isolated the T cells are genetically engineered to express a specific CAR, which programs them to target an antigen that is present on the surface of a cancer cells, e.g. a tumour. For safety, CAR T cells are typically engineered to be specific to an antigen that is expressed by the cancer but is not expressed on healthy cells.

Necessarily, cellular therapies, such as CAR T cell therapies, require a complex supply chain of reagents and time-consuming and expensive processing in order to yield the pharmaceutical product. In addition to the complexity and difficulty of manufacturing cell-based therapies, there is the difficulty of obtaining the cells that are the base material for providing the therapeutic product. Autologous cells are used because of the lower risk of patient side-effects, but not all patients are able to give sufficient autologous cells (such as T cells) in order to provide a useful therapy. The use of allogeneic cells (such as T cells) has a greater risk of side-effects.

Thus, there is a need for cell-based therapies with reduced costs of preparation and simplified logistics for administration. There is also a need for cell-based therapies that are effective against a wider variety of types of cancer.

The invention provides a retroviral vector having a lipid bilayer envelope comprising:
(a) a single-domain antibody (sdAb) binding domain displayed on the exterior of the envelope that is cell-type specific;
(b) a Lassa virus envelope protein displayed on the exterior of the envelope that is able to facilitate infection of the same cell type as in (a); and
(c) a nucleic acid molecule comprising a promoter expressible in the same cell type as in (a).

The lipid bilayer (or phospholipid bilayer) is a thin polar membrane made of two layers of lipid molecules. These membranes are flat sheets that form a continuous barrier around all cells. The cell membranes of almost all organisms and many viruses are made of a lipid bilayer. Thus, the viral vector described herein may be based on an enveloped virus.

A single-domain antibody (sdAb or VHH), is polypeptide or antibody fragment consisting of a single monomeric variable antibody domain. Like an antibody comprised of associated polypeptide chains, it is able to bind selectively to a specific antigen. With a molecular weight of only 12-15 kDa, single-domain antibodies are much smaller (typical molecular weight of 12-15 kDa) than common antibodies (150-160 kDa) which are composed of two heavy protein chains and two light chains, and even smaller than Fab fragments (~50 kDa, one light chain and half a heavy chain) and single-chain variable fragments (~25 kDa, two variable domains, one from a light and one from a heavy chain).

The first single-domain antibodies were engineered from heavy-chain antibodies found in camelids; these are called V_{H}H (VHH) fragments. Cartilaginous fishes also have heavy-chain antibodies (IgNAR, "immunoglobulin new antigen receptor"), from which single-domain antibodies called V_{NAR} fragments can be obtained. An alternative approach for producing a single-domain antibody is to split the dimeric variable domains from common immunoglobulin G (IgG) from humans or mice into monomers.

Nanobodies are preferred over other antigen binders, e.g. scFvs, due to their typically higher solubility, stability, and smaller size. The smaller size of nanobodies allows them to access and lodge onto conventionally inaccessible regions on therapeutic targets. In addition, nanobodies are less likely to induce an unwanted allergic response in an individual.

The VHH, and the binding domain thereof, may be bound, through a linker, to a domain that attaches it to or anchors it in the lipid bilayer envelope. Thus, the VHH may be a membrane-anchored VHH. Preferably the anchoring domain is a transmembrane polypeptide domain. Most preferably, the VHH and anchoring domain are separate domains of a single fusion protein.

Advantageously the linker moiety between the VHH, and the binding domain thereof, and the domain that attaches it to or anchors it in the lipid bilayer envelope may modulate transduction efficiency. In particular use of the PDGFR/CD8a hinge scaffold to present the VHH may yield greater transduction efficiency. Furthermore, increased CD69, CD25 and/or PD1 expression, and/or increased cell expansion and/or functional viral titres (titres) may be achieved. These advantages may be considered in comparison to the IgG4 linker for the same VHH. Without wishing to be bound by theory, this technical effect may flow from the linker providing better physical flexibility thus improving or optimising binding to the target and/or achieving an improved distance between the two cells to improve transduction.

The present invention has the advantage that the amount (proportion) of VHH displayed on the exterior of the envelope can be controlled with respect to the amount (proportion) of viral envelope protein also displayed on the exterior of the envelope. The ability to control the amount or proportion of these two displayed moieties means that their proportions and scaffold (as is the case for the targeting VHH) can be optimised, and this yields greater transduction efficiency. It is thus preferred for the antibody domain, acting as a targeting moiety, and the envelope protein to be separately displayed by the viral vector, i.e., no fusion / conjugation of the targeting moiety and the envelope protein should occur. Similarly, it is preferred for the antibody domain, acting as a targeting moiety, and the envelope protein to be separately expressed by the vector-producing cell, i.e., no fusion / conjugation of the targeting moiety and the envelope protein should occur. Without wishing to be bound by theory, during the process of viral entry envelope proteins undergo very specific conformational changes. Accordingly, fusion of a VHH to a viral envelope protein is likely to disrupt the viral entry function of the ENV protein. In addition, separating the VHH and envelope proteins provides the ability to optimize the VHH scaffold without restrictions associated with preserving the viral entry function of the ENV protein.

Thus, the viral vectors of the present invention have the advantage of being cell-type specific. In the context of the present invention "cell-type specific" may mean one particular cell type or a group of related cell types, e.g. immune cells, lymphocytes, tumour-infiltrating lymphocytes (TILs), monocytes, macrophages, T cells or NK cells.

The viral envelope protein is a Lassa virus envelope protein. The viral envelope protein may be Lassa Virus GP1+2. The viral envelope protein may be Lassa Virus GP with tyrosine to alanine substitution Y150A.

Use of antibody-domain targeted lentiviral vectors pseudotyped with the mutant Lassa Virus glycoprotein Y150A (LVGP1x) advantageously enables the targeting of the engineered vectors to specific cell types expressing a target of the antibody domain. Similarly, H141A F147A or Y150A H141A F147A Lassa virus (LVGP3X) glycoprotein enables the specific targeting of the engineered vectors to cells expressing the VHH targets.

The antibody domain or VHH may target (bind to) CD8, CD3 or the T-cell receptor (TCR), CD14 or CCR2. Preferably the VHH targets (binds to) the TCR. Accordingly, the specific targeting of T cells is preferably to cells expressing TCR or CD3 or CD4 or CD8, most preferably the specific targeting is preferably to cells expressing TCR. Preferably, the antibody binding domain or VHH binds the TCR. Preferably the antibody domain or VHH targets (binds to) CD3, most preferably the antibody domain or VHH targets CD3ε (CD3e).

The antibody domain or VHH may target (bind to) CD14 or CCR2. Preferably the VHH targets (binds to) CD14. Accordingly, the specific targeting of myeloid cells is preferably to cells expressing CD14 or CCR2, most preferably the specific targeting is preferably to cells expressing CD14. Preferably, the antibody binding domain or VHH binds the CD14.

By way of example, Lassa virus is advantageous for use in the context of the present invention because only a small proportion of the general population is seropositive for Lassa virus. In addition, a low titre (titer) of neutralising antibodies is produced following Lassa Virus infection in the affected population.

The viral envelope may comprise a cell-cell fusogen, which is preferably a glycoprotein. In the present context cell-cell fusogens are considered to be proteins that facilitate the fusion of cell-to-cell membranes, in particular cell-cell fusogens are proteins that promote plasma membrane fusion among different cells. Most preferably the fusogen is of viral origin, but may be of non-viral origin.

The promoter is preferably cell-type specific. The promoter may be cell-type specific for a cell having one or more of the phenotypes of TCRab, TCRgd, CD3, CD25, CD4, CD8, CD11, CD14, CD16, CCR1, CCR2, CXCR4, CD56, CD20 and/or CD64 [Fc gamma receptor I (FcγRI)], preferably CD3 or CD14, CD56 or CD20. The promoter may be cell-type specific for a cell expressing one or more of CD3, CD25, CD4, CD8, CD14, CD16, CCR1, CCR2, CXCR4, CD56, CD20 and or CD64 [Fc gamma receptor I (FcγRI)], CD56, CD19, CD20, preferably CD3, CD14, CD56, or CD20.

The promoter may be cell-type specific for a cell having the phenotype of CD3, CD25, CD4, CD8, CD11 CD14, CD16, CCR1, CCR2, CXCR4, CD56, CD20 and or CD64 [Fc gamma receptor I (FcγRI)], CD56, CD19, CD20, preferably CD3, CD14, CD56, or CD20.

The promoter may be cell-type specific for a cell expressing CD3, CD25, CD4, CD8, CD11, CD14, CD16, CCR1, CCR2, CXCR4, CD56, CD20 and or CD64 [Fc gamma receptor I (FcγRI)], CD56, CD19, CD20, preferably CD3, CD11, CD14, CD56, or CD20.

The present invention also has an advantage that use of a target-cell specific promoter, which is therefore implicitly not active in the cell that is producing the viral vector particles, means that the producer cell does not have to accommodate the transcriptional and translational load of expressing the "payload" of the viral vector particle. For example, if the "payload" is a chimeric antigen receptor (CAR), then the CAR is not expressed by the producer cell but in the target cell. This has the advantage that use of a cell-specific promoter yields a greater titre of vectors yielded by a producer cell in which the cell-specific promoter is not active. Merely by way of example, advantageously, restricting the expression of surface expressed proteins and/or protein complexes during production of the viral vector particles increases the number of transduction competent lentiviral particles per millilitre (ml; milliter) of product. Preferably this increase is fivefold or greater, tenfold, or greater. Most preferably this increase is fivefold or greater.

The nucleic acid molecule may comprise an open reading frame expressible from the promoter. A promoter is a region of the nucleotide sequence of a gene where transcription of a gene is initiated. The promoter may be a synthetic promoter comprising promoter and enhancer element(s), preferably the promoter and enhancer element(s) are not normally associated with one another.

In the context of the present disclosure an enhancer is a region of the nucleotide sequence that modulates the activity of the promoter. Preferably the presence of the enhancer and/or its interaction with the promoter causes the activity of the synthetic promoter to be specific for a particular cell type or subset of cell types. The open reading frame may encode a single or multiple polypeptides. These polypeptides may include engineered receptors, chimeric antigen receptor (CAR), chimeric auto antibody receptor (CAAR), engineered T cell receptors, engineered B cell receptors, universal CAR receptor, cytokines, growth factors, suicide genes, antibody fragments, enzymes, metabolic factors, switch receptors. In some embodiments the viral genome may also encode for regulatory RNA elements which include interfering RNAs, small hairpin RNAs, microRNAs, and/or binding sites for microRNAs.

The nucleic acid molecule may comprise the promoter and the enhancer element on the same nucleic acid molecule.

The addition of enhancer sequences yields the advantage of increasing expression from the synthetic promoters. Accordingly, synthetic constructs may reach and/or exceed the levels of expression obtained from the ubiquitous promoter EF1a. T-cell specific synthetic promoters may have limited expression in non-T cell lineage cell lines, preferably as compared to expression from the ubiquitous promoter EF1a (human elongation factor 1 alpha) or PGK (promoter from mammalian phosphoglycerate kinase).

Preferably, the promoter is expressed only in a T cell.

Preferably, the promoter is expressed only in a monocyte or a macrophage or a tumor-associated myeloid cell.

Preferably, the promoter is expressed only in a natural killer (NK) cell.

Preferably, the promoter is expressed only in a B cell.

The synthetic promoter may comprise one, two or more enhancer elements, preferably two enhancer elements.

The synthetic promoter may comprise one or more cell specific microRNA elements. Preferably the microRNA elements may have a sequence selected from SEQ ID NO 128 or 129.

The promoter element for specific expression in T cells may comprise a sequence selected from any one of SEQ ID NOs 11 to 20 or 121 to 127. The promoter may have a sequence selected from any one of SEQ ID Nos 11 to 20 or 121 to 127.

Preferably, the viral vector encodes a payload under the control of a T cell specific promoter that comprises or has the sequence of any of SEQ ID NOs 12 to 15.

Preferably, the viral vector encodes a payload under the control of a promoter that comprises or has the sequence of SEQ ID NO. 15.

The promoter element for specific expression in myeloid cells may comprise a sequence selected from any one of SEQ ID NOs 130 to 152 and SEQ ID NO 193. The promoter may have a sequence selected from any one of SEQ ID Nos 130 to 152, 193 and SEQ ID NO 197.

Preferably, the viral vector encodes a payload under the control of a promoter that comprises or has the sequence of SEQ ID NO. 138 or 139 or 193.

The promoter element for specific expression in tumor associated myeloid cells may comprise a sequence selected from any one of SEQ ID NOs 141 to 152. The promoter may have a sequence selected from any one of SEQ ID Nos 141 to 152 and SEQ ID NO 193.

Preferably, the viral vector encodes a payload under the control of a promoter that comprises or has the sequence of SEQ ID NO. 143, 146 or 152.

The viral vector is a retroviral vector, preferably a lentiviral vector.

The viral vector bioavailability may be increased by reducing phagocytosis, increasing half-time of the vector and/or protecting against serum components. The viral vector envelope may display protein domains shielding the vector or protein modifications acting as markers of self. Displayed protein domains and modifications may include the Albumin Binding Domain from G418, the Albumin Binding Domain from Zag, and/or hypersialylation of the lentiviral glycoproteins.

The viral vector may display CD47 on the surface of the envelope. Preferably the viral vector displays a level of CD47 on the surface of the envelope that is at least 1.2 fold the level of CD47 on the envelope of vectors manufactured using producer cells which do not overexpress CD47. Preferably, the viral vector displays a level of CD47 on the surface of the envelope that is at least 1.2 fold higher than the level of CD47 on the envelope of vectors that have been produced from cells with no modification to additionally express CD47 or to express higher levels of CD47. Expression of CD47 on the envelope has the advantage of reducing phagocytosis of the vector, reducing off-target transduction, and increasing the amount of vector available for on-target transduction.

The viral vector optionally displays a high level of CD47, an agonistic CD3 scFv, 41BBL and/or CD80 on the surface of the envelope. Optionally, anti-4-1BB scFv/VHH, anti-CD40 scFv/VHH, anti-CD28 scFv/VHH, CD86, CD86 immunoglobulin variable-like domain, anti-CD40 scFv/VHH, anti-OX40 scFv/VHH, anti-ICOS scFv/VHH, IL2, and/or IL7 is displayed.

The viral vector may be shielded from complement system mediated degradation or deactivation. The viral vector envelope may display modulators of the complement system or protein domains or modifications preventing recognition by the complement system. Displayed modulators of the complement system may include C1 protease inhibitors, C3 or C5 convertase inhibitors, inactivators of C3b or C4b, disruptors of the membrane attack complex, binders of C8 or C9C1-INH, Factor H, decay-accelerating factors, DAF (CD55), MCP (CD46), CR1 (CD35), CD59, CFHR5, CR2, C4BP, kaposica (KSHV ORF4), EMICE, MOPICE, VCP, HVS-CCPH and/or SPICE. Displayed protein domains and modification may include the Albumin Binding Domain from G418, the Albumin Binding Domain from Zag, and/or hypersialylation of the lentiviral glycoproteins.

Preferably the envelope of the viral vector is substantially free from MHC class I. Most preferably, the viral envelope is devoid of MHC class I.

The envelope of the viral vector being substantially free from MHC class I has the advantage of reducing the immunogenicity of the vector and reducing complement-mediated inactivation.

The viral vector may display a T cell activator on the surface of the envelope. The T cell activator may be an agonistic binder of CD3 or the TCR. Agonistic binders of CD3 or TCR have the advantage of inducing T cell activation, expansion and enhancing transduction of resting T cells. It is particularly preferred that the VHH binder is a TCRab VHH. Optionally, the T cell activator is combined with a co-stimulatory ligand expression to achieve further advantages. The co-stimulatory ligand may be OKT3, e.g., via scFv. The co-stimulatory ligand may be CD28, e.g., via scFV. The co-stimulatory ligand may be CD80. Preferably, the co-stimulatory ligand is 4B1-4.

The viral vector may display a binder or protein inducing a T cell costimulatory signal on the surface of the envelope. Preferably, the binder or protein induces co-stimulation through at least one of OX40 or 41BB, CD28, ICOS, HVEM, CD27, DR3, GITR, CD30, SLQM, CD2, 2B4, TIM1, TIM2, TNFRSF15, CD40L or CD226. Binders or proteins inducing T cell co-stimulation have the advantage of inducing T cell growth, memory, and survival in T cells post-activation.

The antibody binding domain may be specific for a T cell.

The antibody binding domain may be specific for CD3ε. The VHH may comprise the amino acid sequence of any of SEQ ID Nos. 1 to 4. Preferably the VHH comprises the amino acid sequence of SEQ ID No. 3.

The antibody binding domain may be specific for CD8a or b subunits. The VHH may comprise the amino acid sequence of any of SEQ ID Nos. 5 to 8. Preferably the VHH comprises the amino acid sequence of SEQ ID No. 7.

The antibody binding domain may have the amino acid sequence of any of SEQ ID NOs: 1 to 8, 9, 10, 29, 34, 41, 44-46, 48, 49, or 54-65

The antibody binding domain may be specific for the T cell receptor (TCR), preferably the TCR constant region. Advantageously, viral vectors described herein effectively transduce T cells, preferably primary T-cells, expressing endogenous levels of the TCR. The antibody binding domain that is specific for the T cell receptor (TCR) may comprise the amino acid sequence of any of SEQ ID NOs 9-10 or 54-65. The antibody binding domain that is specific for the T cell receptor (TCR) may be a VHH of SEQ ID NO. 9, 56 or 62. Preferably the VHH for the T cell receptor (TCR) is of SEQ ID NO. 9.

The antibody binding domain may be specific for a monocyte or a macrophage.

The antibody binding domain may be specific for CD14, CD16, CCR1, CCR2, CXCR4, or CD64, preferably CD14 or CCR2.

The antibody binding domain may be specific for CD14 or CCR2, preferably CD14. Advantageously, viral vectors described herein effectively transduce monocytes/macrophages, preferably primary monocytes/macrophages, expressing endogenous levels of the CD14 or CCR2. The antibody binding domain that is specific for the CD14 or CCR2 may comprise the amino acid sequence of any of SEQ ID NOs 66-120. The antibody binding domain that is specific for the CD14 may be a VHH of SEQ ID NO. 70, 72, 76, 107, or 111. Preferably the VHH for the CD14 is of SEQ ID NO. 76.

The antibody binding domain may be specific for a natural killer (NK) cell.

The antibody binding domain may be specific for CD56, NKp46, or NKG2C.

The antibody binding domain may be specific for a B cell.

The antibody binding domain may be specific for CD19, MS4A1, FCRL1 or CNR2.

The antibody binding domain may be specific for a dendritic cell.

The antibody binding domain may be specific for CD19, MS4A1, FCRL1 or CNR2.

In all cases, the antibody binding domain is preferably in the form of a VHH, or a VHH binding domain.

The invention also provides viral vector according to any preceding claim for use in therapy.

In a particular embodiment a viral vector described herein comprises a VHH binding domain specific for CD8 or TCRab and also an VHH, scFv ir ligand binding domain specific for CD3, CD28, CD86, 41BB, CD40, OX40, IL2 receptor or IL7 receptor displayed on the exterior of the envelope. Use of this viral vector yields the advantageous characteristic of transducing resting T cells.

Embodiments of the present invention have the advantage of the viral vectors of the invention being particularly suitable for administration *in vivo.* In vivo administration of the viral vectors allows genetic modification of the target cells to express the nucleic acid sequences encoded therein. Preferably, this is to express a particular protein, e.g. a chimeric antigen receptor (CAR).

Suitably, the viral vectors of the present invention may be given by systemic or local injection, whereupon the vectors deliver the "payload" genes comprised in the vector to the target cells within the individual or patient. In this way, the target cells are genetically modified in vivo rather than in vitro.

In vivo administration of the viral vector has the advantage that collection or donation of target cells from the patient, or another individual, for modification ex vivo is not required. Accordingly, production of the modified cells that are active in the cell therapy is much simplified and production and administration of the pharmaceutically effective substance is also much simplified. Amongst other things that are not required when ex vivo processing and re-administration are necessary are the following: cell collection (apheresis), cell processing (e.g. centrifugation and cryopreservation), transport, propagation, manufacturing, product packaging and cryopreservation, distribution to treatment sites, processing, and preparation for infusion to an individual, further administration, or re-administration of the cells to an individual.

The viral vectors of the present invention may be used for ex vivo modification of autologous or allogeneic target cells, which may be administered to an individual or patient. Use of this viral vector yields advantageous characteristics, e.g., in T cells: transducing subpopulations of T cells and also reducing the potential for transduction of transformed cells within the circulating peripheral blood cells collected from the patient. The subpopulation of T cells may be a resting population of T cells.

The invention also provides the viral vector for use in diagnosis, prevention, or treatment of disease. Preferably, the disease is cancer.

The cancer may be a blood cancer. The cancer may be gastric cancer. The cancer may be lung cancer. The cancer may be colorectal cancer. The cancer may be breast cancer.

The cancer may be multiple myeloma.

The invention further provides the viral vector for use in therapy. The therapy may be a cancer therapy. Optionally, the therapy is for an autoimmune disorder. The cancer therapy may be for a cancer selected from: bladder cancer, breast cancer, colon and rectal cancer, endometrial cancer, kidney cancer, leukaemia, liver cancer, lung cancer, melanoma, non-Hodgkin lymphoma, pancreatic cancer, prostate cancer, and thyroid cancer.

The cancer may be selected from acute lymphoblastic leukaemia (ALL); acute myeloid leukaemia (AML); adrenocortical carcinoma; astrocytomas, childhood (brain cancer); atypical teratoid/rhabdoid tumour, childhood, central nervous system (brain cancer); basal cell carcinoma of the skin - see skin cancer; bile duct cancer; bile duct cancer; bladder cancer; bone cancer (Ewing sarcoma and osteosarcoma and malignant fibrous histiocytoma); brain tumours; breast cancer; bronchial tumours (lung cancer); Burkitt lymphoma ; cancer of the central nervous system (atypical teratoid/rhabdoid tumour, childhood (brain cancer); cervical cancer; childhood extracranial germ cell tumours; childhood vascular tumours (soft tissue sarcoma); cholangiocarcinoma ; chordoma (bone cancer); chronic lymphocytic leukaemia (CLL); chronic myelogenous leukaemia (CML); chronic myeloproliferative neoplasms; colorectal cancer; craniopharyngioma (brain cancer); cutaneous t-cell lymphoma (mycosis fungoides and Sézary syndrome); ductal carcinoma in situ (DCIS) ; endometrial cancer (uterine cancer); ependymoma, childhood (brain cancer); esthesioneuroblastoma (head and neck cancer); Ewing sarcoma (bone cancer); extracranial germ cell tumour ; extragonadal germ cell tumour; eye cancer (intraocular melanoma; fallopian tube cancer; gallbladder cancer; gastric (stomach) cancer; gastrointestinal neuroendocrine tumours; gastrointestinal stromal tumours (gist) (soft tissue sarcoma); germ cell tumours (childhood central nervous system germ cell tumours (brain cancer); gestational trophoblastic disease; hairy cell leukaemia; head and neck cancer; heart tumours, childhood; hepatocellular (liver) cancer; Hodgkin lymphoma; hypopharyngeal cancer (head and neck cancer); intraocular melanoma (eye); islet cell tumours, pancreatic neuroendocrine tumours; Kaposi sarcoma (soft tissue sarcoma); kidney (renal cell) cancer; Langerhans cell histiocytosis; laryngeal cancer (head and neck cancer); leukaemia; lip and oral cavity cancer (head and neck cancer); liver cancer; lung cancer (non-small cell, small cell, pleuropulmonary blastoma, pulmonary inflammatory myofibroblastic tumour, and tracheobronchial tumour); lymphoma; medulloblastoma and other CNS embryonal tumours, childhood (brain cancer); melanoma; Merkel cell carcinoma (skin cancer); mesothelioma; metastatic squamous neck cancer (head and neck cancer); midline tract carcinoma ; mouth cancer (head and neck cancer); multiple endocrine neoplasia syndromes; multiple myeloma/plasma cell neoplasms; mycosis fungoides (lymphoma); myelodysplastic syndromes, myelodysplastic/myeloproliferative neoplasms; myelogenous leukaemia, chronic (CML); myeloid leukaemia, acute (AML); myeloproliferative neoplasms, chronic; nasal cavity and paranasal sinus cancer (head and neck cancer); nasopharyngeal cancer (head and neck cancer); neuroblastoma; neuroendocrine tumours (gastrointestinal); non-Hodgkin lymphoma; non-small cell lung cancer; oesophageal cancer; oral cancer, lip and oral cavity cancer and oropharyngeal cancer (head and neck cancer); oropharyngeal cancer; osteosarcoma (bone cancer); osteosarcoma and undifferentiated pleomorphic sarcoma of bone ; ovarian cancer; ovarian germ cell tumours; pancreatic cancer; pancreatic neuroendocrine tumours (islet cell tumours); papillomatosis (childhood laryngeal); paraganglioma; paranasal sinus and nasal cavity cancer (head and neck cancer); parathyroid cancer; penile cancer; pharyngeal cancer (head and neck cancer); pheochromocytoma; pituitary tumour; plasma cell neoplasm/multiple myeloma; pleuropulmonary blastoma (lung cancer); pregnancy and breast cancer; primary central nervous system (CNS) lymphoma; primary peritoneal cancer; prostate cancer; pulmonary inflammatory myofibroblastic tumour (lung cancer); rectal cancer; renal cell (kidney) cancer; retinoblastoma; rhabdomyosarcoma, childhood (soft tissue sarcoma); salivary gland cancer (head and neck cancer); sarcoma (childhood rhabdomyosarcoma (soft tissue sarcoma); Sézary syndrome (lymphoma); skin cancer; small cell lung cancer; small intestine cancer; soft tissue sarcoma; squamous cell carcinoma of the skin ; squamous neck cancer with occult primary, metastatic (head and neck cancer); stomach (gastric) cancer; T-cell lymphoma, cutaneous (mycosis fungoides and Sézary syndrome); testicular cancer; throat cancer (head and neck cancer; thymoma and thymic carcinoma; thyroid cancer; tracheobronchial tumours (lung cancer); transitional cell cancer of the renal pelvis and ureter (kidney (renal cell) cancer); ureter and renal pelvis, transitional cell cancer (kidney (renal cell) cancer; uterine cancer; uterine sarcoma; vaginal cancer; vascular tumours (soft tissue sarcoma); vulvar cancer; and Wilms tumour.

The therapy may be for an inflammatory disease, including but not limited to autoimmune diseases, alopecia areata, pemphigus, scleroderma, Sjogren's syndrome, and/or vitiligo. Most preferably the autoimmune disease is Lupus, arthritic diseases such as rheumatoid arthritis (RA), osteoarthritis, gouty arthritis, spondylitis, and reactive arthritis; Behcet's syndrome; sepsis; septic shock; endotoxic shock; gram negative sepsis; gram positive sepsis; toxic shock syndrome; multiple organ injury syndrome secondary to septicemia (septicaemia), trauma, or hemorrhage (haemorrhage); ophthalmic disorders including but not limited to allergic conjunctivitis, vernal conjunctivitis, uveitis, and thyroid-associated ophthalmopathy; eosinophilic granuloma; pulmonary or respiratory conditions including but not limited to asthma, chronic bronchitis, allergic rhinitis, adult respiratory distress syndrome (ARDS), severe acute respiratory syndrome (SARS), chronic pulmonary inflammatory diseases (e.g., chronic obstructive pulmonary disease), silicosis, pulmonary sarcoidosis, pleurisy, alveolitis, vasculitis, pneumonia, bronchiectasis, hereditary emphysema, and pulmonary oxygen toxicity; ischemic-reperfusion injury, e.g., of the myocardium, brain, or extremities; fibrosis including but not limited to cystic fibrosis; keloid formation or scar tissue formation; atherosclerosis; autoimmune diseases including but not limited to systemic lupus erythematosus (SLE), lupus nephritis, autoimmune thyroiditis, multiple sclerosis, some forms of diabetes, and Reynaud's syndrome; tissue or organ transplant rejection disorders including but not limited to graft versus host disease (GvHD) and allograft rejection; chronic or acute glomerulonephritis; inflammatory bowel diseases including but not limited to Crohn's disease, ulcerative colitis and necrotizing enterocolitis; inflammatory dermatitis including but not limited to contact dermatitis, atopic dermatitis, psoriasis, and urticaria; fever and myalgias due to infection; central or peripheral nervous system inflammatory conditions including but not limited to meningitis (e.g., acute purulent meningitis), encephalitis, and brain or spinal cord injury due to minor trauma; Sjogren's syndrome; diseases involving leukocyte diapedesis; alcoholic hepatitis; bacterial pneumonia; community acquired pneumonia (CAP); pneumocystis carinii pneumonia (PCP); antigen-antibody complex mediated diseases; hypovolemic shock; Type I diabetes mellitus; acute and delayed hypersensitivity; disease states due to leukocyte dyscrasia and metastasis; thermal injury; granulocyte transfusion associated syndromes; cytokine-induced toxicity; stroke; pancreatitis; myocardial infarction, respiratory syncytial virus (RSV) infection; and spinal cord injury.

A chimeric antigen receptor (CAR) for targeting epitopes displayed by cancer cells of one, a multiplicity, or any of the cancers above may be used in the context of the present invention. The CAR may be a B-cell maturation antigen (BCMA) CAR. The CAR may be a CD19 CAR. The CAR may be a Claudin 18.2 CAR; optionally, the sequence encoding the Claudin 18.2 CAR is derived from SEQ ID NO: 53. The CAR may be a HER2 CAR. The CAR may be a Mesothelin CAR.

An engineered TCR for targeting epitopes displayed by cancer cells of one, a multiplicity, or any of the cancers above may be used in the context of the present invention. The TCR may be specific for the cancer testis antigens. The cancer testis antigens may be NY-ESO or MAGEA4 or PRAME. The TCR may be specific for the viral antigens. The viral antigen may be from HPV. The TCR may be specific for WT1.

An engineered TCR may be a "domain swapped" TCR. Exogenous human TCR alpha beta transduced in human CD3+ T lymphocytes may mispair with the endogenous TCR alpha beta chains and potentially lead to toxicity from recognition of a self-antigen by the mispaired TCR. To reduce this risk of mispairing between exogenous and endogenous TCR chains, the region of the constant domain of the alpha (Calpha; Cα) and beta (Cbeta; Cβ) chains is "swapped" between the two chains. Using this "domain swap" approach, the alpha receptor is composed of the variable alpha (Valpha) and the Cbeta domains (noted VaCb) while the beta receptor will be composed of the variable beta (Vbeta) and the Calpha domains (noted VbCa). The two domain-swapped receptors can still form heterodimer together but can no longer pair with any wildtype TCR alpha or beta chain, thus preventing mispairing of the TCR chains.

The invention further provides a method of making the above retroviral vector, comprising the steps of:
(a) engineering a cell to express a membrane-anchored sdAb binding domain that is cell-type specific;
(b) engineering the cell to express a Lassa virus envelope protein that is able to facilitate infection of the same cell type as in (a);
(c) replicating in the cell a nucleic acid vector comprising a promoter expressible in the same cell type as in (a); and
(d) harvesting the retroviral vector in the form of a vesicles from the cell.

A vesicle is a structure comprising of liquid or cytoplasm enclosed by a lipid bilayer. The vesicle released from the cell may be a bleb. In cell biology, a bleb is a bulge of the plasma membrane of a cell, characterized by a spherical, "blister-like", bulky morphology. Blebbing, or zeiosis, is the formation of blebs. Formation of blebs by blebbing is how virus particles of enveloped viruses are released from the infected cell that is producing them.

The nucleic acid vector may be a plasmid.

The method may comprise the step of engineering the producer cell to knock down expression of MHC class I. Preferably, the method comprises the step of engineering the producer cell to knock out expression of MHC class I.

Most preferably, the method comprises the step of modifying MHC class I expression by introducing a change in the sequence of the nuclear genome. Thus, the viral vector envelope may be engineered to be devoid of MHC class I.

Deletion of MHC class-I on the envelope of producer cells yields vectors without MHC class I on the surface of the vector which, in turn, advantageously reduces the immunogenicity of the associated vector and reduces complement-mediated inactivation.

Editing proteins in the forms of nucleases, base editors, and prime editors may be packaged within the vectors to mediate introduction of the change in the sequence of the nuclear genome.

In the current context "mediate" is defined as causing all yielding the first stage or substrate of a physiological pathway or process.

The endonuclease may be an RNA-directed DNA-specific endonuclease. Preferably, the endonuclease is a CRISPR-Cas ribonucleoprotein complex.

The endonuclease may be a TALEN. The endonuclease may be a Zinc-finger nuclease.

The producer cell line may be the 293T cell line, or a cell line derived therefrom.

The producer cell may be engineered to express or overexpress a cell surface protein or intracellular protein. In some embodiments this protein is CD47. Advantageously, expression of CD47 on the envelope reduces phagocytosis of the vector yielded by the producer cell, reduces off-target transduction, and increases the amount of vector available for on-target transduction.

In some embodiments the producer cell may express a protein that is destined to be packaged into the budding viral particle. In some embodiments, this protein may be Vpx. Advantageously, delivery of Vpx to the transduced cell in vivo can increase the efficiency of target cell reprogramming through the Vpx mediated inhibition of the cell's antiviral defences. In another embodiment this protein may be a gene editing protein including Cas9, Cas12, Cpf1,a Zinc finger nuclease, a transcription activator-Like effector nuclease (TALEN), a base editor or a prime editor. The gene editing proteins may be modified to enhance packaging within the viral particles. Advantageously, delivery of the gene editor protein using the vector described herein provides a mechanism for cell specific gene editing in vivo.

In any case, it should be understood that the producer cell may be modified in an appropriate way to allow the production of a viral vector according to the invention, as outlined above and below.

The invention further provides an ex vivo method of modifying a cell comprising the step of exposing the cell to a retroviral vector as described herein.

The cell to be modified may display an antigen on its exterior that may be bound by the antibody binding domain (VHH binding domain) displayed on the envelope of the viral vector.

In some embodiments vectors targeting different cell types may be administered together or in short succession to enable in vivo reprogramming of different cell types. The different cell specific vectors may carry the same cargo or transgene or each a different cargo or transgene. Advantageously, this provides a method for reprogramming different cell types with disease fighting mechanisms that may be synergistic and non-redundant so as to provide more effective treatment of disease. The cell to be modified may be a T cell. Modified T cells are used in various therapies, e.g. CAR-T cell therapies, however the ability to successfully modify T cells in vivo thus far has been limited, making *ex vivo* modification of T cells the preferred option. The invention thus overcomes previous limitations, meaning an effective method of producing modified T cells in vivo is provided.

The cell to be modified is preferably an immune effector cell.

The cell to be modified may be a T cell.

The antigen is preferably one that is highly conserved in T cells and is expressed on the cell membrane.

The antigen may be the T cell receptor (TCR).

The antigen may be of the CD3ε phenotype.

The cell to be modified may be a monocyte or a macrophage. The cell to be modified may be a B cell. The cell to be modified may be a dendritic cell (DC).

The antigen is preferably one that is highly conserved in monocytes and/or differentiated forms thereof and is expressed on the cell membrane.

The antigen may be selected from CD14, CD16, CCR1, CCR2, CXCR4, and CD64. Preferably the antigen is selected from CD16, CXCR4, and CCR1.

The cell to be modified may be a natural killer (NK) cell.

The antigen is preferably one that is highly conserved in NK cells and is expressed on the cell membrane.

The viral vector may be administered in an amount greater than 10⁶, greater than 10⁷, greater than 10⁸, greater than 10⁹, greater than 10¹⁰, or greater than 10¹¹ vector particles, preferably greater than 10⁸ vector particles.

The viral vector may be administered at a frequency of less than two weeks, i.e. a second or supplementary administration of the viral vector is carried out less than two weeks after the first administration of the viral vector. Preferably a second or supplementary administration of the viral vector is carried out less than one week after the first administration of the viral vector.

A first course of the viral vector (one or more doses) may be administered followed by a second or supplementary administration of the viral vector following a period selected from: six months, one year, eighteen months, two years, three years, four years, five years, or six years.

In another embodiment, the disclosure provides a virus-like particle having a lipid bilayer envelope comprising:
(a) a VHH binding domain displayed on the exterior of the envelope that is cell-type specific; and
(b) an envelope protein displayed on the exterior of the envelope that is able to facilitate infection of the same cell type as in (a), wherein the envelope protein is as described elsewhere herein.

The virus-like particle is suitable for delivery of materials contained within the virus-like particle to specific types of cells, as described elsewhere herein.

The invention thus provides a highly specific retroviral vector, methods of using the same in therapy, methods of making the same and methods of modifying cells using the same *ex vivo.*

### List of Figures and Drawings

The invention is now illustrated with reference to the following figures in which:

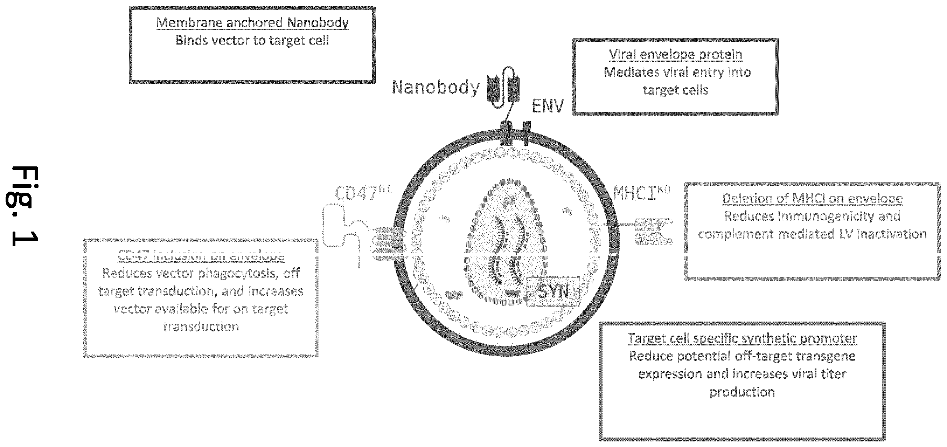

Fig. 1 Shows an illustrative example of a vector according to the disclosure.

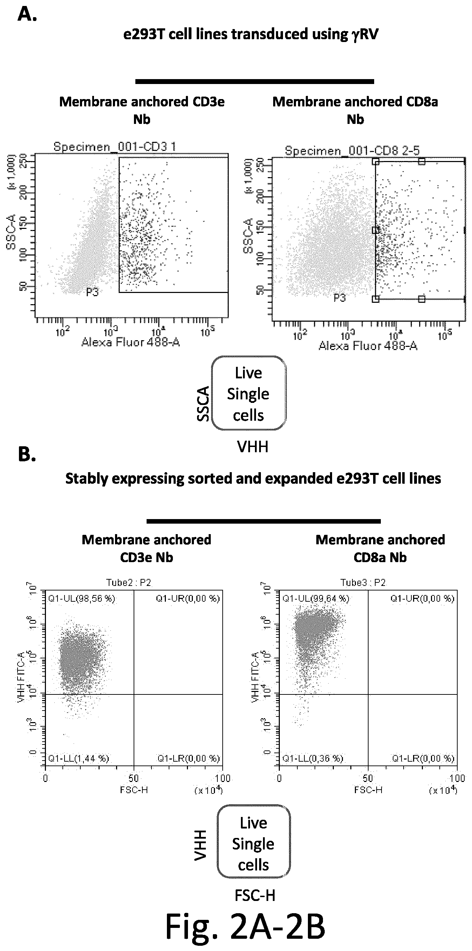

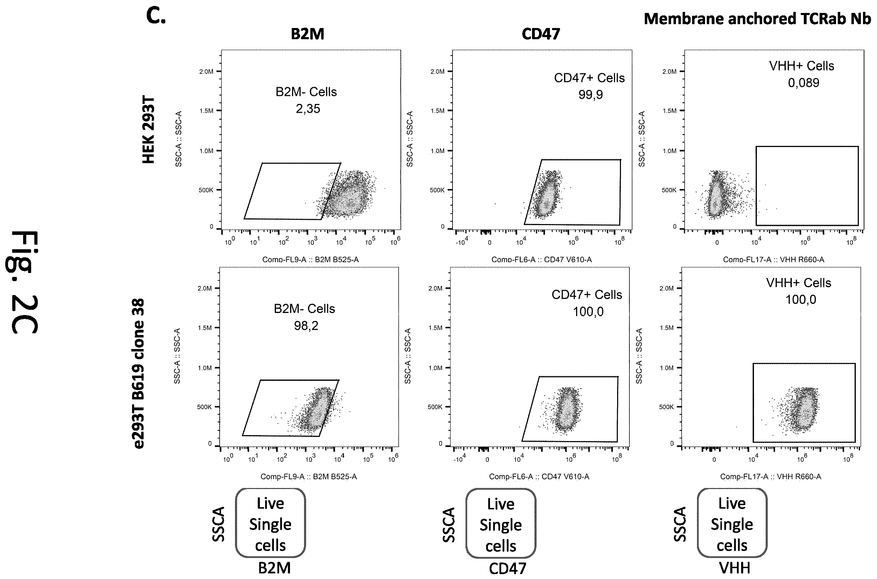

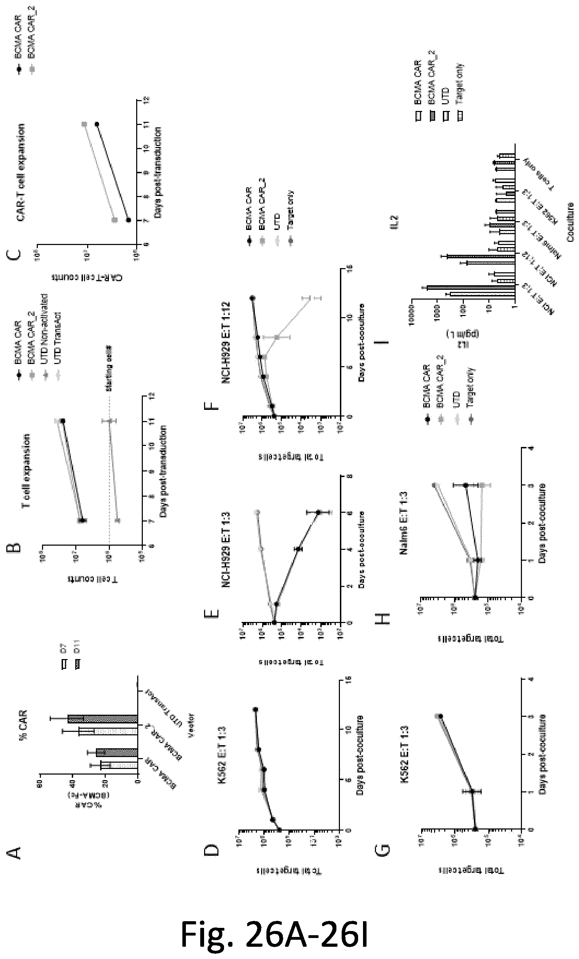

Fig. 2 Depicts flow cytometry scatter plots of the engineered 293T packaging monoclonal cell lines that are used to generate specific embodiments of the engineered lentiviral vectors. Parental 293T overexpressing surface hCD47 (middle panel) and depleted of MHCI (e293T) were transduced with a gamma retrovirus (gRV) encoding CD3e or CD8a membrane anchored VHH and gated (P3) to isolate the stably expressing cells by fluorescence-activated cell sorting (A). The polyclonal cell lines thus generated (B) were expanded and frozen for long-term storage and use. (C) In another iteration, the parental e293T were transduced with a lentivirus (LV) encoding a TCRab membrane anchored VHH and monoclonal cell lines were isolated by limiting dilution cloning. e293T B619 clone 38 is a monoclonal producer cell line derived by such a method and expression of B2M, CD47 and membrane anchor nanobody (VHH) was confirmed by flow cytometry compared to the reference wildtype cell line HEK 293T known to be B2M positive, CD47 baseline level and VHH negative.

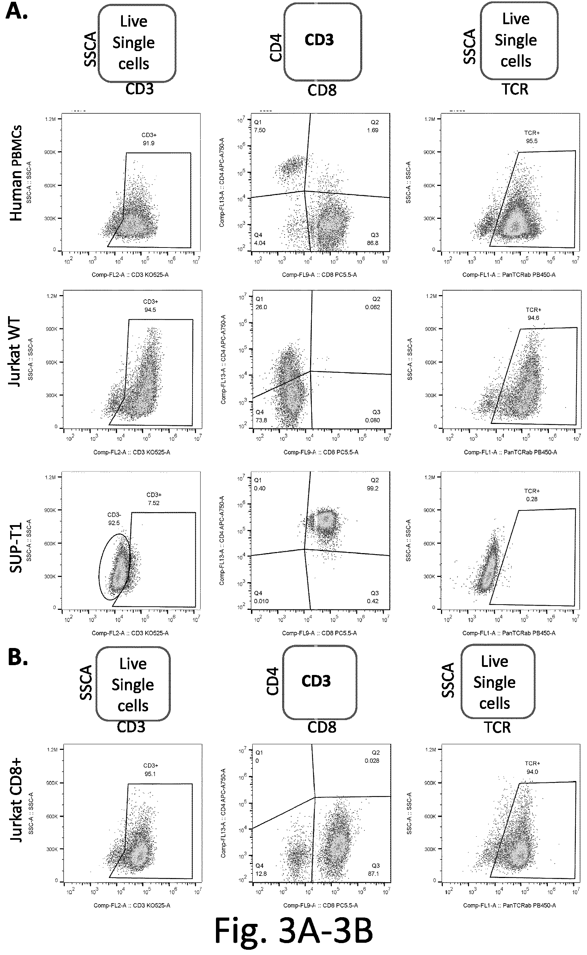

Fig. 3 shows flow cytometry scatter plots of Jurkat wild type cells (WT), Sup-T1 cells, a Jurkat cell line stably expressing hCD8a (Jurkat CD8+) and primary human PBCMs stained with CD3, CD4, CD8 and TCR antibodies. The result demonstrates that contrary to Sup-T1 cells, which express surface CD3 and CD8, Jurkat WT cells do not express CD8 but express TCR, whereas Jurkat cells stably expressing CD8 display surface expression of CD3, CD4, TCR and transgenic CD8a.

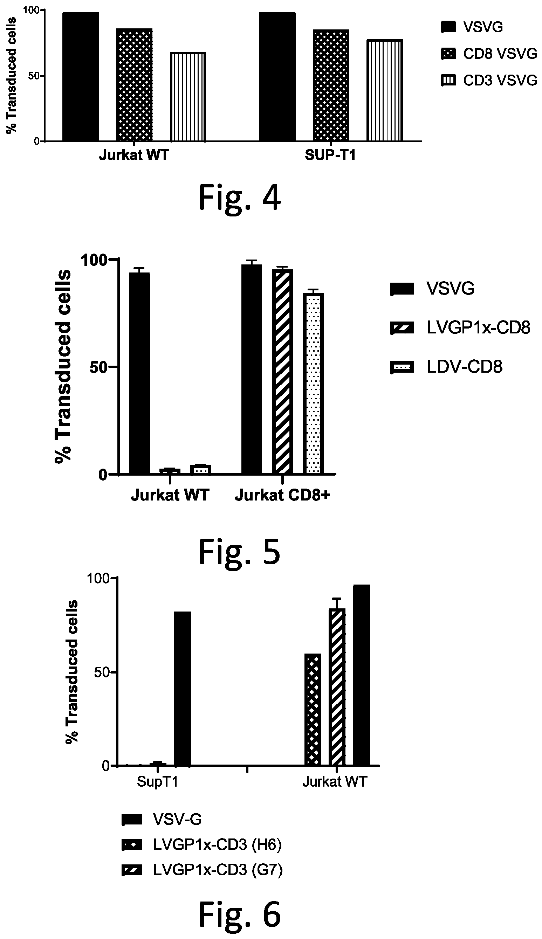

Fig. 4 shows a graph of the quantification by flow cytometry of the percentage transduction, determined by expression of a GFP reporter, in Jurkat WT and Sup-T1 cells transduced with different types of wild type Vesicular Stomatitis virus (VSVG), pseudotyped lentiviruses. Jurkat WT cells and Sup-T1 cells were transduced with VSVG pseudotyped lentivectors expressing a GFP reporter and displaying either a CD8a VHH on its envelope (CD8 VSVG) or a CD3e VHH (CD3 VSVG) or no VHH (VSVG). 3 days post-transduction, the cells were analysed by flow cytometry for the expression of the GFP reporter. As shown in this figure, transduction of VSVG wild type pseudotyped vectors displaying either CD3e or CD8a membrane anchored nanobodies, is equivalent between Jurkat WT (which do not express CD8) and Sup-T1 (which do not express CD3). This result demonstrates that displaying a membrane anchored VHH on the surface of lentiviral vectors pseudotyped with the wild type vesicular stomatitis virus glycoprotein is not sufficient to achieve cell specific transduction.
Fig. 5 shows a graph of the quantification by flow cytometry of the percentage transduction, determined by expression of a GFP reporter, in Jurkat WT and Jurkat CD8+ cells transduced with different types of engineered lentiviruses compared. Jurkat cells (Jurkat WT) or Jurkat cells stably expressing human CD8a (Jurkat CD8+) were transduced with GFP expressing lentiviral vectors pseudotyped with the mutant Lassa Virus glycoprotein Y150A (LVGP1x) or the wild type Le Dantec virus glycoprotein (LDV) and displaying a membrane anchored CD8a VHH on the viral envelope. This result demonstrates that contrary to broad tropism wild type VSVG lentiviral particles, CD8a VHH targeted lentiviral particles LVGP1x-CD8 and LDV-CD8 specifically target the engineered vectors to Jurkat cells expressing the VHH targets (Jurkat CD8+).
Fig. 6 shows graphs of the quantification by flow cytometry of the percentage transduction by targeted (LVGP1x-CD3) and non-targeted (VSVG) vectors on target (Jurkat WT) and non-target (Sup-T1) cells. Sup-T1 cells and Jurkat WT cells were transduced either with a broad tropism VSVG lentiviral vector or a CD3 VHH displaying vector pseudotyped with the LVGP1x glycoprotein. This result shows that contrary to the VSVG vector, the CD3e VHH displaying LVGP Y150A lentiviral particle transduces CD3 expressing cells only. This demonstrates that targeting LVGP1x pseudotyped vectors with a VHH different from CD8a also achieves target specific transduction.

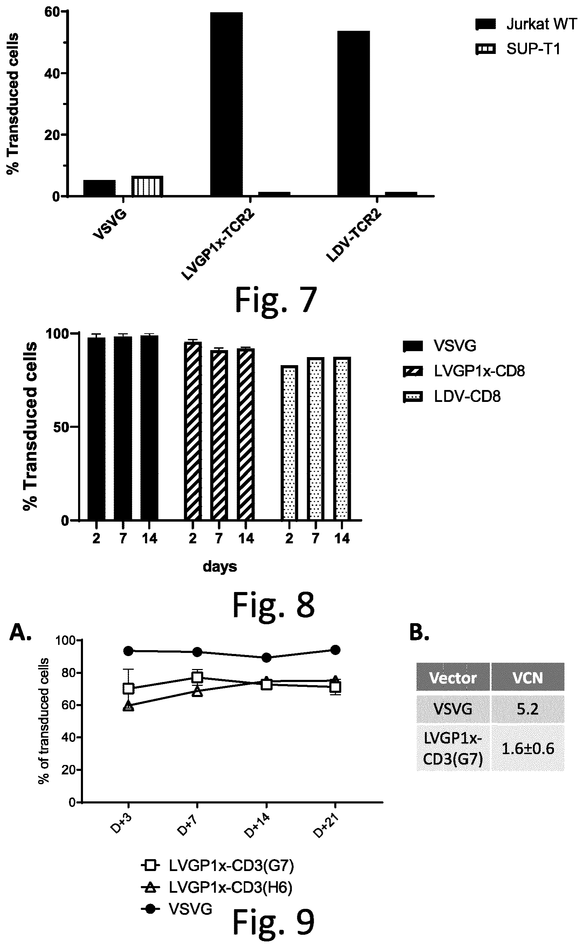

Fig. 7 shows a graph of the quantification by flow cytometry of the percentage transduction efficiency of targeted (LVGP1x-TCR2 and LDV-VHHTCR2) and non-targeted (VSVG) vectors on target (Jurkat WT) and non-target (Sup-T1) cells. Sup-T1 cells and Jurkat WT cells were transduced either with a broad tropism VSVG lentiviral vector (VSVG) or a TCR VHH displaying vectors pseudotyped with LVGP1x or LDV (LVGP1x-TCR2 and LDV-VHHTCR2). This result shows that contrary to VSVG vector, the TCR VHH targeted LVGP1x or LDV lentiviral particles transduce only TCR expressing cells (Jurkat WT). This demonstrates that targeting LVGP1x and LDV pseudotyped vectors with nanobodies for different T cell markers achieves target specific transduction.
Fig. 8 shows a graph of the quantification by flow cytometry of the percentage transduction efficiency of targeted (LVGP1x-CD8a and LDV-CD8a) and non-targeted (VSVG) vectors on target cells over time. Jurkat CD8+ cells stably expressing human CD8a were transduced as with VSVG, LVGP1x-CD8a or LDV-CD8a GFP expressing vectors and GFP expression was quantified by flow cytometry over 14 days in culture. This result demonstrates that as with VSVG lentiviral particles, CD8a VHH targeted lentiviral particles pseudotyped with LVGP1x and LDV stably transduce target cells.
Fig. 9 shows a graph of the quantification by flow cytometry of the percentage transduction efficiency of targeted (LVGP1x-CD3) and non-targeted (VSVG) vectors on target cells over time and a quantification of viral genome insertion by quantitative PCR. Jurkat WT cells in culture were transduced with a broad tropism VSVG vector or target specific vectors pseudotyped with LVGP1x associated with either of two different CD3e nanobodies (G7 and H6). Expression of the GFP transgene was monitored over time using flow cytometry and vector insertion into the host genome was assessed at day 21 by quantitative PCR amplification of the inserted vector sequence. This result shows that transduction using LVGP1x-CD3e VHH targeted vectors is stable over 21 days (A.) and results in insertion of the viral genome in the host DNA (B.).

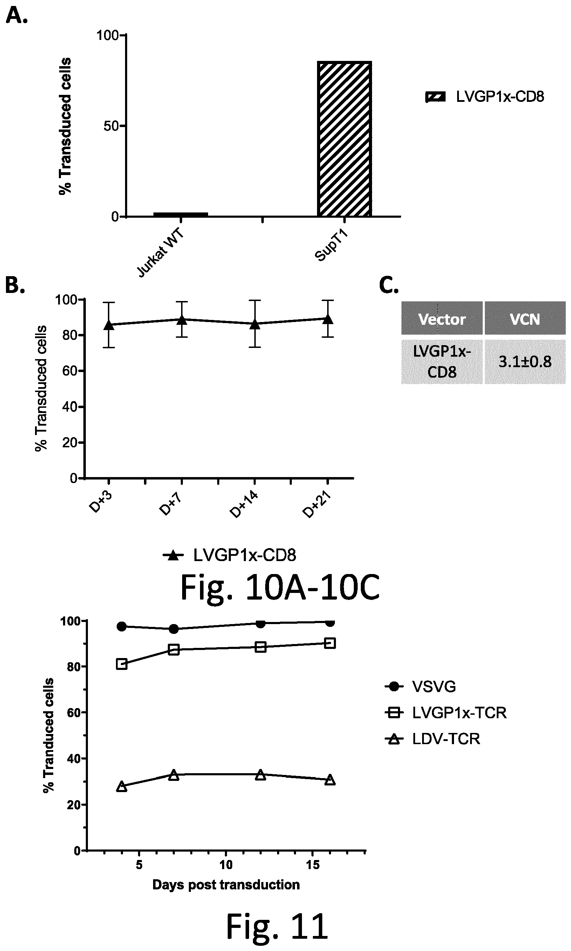

Fig. 10 shows a graph of the quantification by flow cytometry of the percentage transduction efficiency of targeted (LVGP1x-CD8) and non-targeted (VSVG) vectors on target (Sup-T1) and non-target cells (Jurkat WT) at Day 3 (A.) and over time (B.) and a quantification of viral genome insertion by quantitative PCR (C.). Jurkat WT and Sup-T1 cells in culture were transduced with a broad tropism VSVG vector or target specific vectors pseudotyped with LVGP1x displaying a CD8a VHH. Expression of the GFP transgene was monitored over time using flow cytometry and vector insertion of the genome was assessed at day 21 by quantitative PCR amplification of the inserted vector sequence. This result shows that transduction using LVGP1x-CD8 VHH targeted vectors is specific to target cells expressing CD8a (A.), is stable over 21 days (B.) and results in insertion of the viral genome in the host DNA (C.).
Fig. 11 shows a graph of the quantification by flow cytometry of the percentage transduction efficiency of targeted (LVGP1x-TCR and LDV-TCR) and non-targeted (VSVG) vectors on target (Jurkat WT) over time. Jurkat WT cells in culture were transduced with a broad tropism VSVG vector or target specific vectors pseudotyped with LVGP or LDV displaying a TCR targeting VHH. Expression of the GFP transgene was monitored over time using flow cytometry. This result shows that transduction using LVGP or LDV TCR VHH targeted vectors is stable over 16 days.

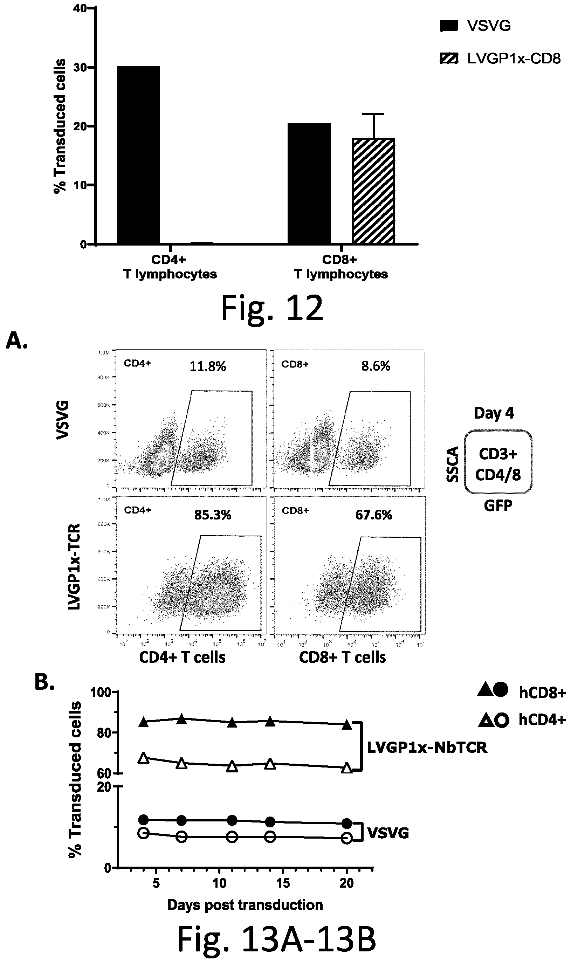

Fig. 12 shows a graph of the quantification by flow cytometry of the percentage transduction efficiency of targeted (LVGP1x-CD8) and non-targeted (VSVG) vectors on human primary CD4+ and CD8+ T cells. Human PBMCs were activated in culture and transduced with LVGP1x-CD8 or VSVG vectors and GFP expression was quantified by flow cytometry 7 days post transduction. This result demonstrates that a LVGP1x-CD8 vector contrary to a VSVG pseudotyped vector specifically transduces primary T cells expressing endogenous levels of the CD8a VHH target, namely CD8a.
Fig. 13 shows flow cytometry plots and a graph of the quantification by flow cytometry of the percentage transduction efficiency of targeted (LVGP1x-TCR) and non-targeted (VSVG) vectors on human primary CD4+ and CD8+ T cells. Human PBMCs were activated in culture and transduced with LVGP1x-TCR or VSVG vectors and GFP expression was quantified by flow cytometry 4 days post transduction (A.) and over 20 days (B.). This result demonstrates that a LVGP1x-TCR vector effectively transduces primary T cells expressing endogenous levels of the TCR and that LVGP1x-TCR transduction is stable and more effective than that of the VSVG pseudotyped vector under the same conditions.

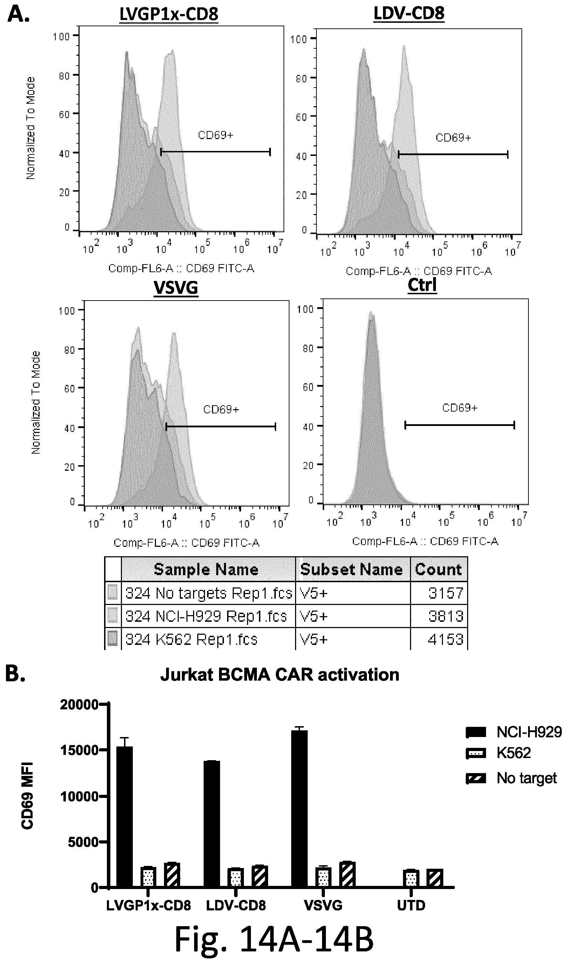

Fig. 14 shows flow cytometry plots of BCMA CAR transduced Jurkat-CD8+ cells in co-culture with BCMA expressing or non-expressing cells or transduced cells alone. Jurkat-CD8 cells were transduced with LVGP1x-CD8 or LDV-CD8 or broad tropism VSVG lentiviral vectors coding for BCMA CAR. The transduced cells were cultured alone (No target), with BCMA expressing cells (NCI-H929) or with a cell line in which BCMA expression is absent (K562). The surface expression of CD69, a marker of T cell activation, was assessed by flow cytometry (A.) and a graph summarizing the data is presented in (B.) This result shows that co-culture of BCMA CAR expressing Jurkat-CD8 cells with NCI-H929 cells results in increased CD69 surface expression compared to conditions with T cells alone or in co-culture with non-target cells. This result demonstrates that transduction of Jurkat-CD8 with LVGP1x-CD8 or LDV-CD8 vectors coding for BCMA CAR results in functional expression of the CAR construct and signalling consistent with T cell activation comparable to cells transduced with a broad tropism VSVG vector.

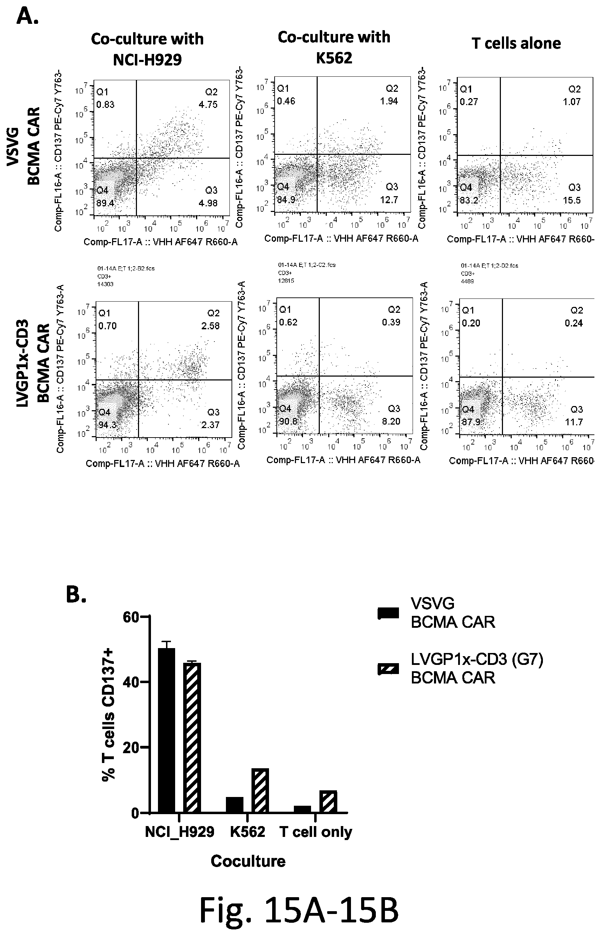

Fig. 15 shows flow cytometry plots of BCMA CAR transduced primary T cells in co-culture with BCMA expressing or non-expressing cells or T cells alone. Activated primary human T cells were transduced with targeted LVGP1x-CD3 or broad tropism VSVG lentiviral vectors coding for BCMA CAR. The transduced cells were cultured alone (No target), with BCMA expressing cells (NCI-H929) or with a cell line in which BCMA expression is absent (K562). The surface expression of CD137, a marker of T cell activation, was assessed by flow cytometry (A.) and a graph summarizing the data is presented in (B.) This figure shows that co-culture of BCMA CAR expressing T cells with NCI-H929 cells results in increased CD137 surface expression compared to conditions where the BCMA CAR-T cells were cultured either alone or with non-target cells. This result demonstrates that transduction of primary T cells with LVGP1x-CD3 vector coding for BCMA CAR results in functional expression of the CAR construct and signalling consistent with T cell activation, which is comparable to cells transduced with a broad tropism VSVG vector.

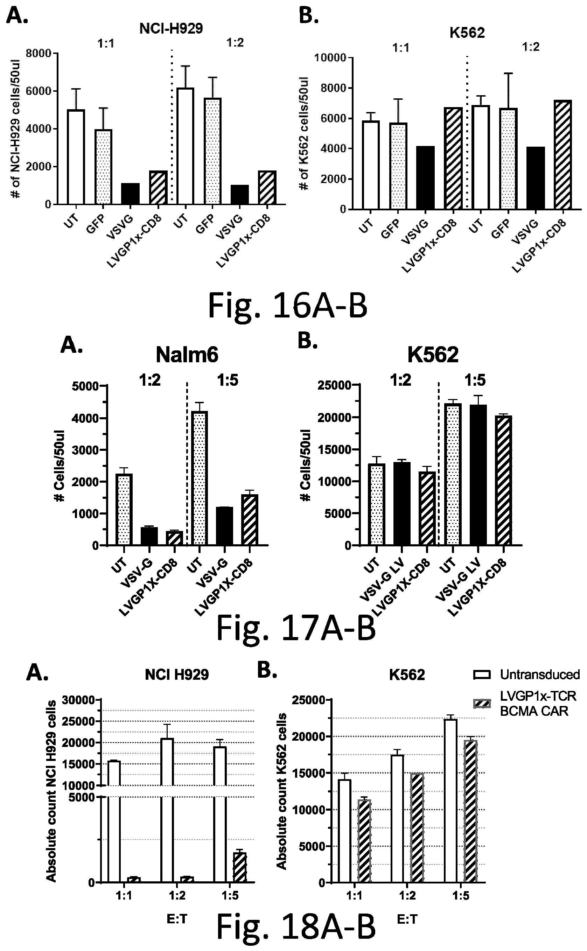

Fig. 16 shows graphs summarizing flow cytometry quantification of target and non-target cell numbers in BCMA CAR-T co-cultures. Activated human PBMCs were transduced with LVGP1x-CD8 or broad tropism VSVG lentiviral vectors coding for BCMA CAR under the control of a T cell specific synthetic promoter (SYN 17; for the LVGP1x-CD8 vector) or a constitutive promoter (EF1a; for the VSVG vector). The transduced cells were expanded and co-cultured with NCI-H929 (BCMA expressing cells) or K562 non-target cells (not expressing BCMA) at ratios of 1:1 or 1:2 (ratio of effector T cells to target/non-target cell line). The number of Cell Trace Violet stained target/non-target cells was quantified by flow cytometry in 50ul of media and summarized in graphs A. and B. This figure shows that co-culture of CD19 CAR-T cells results in a significant decrease in the number of target cells (NCI-H929) but not of non-target cells (K562) compared to co-cultures with VSVG-GFP transduced or non-transduced T cells. This result demonstrates that transduction of primary T cells with LVGP1x-CD8 coding for BCMA CAR results in functional expression of the CAR construct and specific cytotoxicity of the transduced cells for target cells comparable to T cells transduced with a broad tropism VSVG vector.
Fig. 17 shows graphs summarizing flow cytometry quantification of target (Nalm6) and non-target (K562) cell numbers in CD19 CAR-T co-cultures. Activated human PBMCs were transduced with LVGP1x-CD8 or broad tropism VSVG lentiviral vectors coding for CD19 CAR under the control of a T cell specific synthetic promoter (SYN 17; for the LVGP1x-CD8 vector) or a constitutive promoter (EF1a; for the VSVG vector). The transduced cells were expanded and co-cultured with Nalm6 (CD19 expressing cells) or K562 non-target cells (not expressing CD19) at ratios of 1:2 or 1:5 (ratio of effector T cells to target/non-target cell line). The number of Cell Trace Violet stained target or non-target cells was quantified by flow cytometry in 50ul of media and summarized in graphs A. and B. respectively. This figure shows that co-culture of CD19 CAR-T cells results in a significant decrease in the number of target cells (Nalm6) but not of non-target cells (K562) compared to co-cultures with non-transduced T cells. This result demonstrates that functional transduction using the LVGP1x-CD8 vector is applicable beyond BCMA CAR and that transduction of primary T cells with LVGP1x-CD8 coding for CD19 CAR results in functional expression of the CAR construct and cytotoxicity of the transduced cells specific for target cells comparable to T cells transduced with a broad tropism VSVG vector.
Fig. 18 shows graphs summarizing flow cytometry quantification of target and non-target cell numbers in BCMA CAR-T co-cultures. Activated human PBMCs were transduced with LVGP1x-TCR coding for BCMA CAR under the control of a T cell specific synthetic promoter (SYN19). The transduced cells were expanded and co-cultured with NCI-H929 (BCMA expressing cells) or K562 non-target cells (not expressing BCMA) at ratios of 1:1 or 1:2 or 1:5 (ratio of effector T cells to target/non-target cell line). The number of Cell Trace Violet stained target/non-target cells was quantified by flow cytometry in a fixed volume of media and summarized in graphs A. and B. This figure shows that co-culture of BCMA CAR-T cells result in a significant decrease in the number of target cells (NCI-H929) but not of non-target cells (K562) compared to co-cultures with non-transduced T cells. This result demonstrates that transduction of primary T cells with LVGP1x-TCR coding for BCMA CAR under the control of the T cell specific promoter results in functional expression of the CAR construct and cytotoxicity of the transduced cells specific for target cells.

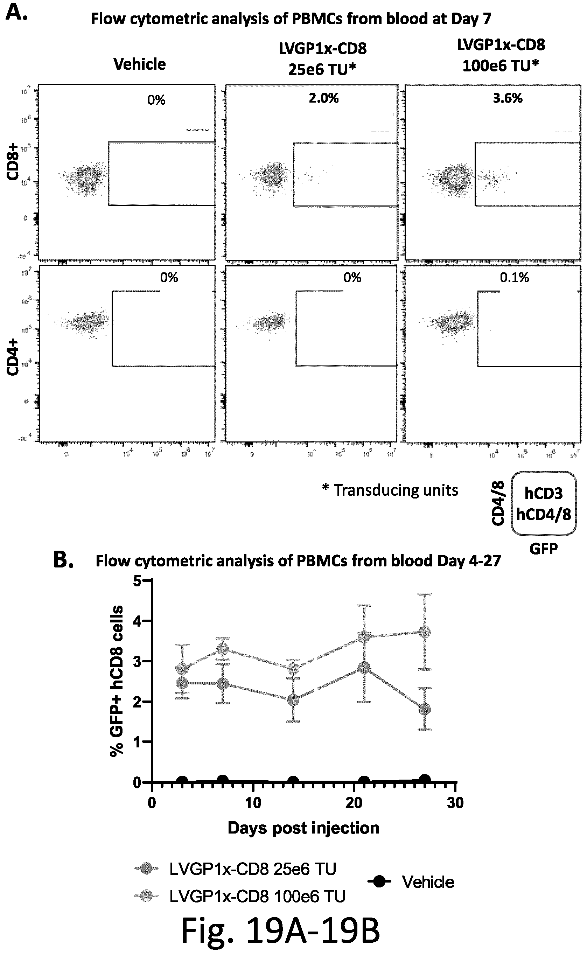

Fig. 19 shows flow cytometry plots of human CD8 or CD4 T cells in peripheral blood of humanized NSG mice injected with targeted LVGP1x-CD8 vector and the persistence of the CD8+ transduced cells over 27 days. Activated human PBMCs were injected intraperitoneally in immune deficient NSG mice on day 0 and one day later viral vectors coding for GFP under the control of a ubiquitous EF1a promoter were injected intraperitoneally. Transduction of human T cells was assessed by flow cytometry for GFP and cell specific markers in peripheral blood of the mice at Day 4, 7, 14, 21 and 27. This figure shows that injection of 25×106 or 100×106 transducing units (TU) of LVGP1x-CD8 results in transduction of CD8+ human T cells specifically (A.) and that the expression of the transgene and the transduced CD8 T cells persist over 27 days (B.). This result demonstrates target specific and stable transduction of human CD8 T cells in vivo using the CD8 VHH targeted vector LVGP1x-CD8.

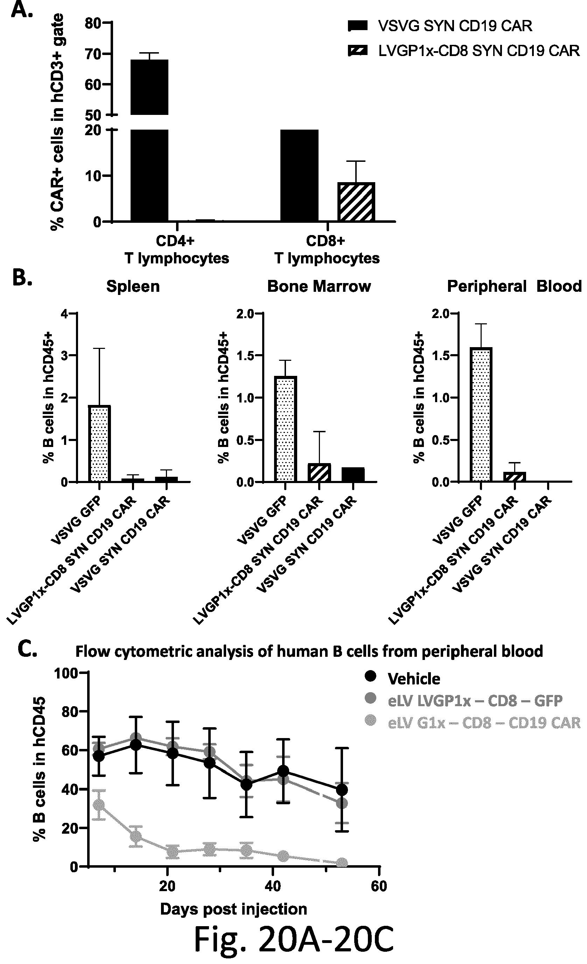

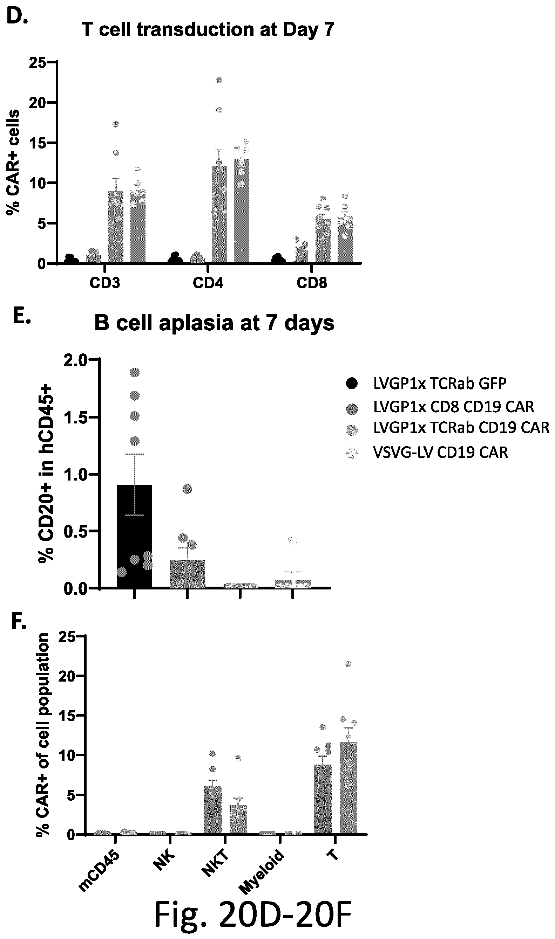

Fig. 20 (A, B) shows graphs summarizing flow cytometry quantification of transduced human CD8 and CD4 T cells and levels CD20 B cells in humanized NSG mice injected intraperitoneally with activated human PBMCs and viral vectors. Activated human PBMCs were injected intraperitoneally in immune deficient NSG mice on Day 0 and one day later a VSVG vector coding for GFP or CD19 CAR under the control of the EF1a promoter (VSVG GFP and VSVG EF1a CD19 CAR) or LVGP1x-CD8 vector encoding CD19CAR under the control of a synthetic T cell specific promoter (LVGP1x-CD8 SYN CD19 CAR) were injected intraperitoneally. CD19 CAR expression in human T cells was assessed in peripheral blood at day 7 post viral injection by flow cytometry (A.). B cell aplasia was assessed in dissociated spleen, bone marrow or in peripheral blood using flow cytometry for the B cell marker CD20 at day 7 post viral Injection (B.). This figure shows that injection of 100e6 transducing units of LVGP1x-CD8 results in transduction of CD8+ human T cells specifically, whereas injection of the same dose of VSVG vector results in broad transduction of both CD4 and CD8 cell populations. In addition, the percentage of B cells in spleen, bone marrow and peripheral blood is significantly decreased in mice injected with the CD19 CAR coding vectors compared to animals injected with the GFP coding vector. This result demonstrates target specific transduction of human CD8 T cells in vivo using LVGP1x-CD8 coding for a CAR construct which results in target cell depletion at levels similar to those observed in mice injected with a CAR coding broad tropism VSVG vector. (C) shows a graph representing the quantification by flow cytometry of the percentage of human B cells circulating in hCD34+ humanized NCG mice injected intravenously with eLV-LVGP1X-CD8a viral vectors. Healthy hCD34+ stably humanized NCG mice were injected intravenously with eLV-LVGP1X-CD8a vectors expressing GFP or a CD19 CAR construct under the control of the T cell specific synthetic promoter #19. Percentage of CD20+ (B cells) in peripheral blood assessed by flow cytometry. The data represent the mean ± SEM from 4-6 animals per condition. These results demonstrate potent and persistent anti-target cell activity of CD19 CAR-T cells reprogrammed in vivo reprogrammed from a single injection of eLV-LVGP1X-CD8a targeted vector expressing a BCMA CAR under the control of the T cell specific promoter #19.
   (D-F) shows graphs summarizing flow cytometry quantification of human CD20+ (B cells), CD3+, CD4+ and CD8+ (T lymphocytes) cells in peripheral blood of humanized NSG mice injected with targeted LVGP1x-CD8 - CD19 CAR (10E+06 TU), LVGP1x-TCRab - CD19 CAR (10E+06 TU) or LVGP1x-TCRab - GFP (50E+06 TU) vectors or the broad tropism VSVG LV vector expressing CD19-CAR (50E+06 TU). Activated human PBMCs were injected intraperitoneally in immune deficient NSG mice on day 0 and one day later viral vectors coding for a CD19 CAR construct under the control of the T cell specific synthetic promoter SYN23 or GFP under the control of a ubiquitous EF1a promoter were injected intraperitoneally. Levels of human B cells and levels of T cell transduction were assessed by flow cytometry in peripheral blood of the mice at Day 7. (D.) CD19 CAR T cells were detected as early as day 7 post vector administration in animals injected with CD19 CAR vector. LVGP1x-TCRab - CD19 CAR vector effectively transduced both CD4+ and CD8+ T cells, whereas the LVGP1x-CD8 - CD19 CAR vector transduction was specific for CD8+ T cells. Transduction efficiency of the LVGP1X-TCRab was significantly higher compared to broad tropism VSVG LV, reaching the same level of T cell transduction at 1/5th of the dose. (E). B cell aplasia was complete day 7 post vector infusion in the LVGP1x-TCRab - CD19 CAR group and near complete for the LVGP1x-CD8. This result demonstrates the potent anti target activity of the in vivo reprogrammed CD19 CAR-T cells using LVGP1X T cell targeted vectors. (F.) Flow cytometric analysis of peripheral blood for the percentage of CAR+ cells within different immune cell types (murine CD45+ cells, hCD45+/CD56+ human NK cells, hCD45+/CD3+/CD56+ human NKT cells, hCD45+/CD14+ human myeloid cells, hCD45+/CD3+/CD4+ and CD8+ human T cells) at 11 days post vector injection. The results represent the mean ± SEM from 8 animals. Note: B cell expression of CD19CAR was not assessed due to B cell aplasia.

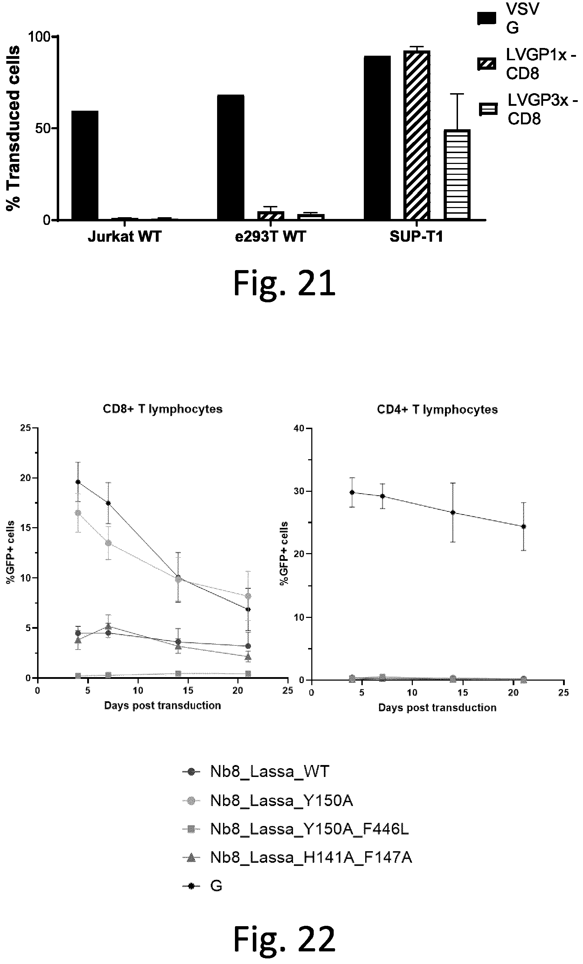

Fig. 21 shows a graph of the quantification by flow cytometry of the percentage transduction of CD8a target negative cell lines (Jurkat WT and 293T) versus CD8a positive cell line (Sup-T1) using broad tropism VSVG lentiviral vector and CD8a targeted lentiviral vectors pseudotyped with Y150A (LVGP1X) and Y150A H141A F147A Lassa virus (LVGP3X) glycoprotein. CD8a negative cell lines Jurkat WT and 293T cells and CD8a positive Sup-T1 cells were transduced with VSVG and LVGP1x-CD8 or LVGP3x-CD8 lentiviral vectors and GFP expression was quantified by flow cytometry at 3 days post-transduction. This result shows that LVGP1x and LVGP3x-CD8 vectors transduce target cells specifically compared to a broad tropism VSVG lentiviral vector, demonstrating that LVGP envelope glycoproteins mutations targeting residues important for Lassa target binding can be used in association with membrane anchored nanobodies to target transduction to cells of specific cell type.
Fig. 22 shows graphs of the quantification by flow cytometry of the percentage transduction in vitro of human PBMCs transduced with GFP-expressing CD8-targeted lentiviral vectors pseudotyped with WT Lassa GP protein or Lassa GP mutants (Lassa Y150A; Lassa H141A, F147A;Lassa Y150A, F446L). Vectors were used for in trio transduction at MOI 10 (G, Lassa Y150A; Lassa H141A, F147A; Lassa Y150A, F446L) or MOI 2 (WT) based on titration performed on SupT1. The results represent the mean±SEM of transduction of PBMCs for three different human donors. These results demonstrate that whereas mutation F446L abrogates the fusion ability of the Lassa GP protein, the Lassa WT, Lassa Y150A, or Lassa H141A, F147A pseudotyping enables specific and persistent transduction of CD8+ cells by the CD8 targeted vectors.

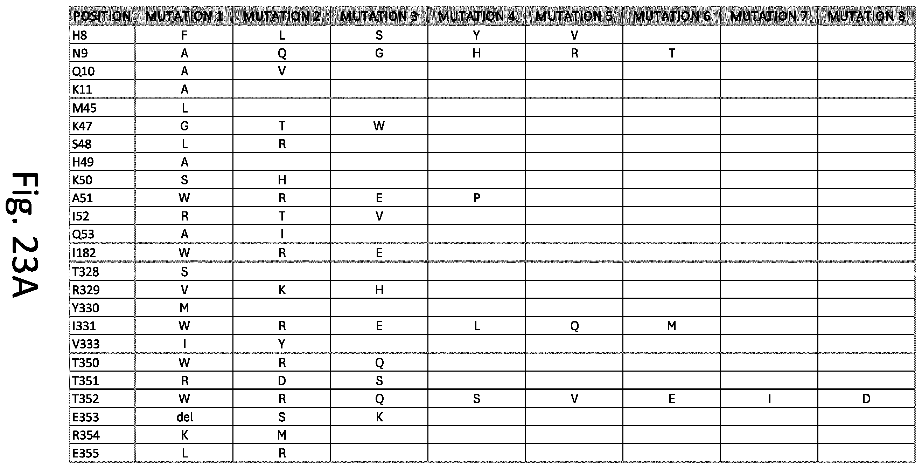

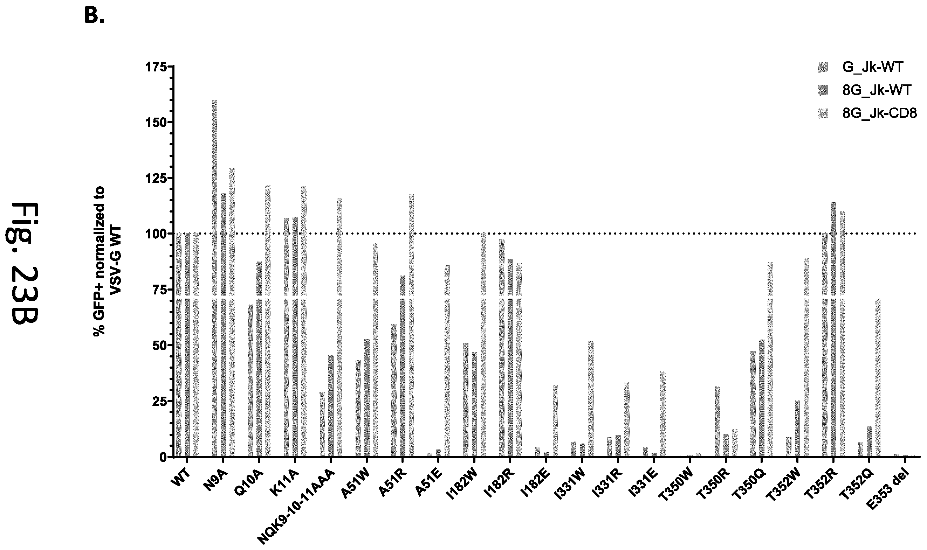

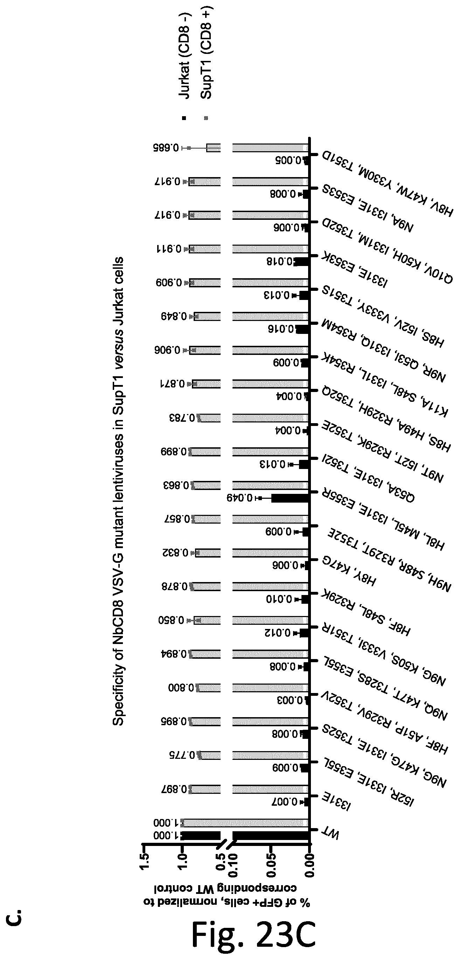

Fig. 23 Shows data supporting the selection and testing of mutants of VSVG to identify point mutations which abrogate VSVG binding to LDLR and promote target specific transduction of VHH targeted eLV. (A) Table of mutated position in the original Indiana Strain Vesicular Stomatitis Virus Glycoprotein (VSV-G, Accession number P03522 on UniProtKB). 24 positions identified on the available crystal structure of VSV-G were mutated to sterically hinder VSV-G binding to its natural ligand, LDL-R. Single letter amino acid code was used (G: Gly, A: Ala, L: Leu, M: Met, F: Phe, W: Trp, K: Lys, Q: Gln, E: Glu, S: Ser, P: Pro, V: Val, I: Ile, C: Cys, Y: Tyr, H: His, R: Arg, N: Asn, D: Asp, T: Thr, del: deletion). (B). Flow cytometry analysis of wildtype Jurkat cells (CD8 negative) transduced with GFP expressing lentivirus pseudotyped with wild type or VSV-G mutant envelop proteins alone (grey) or with a VHH targeting the CD8 protein (blue), or Jurkat CD8+ (stable transduction of CD8alpha/beta heterodimer) transduced with GFP expressing lentivirus pseudotyped with wild type or VSV-G mutant envelop proteins and the VHH targeting CD8 protein (orange; last in list in the figure key as 8G_Jk-CD8). Graphs depict the % of GFP+ normalized to 100% for the wildtype VSV-G. (C). shows a graph of the quantification by flow cytometry of the percentage transduction of CD8a target negative cell lines (Jurkat WT) versus CD8a positive cell line (Sup-T1) using broad tropism VSVG lentiviral vector (WT) and CD8a targeted lentiviral vectors pseudotyped with mutants of VSVG designed to abrogate tropism of the VSVG protein.

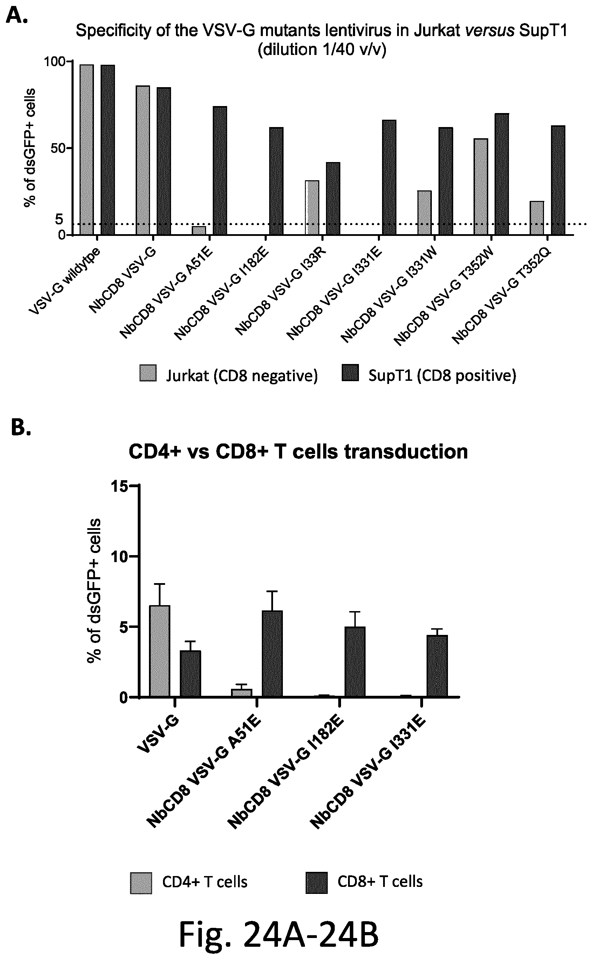

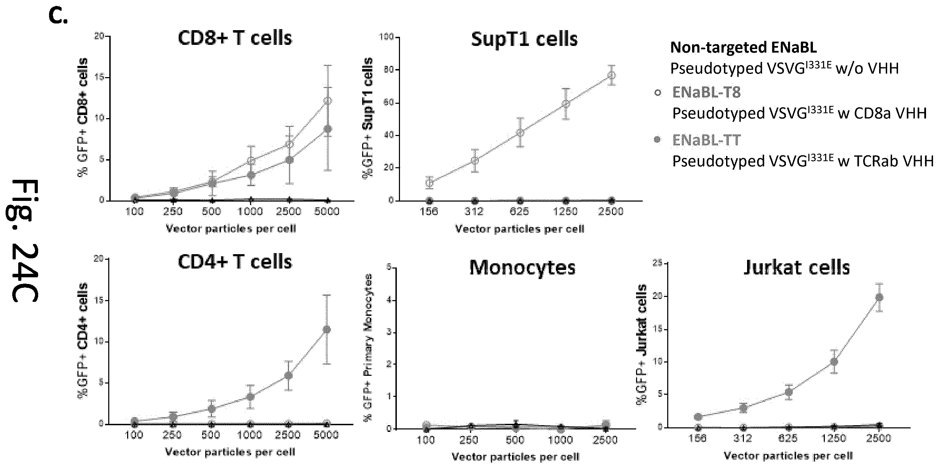

Fig. 24 shows a graph of the quantification by flow cytometry of the percentage transduction of CD8a target negative and positive cell lines, as well as primary CD4+ and CD8+ human T lymphocytes with CD8a VHH targeted vectors pseudotyped with different VSVG mutants designed to abrogate tropism of the VSVG protein. (A) CD8a target negative cell lines (Jurkat WT) versus CD8a positive cell line (Sup-T1) or (B) activated primary CD4+ and CD8+ human T lymphocytes were transduced using a broad tropism VSVG lentiviral vector (WT) and CD8a targeted lentiviral vectors pseudotyped with VSVG mutants and GFP expression was quantified by flow cytometry at 3 days post-transduction. This result shows that the three CD8 lentiviral vectors pseudotyped with mutant VSVG glycoproteins transduce target cells specifically compared to a broad tropism VSVG lentiviral vector. This demonstrates that A51E, I182E and I331E mutations of the VSVG protein abrogate the broad tropism targeting function of VSVG and support VHH mediated targeting to transduce marker positive cell types. (C) Shows a graph of the quantification by flow cytometry of the percentage transduction of CD8 target positive cells (CD8+ primary human T cells, SupT1 cells), TCR target positive cells (CD4/CD8+ primary human T cells, Jurkat cells), CD8 target negative cells (CD4+ primary human T cells, primary human monocytes and Jurkat cells) and TCR target negative cells (SupT1 cells, primary human monocytes) using CD8a or TCRab targeted lentiviral vectors pseudotyped with the targeting deficient mutant VSVGI331E (G1x). CD3/28 activated human CD4/CD8 primary human T cells, isolated primary monocytes, and the T cell lymphoblastic cell lines (Jurkat and SupT1) were transduced with increasing numbers of vector particles of non-targeted G1x eLV, G1x CD8 eLV or G1x TCRab eLV all pseudotyped with VSV-GI331E. The percentage of transduced cells was assessed 7 days post transduction by flow cytometry. This result shows that pseudotyping a CD8a VHH or TCRab VHH targeted eLV vectors (ENaBL-T8 and ENaBL-TT respectively) with VSV-GI331E induced specific transduction of CD8a expressing cells (CD8+ T cells, SupT1) and TCRab expressing cells (CD4+ T cells, CD8+ T cells, Jurkat) respectively, with levels of transduction of target negative cells <1%. Importantly, there was no detectable transduction of non-targeted (VHH deficient) ENaBL vector pseudotyped with VSV-GI331E in target positive cells. The results represent the mean±SEM of two separate studies using two individual human PBMCs donors.

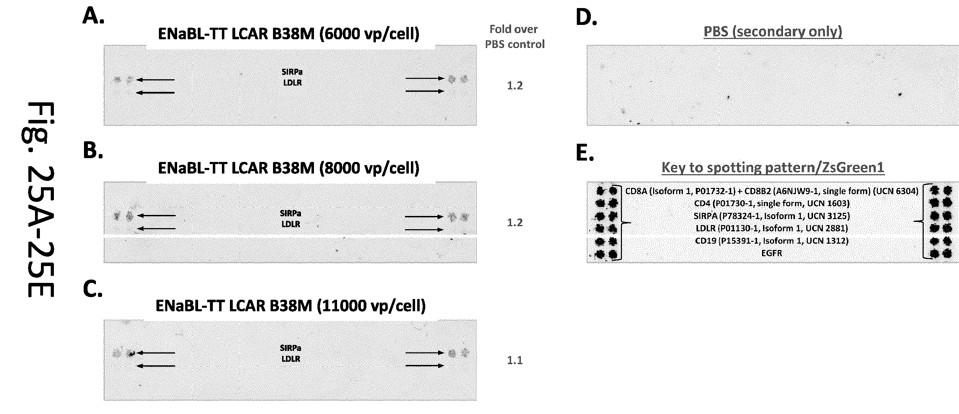

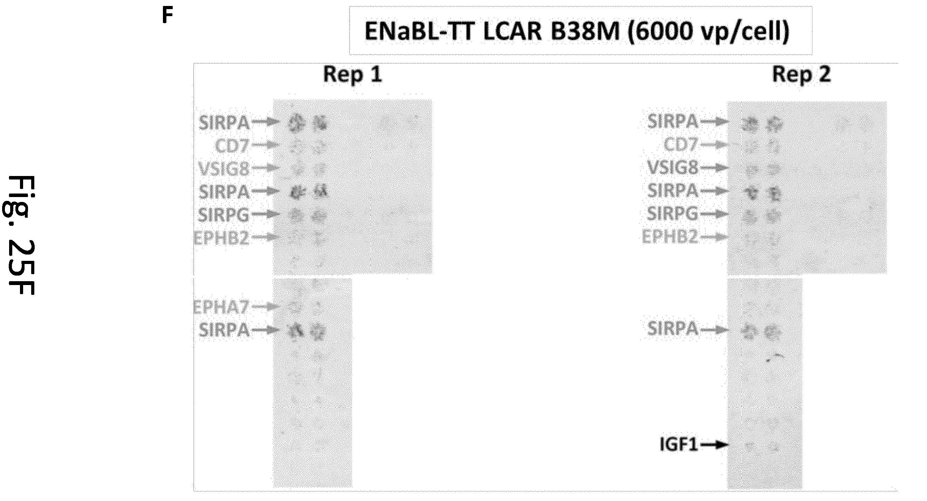

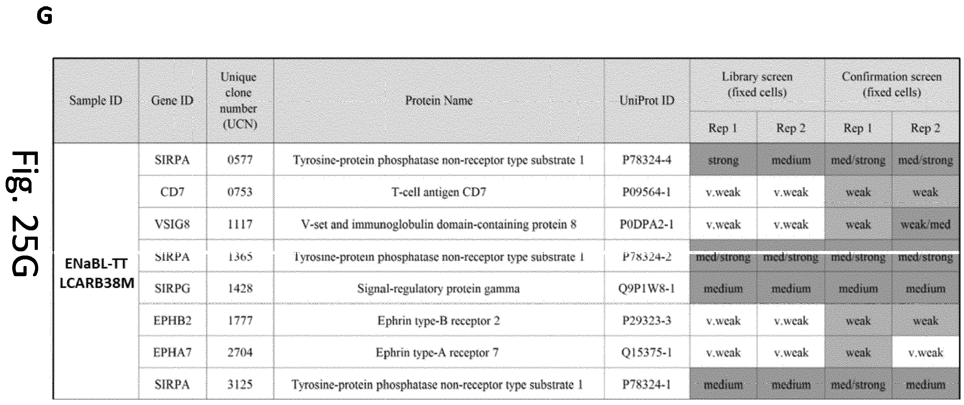

Fig. 25 Shows the binding of TCRab VHH VSVGI331E eLV vector particles to a Retrogenix cellular microarray of HEK293T cells expressing surface human proteins to assess the potential off target binding of the vector. TCRab VHH VSV-GI331E eLV encoding for the BCMA CAR was seeded at 3 different physical MOIs (6000, 8000, 11000 vector particles per cell, A., B., C., respectively) on fixed HEK293 microarrays expressing six different targets (SIRPa, CD19, CD8A+CD8B2 heterodimer, CD4, LDLR and EGFR) and a fluorescently labelled anti-VHH antibody was used to detect binding of the vector to selected targets. No signal was detected when the array was probed with vehicle (PBS, D.) and expression of each target was confirmed with a transfection control (E.). The Retrogenix Cell Microarray does not include HEK293 expressing the TCRab complex (which requires the co-expression of multiple proteins to be adequately expressed at the membrane). SIPRa, the receptor for CD47 at the surface of the vector was used as a positive control. The results indicate that TCRab VHH VSV-GI331E eLV binds to HEK293 cells expressing SIRPa, while no binding is observed to CD19, CD8A+ CD8B2 heterodimer, CD4 and EGFR. A signal just above background was detected for LDLR, which was not reproduced in the full library screen, further supporting targeting deficiency of the VSV-GI331E mutant. (F-G) Based on these results from A.-E. and the signal intensity compared to the background, 6000 vector particles/cell was selected for the full library screen. TCRab VHH VSV-GI331E eLV binding was tested on the full cellular microarray of HEK293 cells individually expressing 6105 full-length human plasma membrane proteins, and cell surface-tethered human secreted proteins, plus a further 400 human surface protein heterodimers (Retrogenix Cell Microarray Technology). As shown in Figure 25 F, G, the result of 2 repeats of the full library screen and a confirmation screen indicates that TCRab VHH VSV-GI331E eLV binds specifically to SIRPa (isoforms 1, 2 and 4), with very weak/weak binding for CD7, VSIG8, SIRPG, EPHB2 and EPHA7. In conclusion, the data obtained from the Retrogenix cell microarray indicates that the TCRab VHH VSV-GI331E eLV vector has minimal off target binding.

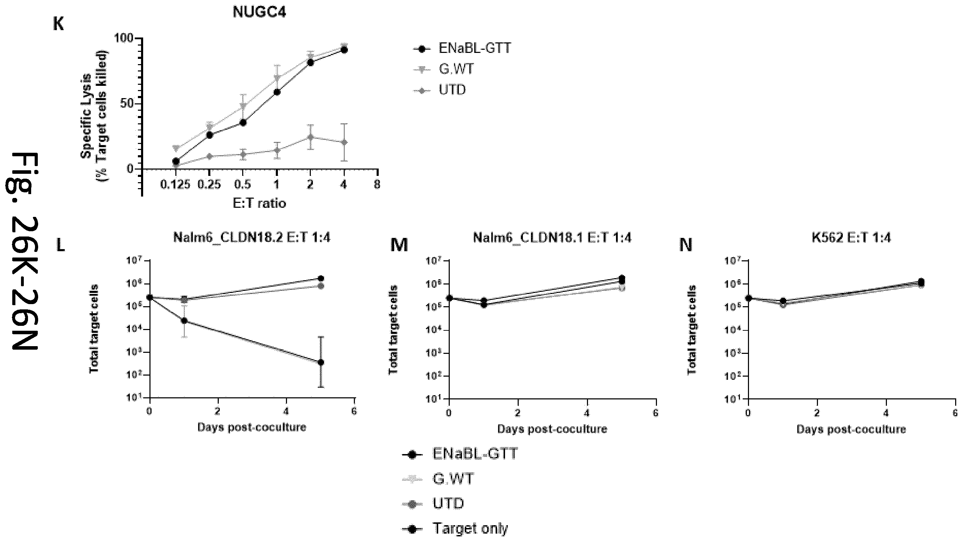

Fig. 26 shows graphs demonstrating production of functional BCMA CAR-T cells in vitro using TCRab VHH VSV-GI331E eLV transduction of resting human PBMCs. (A) 1x106 unstimulated PBMC from 2 independent donors were transduced with BCMA CAR-expressing lentiviral vectors targeted by TCRab_VHH and pseudotyped with VSV-GI331E, at a fixed multiplicity of infection of 0.4 transducing units per cell. CAR expression was assessed by flow cytometry using BCMA-Fc to reveal CAR+ cells on Day 7 and Day 11 post-transduction. (B) T cell expansion was also assessed by flow cytometry, compared to PBMC treated with anti-CD3/CD28 (UTD TransAct) or untreated (UTD Non-activated) as positive and negative controls, respectively, for cell activation and expansion. CAR-T cell counts are shown in (C.), where approximately 10-fold as many CAR+ T cells were obtained by Day 11 post-transduction compared to the starting PBMC number. At Day 11 post-transduction, Cocultures were then carried out with various target cells expressing GFP, at effector-to-target (E:T) ratios based on CAR+ cells. CAR-T cells were normalized with untransduced PBMC from each donor in order to achieve equivalent T cell counts under each E:T ratio between BCMA_CAR, BCMA_CAR_2, and untransduced (UTD) TransAct-treated PBMC controls, and cytotoxicity of target cells was assessed by flow cytometry. Two experiments were separately performed, one with BCMAHigh NCI-H929 target cells and BCMANeg K562 cells (D-F), as well as with BCMALow Nalm6 target cells and BCMANeg K562 cells (G-H). BCMA_CAR and BCMA_CAR_2 T cells demonstrated no background cytotoxicity of K562 cells (D), while effectively lysing BCMAHigh NCI-H929 cells (E) under non-challenging E:T ratios. Under more challenging conditions of a low E:T ratio with BCMAHigh NCI-H929 cells (F), or coculture with BCMALow Nalm6 cells (H), the more effective BCMA CAR_2 demonstrated control of target cell growth. (I) IL2 production was also assessed in supernatants from cocultures day 1 post-coculture by titration on HEK_Blue_IL2 cells, demonstrating clear IL2 production in coculture with BCMAHigh NCI-H929 target cells. Results represent the mean±SEM for linear scaled graphs, or the geometric mean ± geometric SD for logarithmic-scaled graphs, of two independent PBMC donors (n = 2). Overall, these results demonstrate production of polyfunctional CAR-T cells by transduction of resting human PBMCs using TCRab VHH VSV-GI331E eLV vectors. (K-N) shows graphs demonstrating production of functional CLDN18.2 CAR-T cells using TCRab VHH VSV-GI331E eLV vectors. 1x106 unstimulated PBMC from 2 independent donors were transduced with CLDN18.2 CAR-expressing lentiviral vector carrying the TCRab_VHH targeting molecule and pseudotyped with VSVgI331E (ENabL-GTT). Alternatively, 1x10⁶ PBMC were stimulated with TransAct, and transduced 24h later with CLDN18.2 CAR-expressing lentiviral vector pseudotyped with VSVg (G.WT). Cocultures were then carried out with various target cells expressing luciferase, at various effector-to-target (E:T) ratios based on CAR+ cells. CAR-T cells were normalized with untransduced TransAct-activated PBMC from each donor in order to achieve equivalent T cell counts under each E:T ratio between ENabL-GTT, G.WT, and untransduced (UTD) TransAct-treated PBMC controls. Cytotoxicity of target cells was assessed 18 hours post-coculture by quantification of the reduction in luciferase signal from natively CLDN18.2+ target cells NUGC4 (K). ENaBL-GTT and G.WT CAR T cells demonstrated equivalent cytotoxicity of NUGC4 cells. Cocultures were also carried out on Day 9 post-transduction / activation with various target cells expressing GFP, at effector-to-target (E:T) ratios based on CAR+ cells. CAR-T cells were normalized with untransduced TransAct-activated PBMC from each donor in order to achieve equivalent T cell counts under each E:T ratio between ENabL-GTT, G.WT, and untransduced (UTD) TransAct-treated PBMC controls, and cytotoxicity of target cells was assessed by flow cytometry. Both ENaBL-GTT and G.WT CAR T cells effectively lysed Nalm6 cells engineered to express CLDN18.2 (L), but not negative control Nalm6 cells engineered to express CLDN18.1 (M), or K562 cells (N) Results represent the mean±SEM for linear scaled graphs, or the geometric mean ± geometric SD for logarithmic-scaled graphs, of two independent PBMC donors (n = 2). Overall, these results demonstrate production of functional CAR-T cells demonstrating equivalent using TCRab VHH VSV-GI331E eLV vectors.

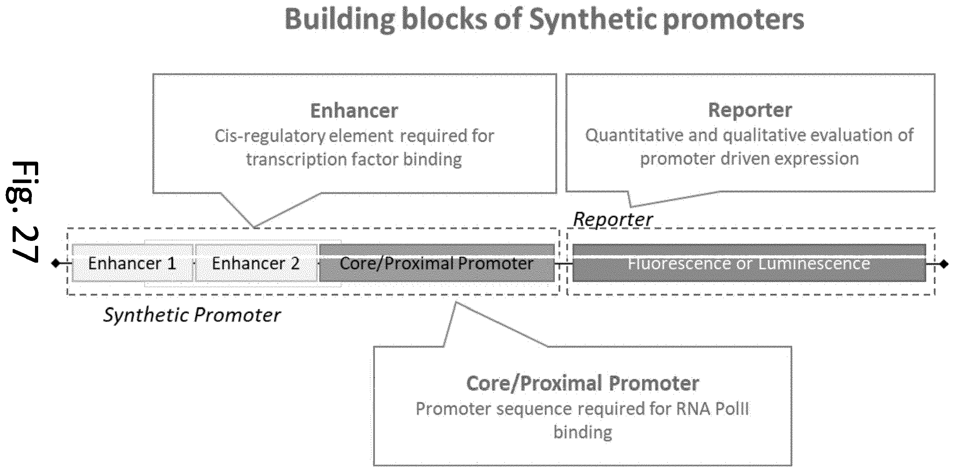

Fig. 27 shows a graphical representation of the vector used to test the cell specificity and promoter potency of the candidate synthetic promoters. The synthetic promoter portion of the vector is composed of enhancer DNA elements associated with core or proximal promoter sequences. The specificity and potency of the promoter is tested in cultured cell lines and primary cells of different origins by transduction of VSVG broad tropism lentiviral vectors. Luminescence or fluorescence readout from the expression of the reporter construct is quantified using a luminescence reader or by flow cytometry.

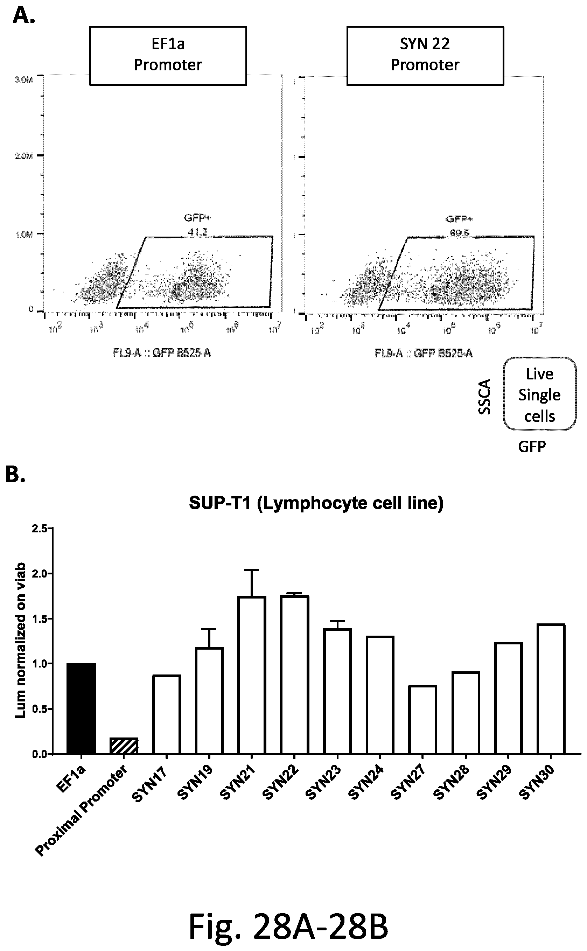

Fig. 28 shows representative flow cytometry plots and a graph of the normalized luminescence values for Sup-T1 cells transduced with VSVG lentiviral vectors expressing a luciferase/GFP dual reporter under the control of the ubiquitous promoter (EF1a), a Proximal Promoter and different synthetic promoters (FIG. 23). Sup-T1 cells were transduced in culture with an equal number of functional VSVG lentiviral vectors expressing a luciferase/GFP dual reporter under the control of different promoters. Transduction efficiency (percentage of cells expressing GFP) was assessed by flow cytometry (A.) and promoter activity was measured by the luminescence output from the luciferase reporter. (B.) This figure shows that addition of enhancer sequences significantly increases expression from the synthetic promoters, with specific synthetic constructs reaching and exceeding levels of expression from EF1a in Sup-T1 cells.

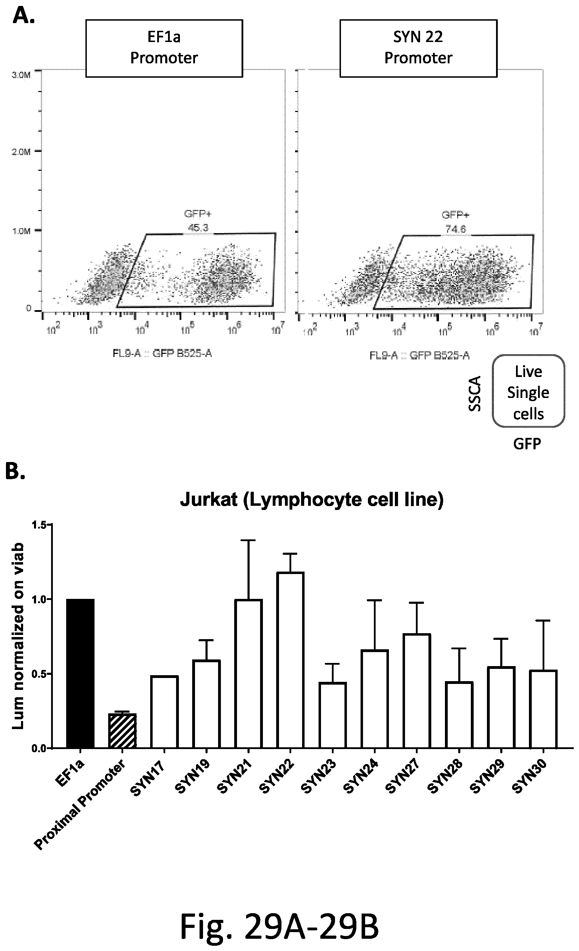

Fig. 29 Shows representative flow cytometry plots and a graph of the normalized luminescence values for Jurkat cells transduced with VSVG lentiviral vectors expressing a luciferase/GFP dual reporter under the control of the ubiquitous promoter (EF1a), a Proximal Promoter and different synthetic promoters (FIG. 23). Jurkat cells were transduced in culture with equal functional titres (titers) of VSVG lentiviral vectors expressing a luciferase/GFP dual reporter under the control of different promoters. Transduction efficiency (percentage of cells expressing GFP) was assessed by flow cytometry (A.) and promoter activity was measured by the luminescence output from the luciferase reporter. (B.) These results shows that addition of enhancer sequences significantly increases expression from the synthetic promoters, with specific synthetic constructs reaching and exceeding levels of expression from EF1a in Jurkat cells.

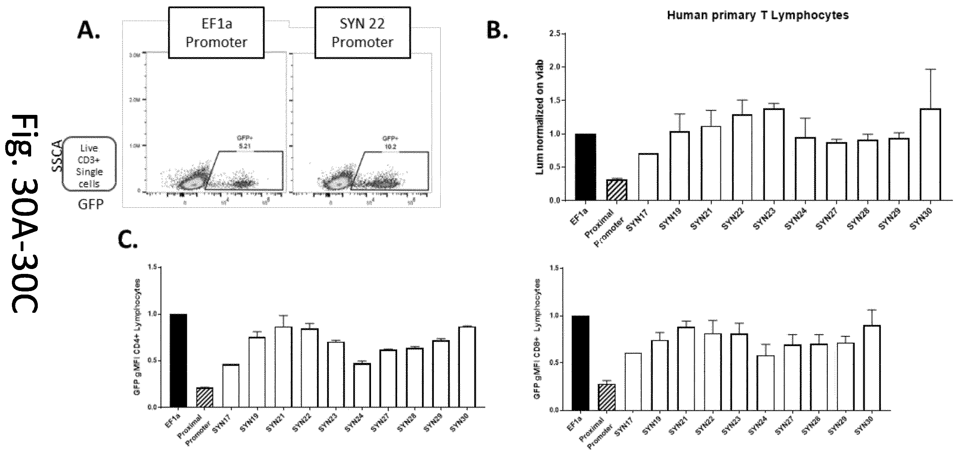

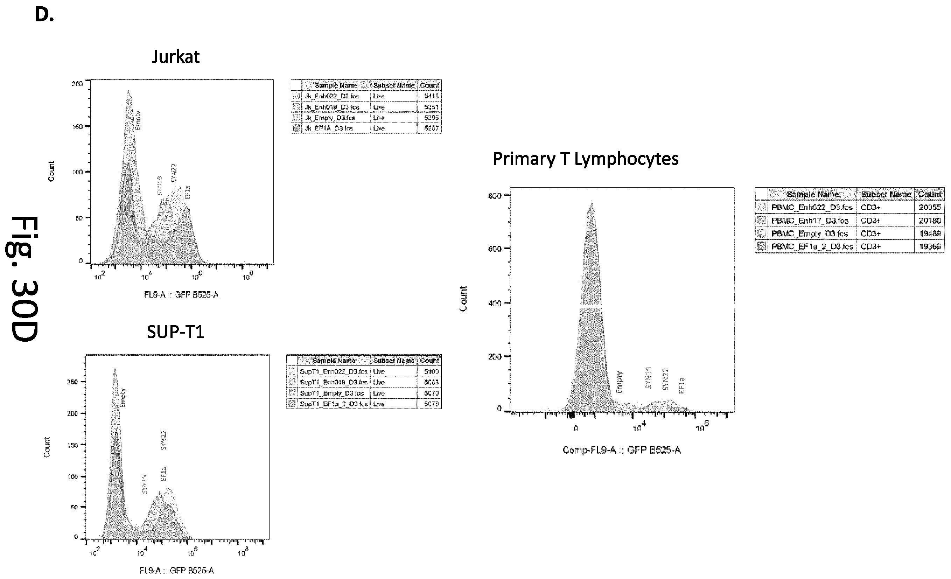

Fig. 30 shows representative flow cytometry plots and a graph of the normalized luminescence values and geometric mean fluorescence intensity (gMFI) for human primary lymphocytes cells transduced with VSVG lentiviral vectors expressing a luciferase/GFP dual reporter under the control of the ubiquitous promoter (EF1a), a Proximal Promoter and different synthetic promoters (FIG. 23). Activated human primary lymphocytes were transduced in culture with equal functional titers of VSVG lentiviral vectors expressing a luciferase/GFP dual reporter under the control of different promoters. Transduction efficiency (percentage of CD3+ cells expressing GFP) and gMFI of GFP in the CD4+ or CD8+ transduced T cell population) was assessed by flow cytometry (A. and C. respectively) and promoter activity was measured by the luminescence output from the luciferase reporter in CD3+ cells (B.). (D.) shows representative flow cytometry histogram plots lymphocyte cell lines (Jurkat and SUPT1) and primary human T cells transduced with VSVG lentiviral vectors expressing a luciferase/GFP dual reporter under the control of the ubiquitous promoter (EF1a) and two different synthetic promoters (SYN19 and SYN22). SUP-T1 cells were transduced in culture with equal functional titers of VSVG lentiviral vectors expressing a luciferase/GFP dual reporter under the control of different promoters. This figure shows that addition of enhancer sequences significantly increases expression from the synthetic promoters, with specific synthetic constructs reaching median intensity of reporter expression within less than two-fold that of EF1a in T cell lines or primary T cells.

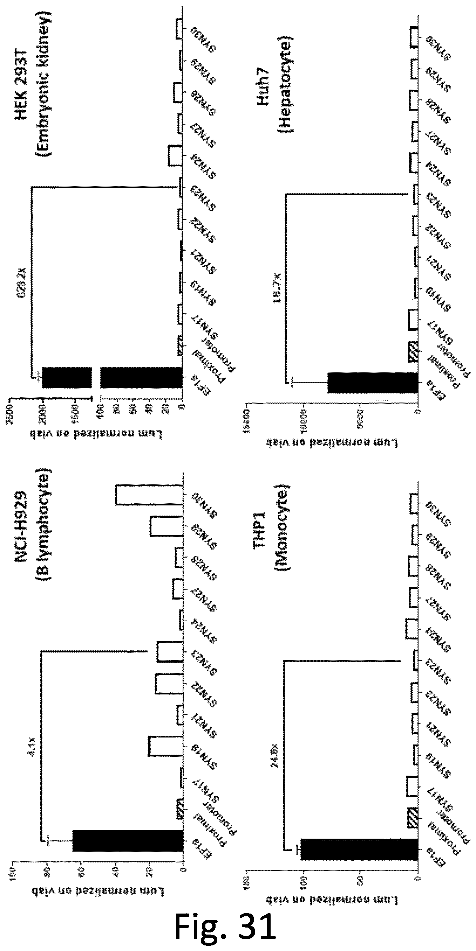

Fig. 31 shows graphs of the luminescence values (normalized for cell viability) for each for cell type transduced with VSVG lentiviral vectors expressing a luciferase/GFP dual reporter under the control of the ubiquitous promoter (EF1a), a Proximal Promoter and different synthetic promoters (FIG. 27). Cell lines of different origins were transduced in culture with equal functional titers of VSVG lentiviral vectors expressing a luciferase/GFP dual reporter under the control of different promoters. Promoter activity was measured by the luminescence output from the luciferase reporter and normalised for the viability of the cells. For the quantification of reporter activity in NCI-H929 and THP1 cells, the data is representative of 2 separate experiments and the luciferase signal was further normalized against the EF1a condition for each experiment. This result shows that the selected synthetic promoters have limited expression in non-T cell lineage cell lines compared to EF1a.

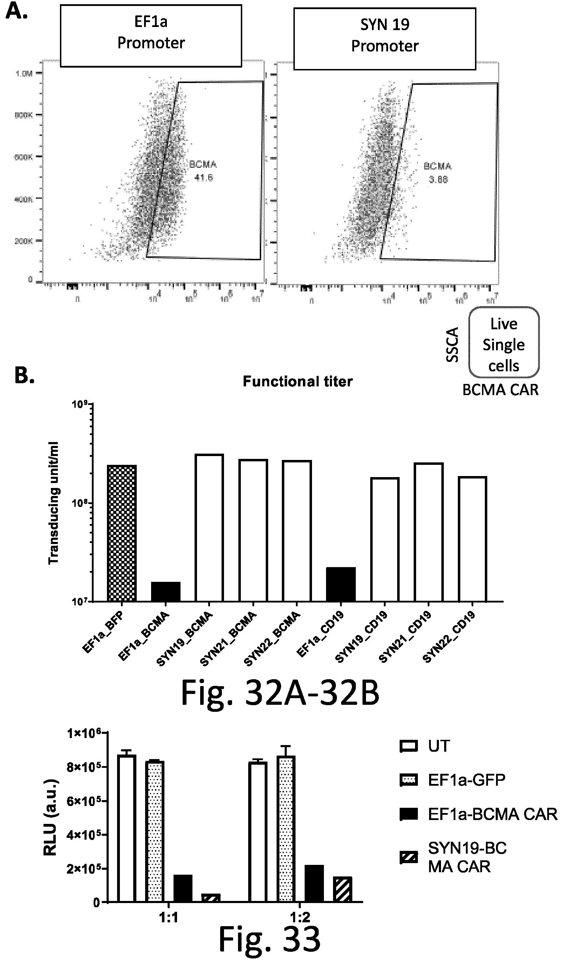

Fig. 32 shows representative flow cytometry plots for the expression of surface BCMA CAR on 293T producer cells during lentiviral production and a graph summarizing the functional titers of LVGP1x-CD8 lentiviral vectors produced for the expression of BFP, BCMA or CD19 CAR under the control of ubiquitous promoter (EF1a) or different synthetic promoters (SYN19, SYN21, SYN22). 293T producer cell line transiently transfected with lentiviral transfer and packaging plasmids were analyzed by flow cytometry for the surface expression of the BCMA CAR transgene encoded in the lentiviral transfer vector at the end of the production process. The result in A. shows that placing the BCMA CAR coding sequence under the control of the T cell specific promoter reduces by over 10-fold the surface expression of BCMA CAR on the surface of the producer cells and therefore on the viral envelope of the resulting lentiviral particles. (B.) The LVGP1x-CD8 lentiviral vectors produced in A. were tittered on target cells (Sup-T1). The result in B. demonstrates that restricting the expression of surface expressed BCMA CAR or CD19 CAR, but not that of the soluble fluorescent reporter protein BFP, during production of the lentiviral particles increase by over 10-fold the number of transduction competent lentiviral particles per ml.
Fig. 33 shows a graph of the luminescence values from NCI-H929 stably expressing luciferase and co-cultured with GFP or CAR-T lymphocytes. Primary T lymphocytes (within a mixed population of peripheral blood mononuclear cells) were transduced with GFP or BCMA CAR expressing VSVG lentiviral vectors in which the transgene is under the control of the EF1a or the T cell specific synthetic promoter (SYN19). The transduced cells were expanded and co-cultured with NCI-H929 cells stably expressing luciferase at a ratio of 1:1 or 1:2 (effector : target ratio) and luminescence from each condition was measured after 2 days of co-culture. This figure shows a significant decrease (>4 fold) in the luminescence from co-cultures of NCI-H929 cells with BCMA CAR-T cells compared to untransduced T cells or T cells expressing GFP, indicating loss of NCI-H929 cells in presence of BCMA CAR-T cells. This result demonstrates that BCMA CAR-T cells expressing the CAR construct under the control of the T cell synthetic promoter SYN19 are as effective as CAR-T cells expressing the CAR construct under the control of the EF1a promoter.

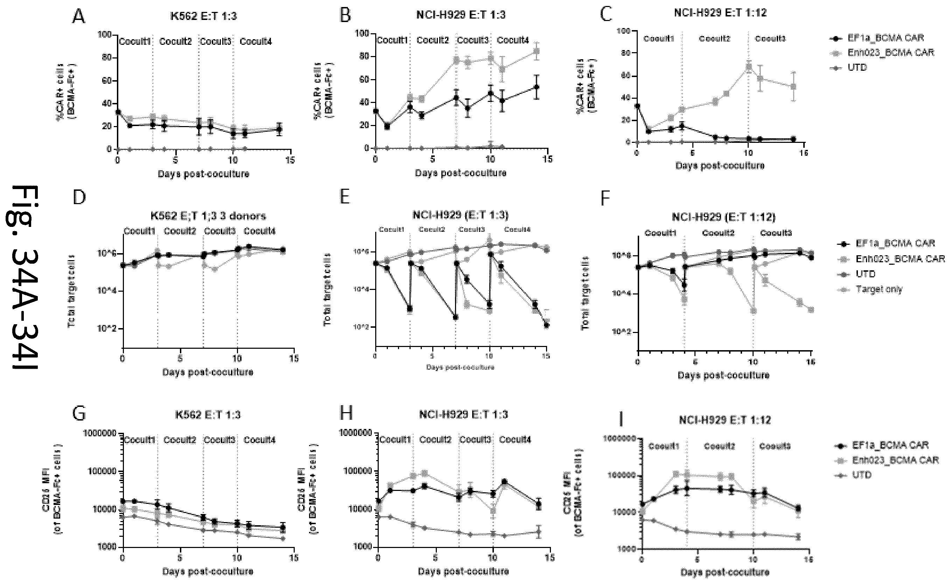

Fig. 34 shows graphs demonstrating advantageous CAR-T phenotype of BCMA CAR under the control of the T cell-specific SYN23 promoter relative to the constitutive EF1a promoter. Three independent donors of PBMC were activated with anti-CD3/CD28 and transduced day 2 post-activation with VSVG pseudotyped lentiviral vectors. CAR expression levels were assessed day 7 post-activation. Cocultures were then carried out with various target cells expressing GFP, at effector-to-target (E:T) ratios based on CAR+ cells. CAR-T cells were normalized with untransduced PBMC from each donor in order to achieve equivalent T cell counts under each E:T ratio between EF1a_BCMA CAR, SYN23_BCMA CAR, and untransduced (UTD) PBMC controls. Repeated cocultures were performed by adding additional BCMAHigh NCI-H929 and BCMANeg K562 target cells at various time points (dotted lines in graphs). CAR expression was assessed by flow cytometry at various time points during coculture, demonstrating a lack of expansion of CAR+ cells in coculture with BCMANeg K562 cells (A), but enhanced expansion of CAR+ cells with SYN23_BCMA CAR relative to EF1a_BCMA CAR in coculture with BCMAHigh NCI-H929 target cells (B, C). CAR-mediated cytotoxicity was also assessed by flow cytometry at various points during the cocultures, demonstrating no CAR-mediated cytotoxicity of BCMANeg K562 target cells (D), and equivalent cytotoxicity of BCMAHigh NCI-H929 target cells between SYN23_BCMA CAR relative to EF1a_BCMA CAR under nonchallenging E:T 1:3 conditions (E). Notably, SYN23_BCMA CAR-T cells demonstrated enhanced target cell lysis under challenging E:T 1:12 conditions (F), with EF1a_BCMA CAR-T cells failing to control the target cell growth beginning in Coculture 2. CAR-T activation was also assessed by flow cytometric quantification of CD25 expression, with no specific activation of CAR-T cells by BCMANeg K562 target cells (G); however, EF1a_BCMA CAR-T cells demonstrated higher background activation in the absence of BCMA antigen. Conversely, in the presence of BCMAHigh NCI-H929 target cells, Enh023_BCMA CAR-T cells demonstrated increased BCMA-specific activation in Cocultures 1&2 (H, I). Results represent the mean±SEM for linear scaled graphs, or the geometric mean ± geometric SD for logarithmic-scaled graphs, of three independent PBMC donors (n = 3). Overall, these results indicate lower background activation, and more sustained CAR expression and function with CAR expression driven by SYN23 promoter relative to EF1a.

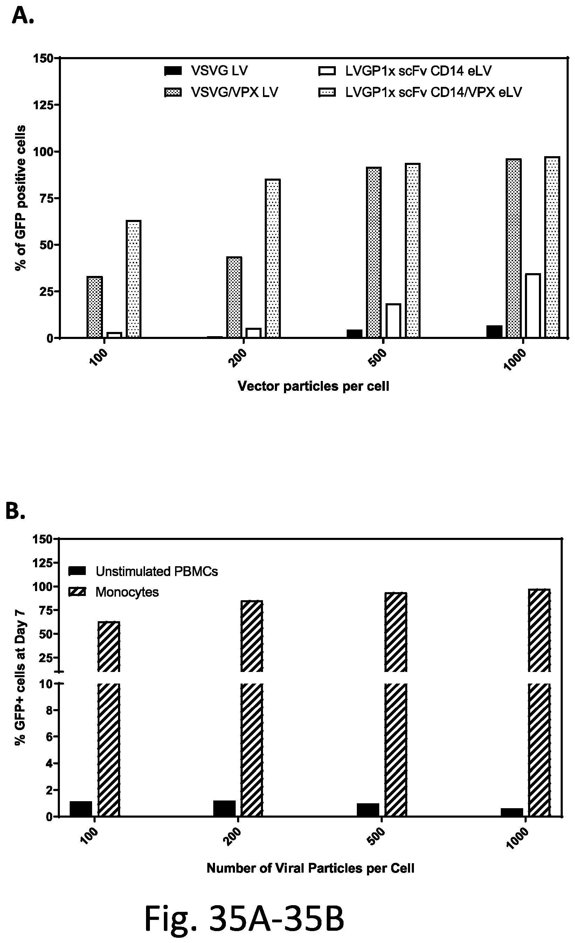

Fig. 35 shows a graph of the quantification by flow cytometry of the percentage transduction of non-stimulated PBMCs and isolated monocyte using an LVGP1X eLV displaying a membrane anchored scFv specific for CD14. Human PBMCs and isolated primary monocytes were transduced with increasing number of LVGP1X-scFVCD14 eLV vectors (physical particles) and percentage of transduced cells was assessed 7 days post transduction by flow cytometry. This result shows that directing LVGP1x eLVs to myeloid cells, using an envelope anchored binder targeting CD14, enables specific transduction of a monocytes.

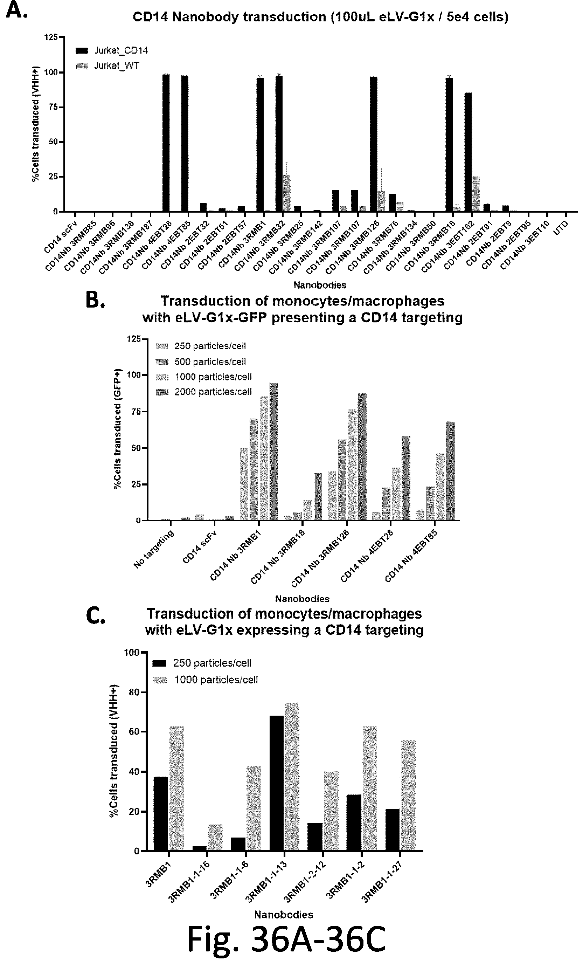

Fig. 36 Shows a graph of the quantification by flow cytometry of the percentage transduction of CD14 target positive cells (monocytes/macrophages and Jurkat cell line stably expressing CD14) and CD14 target negative cells (Jurkat WT cell line and SUP-T1) using lentiviral vectors pseudotyped with the targeting deficient mutant VSVGI331E (G1x) and different CD14 VHH or affinity matured 3RMB1 CD14 VHH. A. 50.000 Jurkat WT and Jurkat CD14 were transduced with 100µl of unconcentrated CD14 scFv or VHH targeted lentiviral vectors pseudotyped with G1x and expressing either the CD14 scFv or the different CD14 VHH. Transduction efficiency was assessed 7 days post transduction by flow cytometry, by staining the Jurkat cells with an anti-VHH antibody. The results represent the mean ±SD of two different experiments. B. 100.000 monocytes isolated from one healthy donor were transduced with 250, 500, 1000 and 2000 vector particles of lentiviral vectors pseudotyped with the targeting deficient mutant VSVGI331E (G1x) with no targeting or expressing either the CD14 scFv or the different CD14 VHH. The percentage of transduced cells was assessed 7 days post transduction by flow cytometry. C. 100.000 monocytes isolated from one healthy donor were transduced with 250 and 1000 vector particles of lentiviral vectors pseudotyped with the targeting deficient mutant VSVGI331E (G1x) with no targeting or expressing either the CD14 scFv or the different affinity matured 3RMB1 CD14 VHH. Transduction efficiency was assessed 7 days post transduction by flow cytometry, by staining the macrophages with an anti-VHH antibody. These result show that directing G1x eLVs to myeloid cells, using an envelope anchored binder targeting CD14, enables specific transduction of a monocytes.

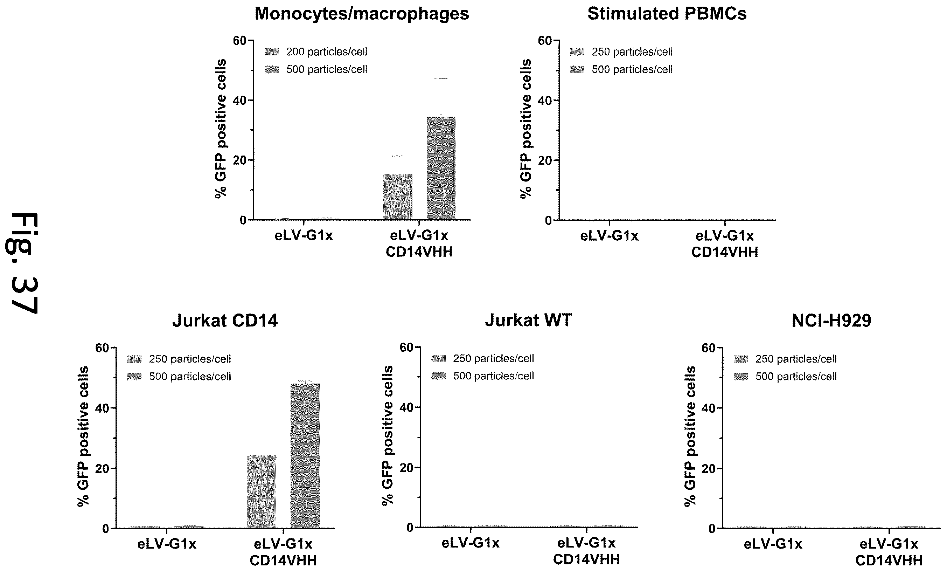

Fig. 37 Shows a graph of the quantification by flow cytometry of the percentage transduction of CD14 target positive cells (Monocytes/Macrophages, Jurkat CD14 cell line) and CD14 target negative cells (Stimulated PBMCs, Jurkat WT cell line and NCI-H929 cells) using lentiviral vectors pseudotyped with the targeting deficient mutant VSVGI331E (G1x) and the CD14 VHH or with G1x alone. Monocytes/macrophages isolated from three separate healthy donors, CD3/28 activated human PBMCs, Jurkat lymphoblastic cell line wild type or stably expressing CD14 and the B cell lymphocytic cell line NCI-H929 were transduced with 250 or 500 vector particles of non-targeted eLV, CD14 targeted eLV, both pseudotyped with VSVGI331E and expressing the fluorescent reporter GFP. The percentage of transduced cells was assessed 7-14 days post transduction by flow cytometry. The results represent the mean ±SD of three donors and two-three separate batches of viruses. These result indicate that pseudotyping a CD14 VHH targeted eLV vectors with VSVGI331E induces specific transduction of CD14 expressing cells (primary monocytes/macrophages and Jurkat cells stably expressing CD14), with levels of transduction of target negative cells <1% (NCI, Jurkat and primary PBMCs). Importantly, there was no detectable transduction of non-targeted (VHH deficient) eLV vector pseudotyped with VSVGI331E in target positive cells.

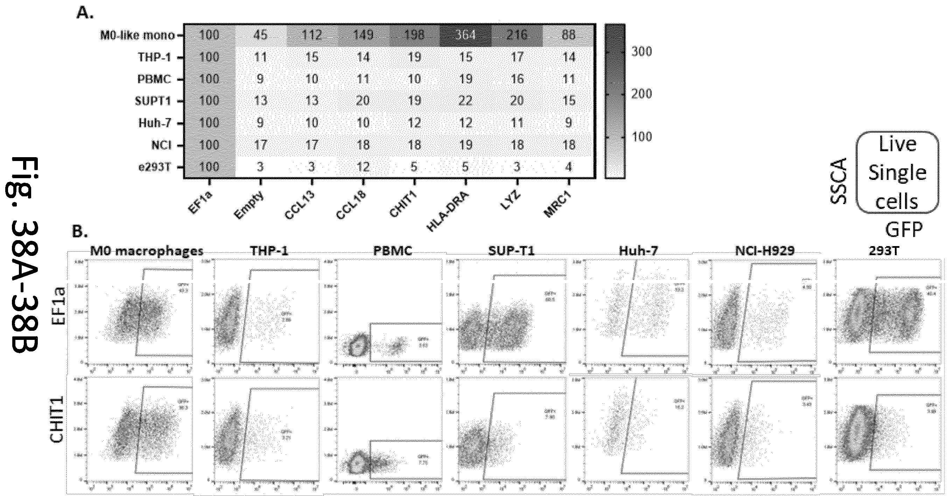

Fig. 38 shows the level of GFP expression, assessed by flow cytometry as the geometric mean fluorescent intensity, of cell types of different lineages (myeloid, T cell, hepatocyte, B cell, kidney) transduced with VSVG pseudotyped LVs encoding a reporter under the control of 6 proximal promoters derived from macrophage-specific genes (See Fig. 27). Primary monocytes, THP-1, primary PBMCs, SupT-1, Huh-7, NCI and e293T cells were transduced with VSVG broad tropism lentivirus encoding a luciferase-eGFP transgene under the control of EF1a, no promoter (empty) or 6 different proximal promoters. The monocytes were transduced and differentiated in MO-like macrophages in presence of 50ng/ml of M-CSF for 7 days. The PBMC were stimulated 2 days with CD3/CD28 before transduction and cultured in presence of 30ng/ml of human IL-2. A. Percentage of GFP gMFI normalised to EF1a for the empty and 6 proximal promoters. B. FACS plot data of each tested cell type for EF1a and CHIT1 proximal promoter. These results show that reporter expression induced by the myeloid specific core promoters selected induce specific expression in primary macrophages at a level equivalent or above that of the EF1a promoter.

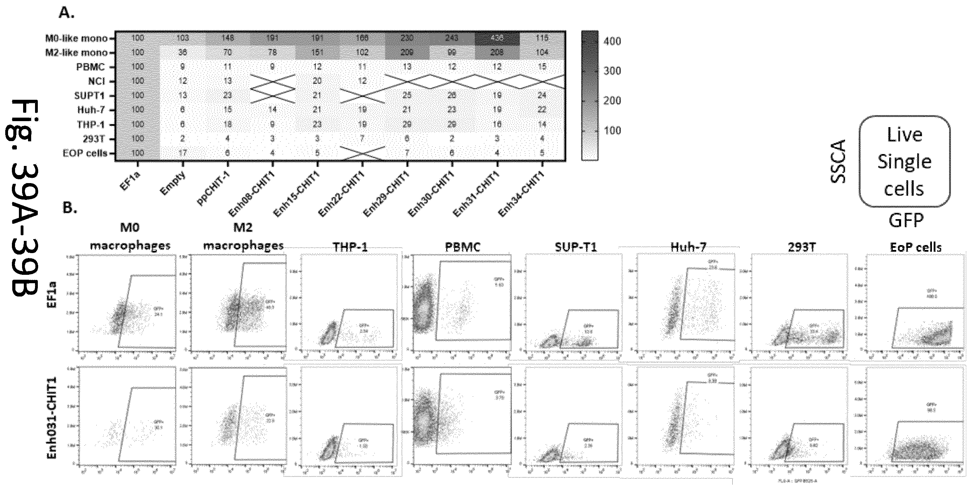

Fig. 39 shows the level of GFP expression, assessed by flow cytometry as the geometric mean fluorescent intensity, of cell types of different lineages (myeloid, T cell, hepatocyte, B cell, kidney) transduced with VSVG pseudotyped LVs encoding a reporter under the control of 7 synthetic promoters derived from macrophage-specific genes (See Fig. 27). Primary monocytes, THP-1, primary PBMCs, SupT-1, Huh-7, NCI and e293T cells were transduced with VSVG broad tropism lentivirus encoding a luciferase-eGFP transgene under the control of EF1a, no promoter (empty) or 6 different proximal promoters. Monocytes were transduced and differentiated in MO-like macrophages in presence of 50ng/ml of M-CSF for 7 days, then in M2-like macrophages in presence of 50ng/ml M-CSF, 20ng/ml IL-4 and 20ng/ml IL-10 for 2 days. PBMC were stimulated 2 days with CD3/CD28 before transduction and cultured in presence of 30ng/ml of human IL-2. A. Percentage of GFP gMFI normalised to EF1a for the empty, CHIT1 core promoter and 7 synthetic promoters incorporating the CHIT1 proximal promoter fused to enhancer sequences. B. FACS plot data of each tested cell type for EF1a and Enh031-CHIT1 synthetic promoter. These results show that addition of enhancer sequences to the CHIT1 core promoter increase expression levels of the reporter while preserving the specificity of expression in myeloid cells.

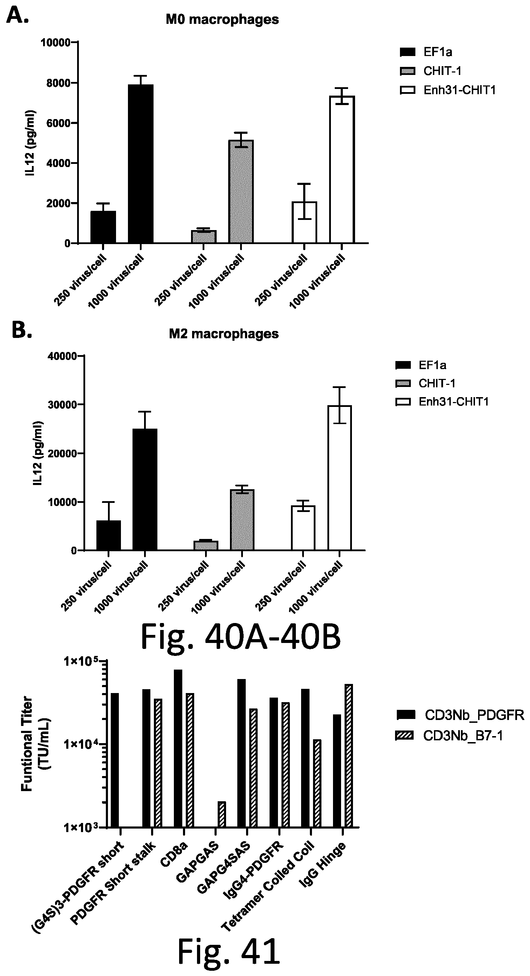

Fig. 40 shows graphs of the quantification of human IL-12 secretion in the supernatant of M0 or M2-like macrophages derived from healthy donor primary monocytes and transduced with VSVG LV encoding h-IL12 transgene under the control EF1a, CHIT1 proximal promoter or Enh031-CHIT1 synthetic promoter. The results in A show the h-IL-12 concentration in MO-like macrophages supernatant 7 days post transduction. The monocytes were transduced and differentiated in M0-like macrophages in presence of 50ng/ml of M-CSF for 7 days. The results in B show the IL-12 concentration in M2-like macrophage supernatant 9 days post-transduction. The monocytes were transduced and differentiated in MO-like macrophages in presence of 50ng/ml of M-CSF for 7 days then in M2-like macrophages in presence of 50ng/ml M-CSF, 20ng/ml IL-4 and 20ng/ml IL-10 for 2 days. The experiment was performed with 2 batches of lentivirus per conditions. These results show that the Enh31-CHIT1 myeloid specific synthetic promoter induces levels of expression of a functional transgene equivalent to those induced by the constitutive promoter EF1a.
Fig. 41 shows a graph of the quantification by flow cytometry of the percentage transduction efficiency on Jurkat cells of LVGP1X-VHHCD3 vectors where the CD3e VHH is anchored on the envelope within different scaffolds. Jurkat WT cells were transduced with different dilutions of the LVGP1X-VHHCD3 vectors each displaying the CD3e VHH in different scaffolds and the functional titre of each vector type was quantified by the percentage transduced cells averaged from at least two linear dilutions. The different scaffolds anchor the CD3e VHH to the envelope either through the transmembrane domain of PDGFR or B7-1 which are linked to the VHH through sequences of varying length and flexibility (e.g. CD8a hinge region or the PGFR receptor proximal membrane region, see X axis labels). This result shows that the scaffold anchoring the VHH to the membrane affects the functional titre of the vector. In the case of the CD3e VHH, the anchoring scaffold containing the PDGFR transmembrane domain and the CD8a hinge region provided the architecture which achieved the highest functional titre.

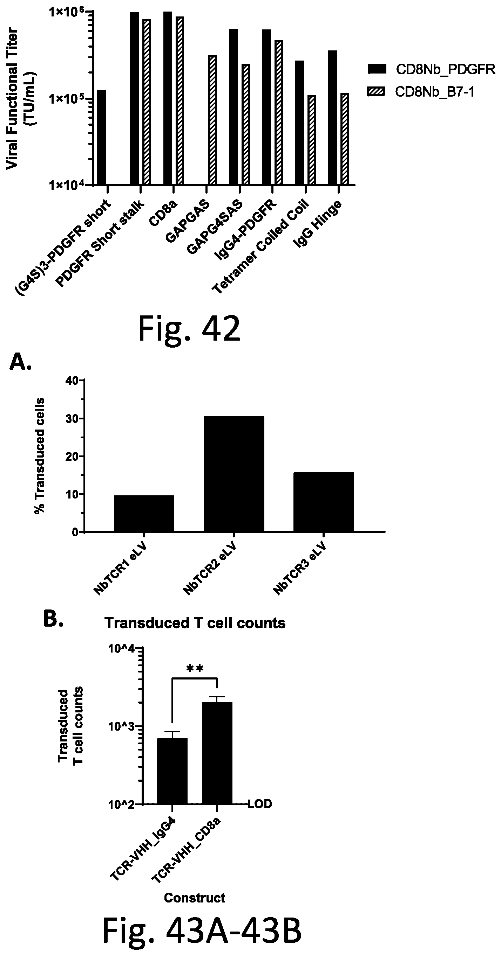

Fig. 42 shows a graph of the quantification by flow cytometry of the percentage transduction efficiency on Sup-T1 cells of LVGP1X-VHHCD8a vectors where the CD8a VHH is anchored on the envelope within different scaffolds (see Fig. 32). Sup-T1 WT cells were transduced with different dilutions of the each LVGP1X-VHHCD8a vectors and the functional titre of each vector type was quantified by the percentage transduced cells averaged from at least two linear dilutions. This result shows that the scaffold anchoring the VHH to the membrane affects the functional titre of the vector. In the case of the CD3e VHH, the anchoring scaffold containing the PDGFR transmembrane domain and the CD8a hinge region or the PDGFR proximal membrane region provided the architectures which achieved the highest functional titre.
Fig. 43 A. shows a graph of the quantification by flow cytometry of the percentage transduction efficiency of TCR targeted (VHHTCR eLV) on target cells (Jurkat WT). Jurkat WT cells were transduced with TCR VHH displaying vectors pseudotyped with a mutant of VSVG (K47A, R354) previously shown to abrogate the broad tropism of wild-type VSVG and where the VHH is anchored on the envelope within different scaffolds. TCR1 scaffold: PDGFR transmembrane domain and the membrane proximal region of PDGFR; TCR2 scaffold: PDGFR transmembrane domain and the membrane proximal region of IgG4; TCR3 scaffold: PDGFR transmembrane domain and a Tetramer coiled coil hinge. This result shows that the scaffold anchoring the VHH to the membrane affects the transduction efficiency of the vector. In the case of the TCR VHH, the anchoring scaffold containing the PDGFR transmembrane domain and membrane proximal region of IgG4 provided the architecture which achieved the highest transduction efficiency in association with the VSVG (K47A, R354) pseudotype. B. shows graphs demonstrating increased transduction and stimulatory capacity of TCRab-targeting lentiviruses using a CD8a hinge relative to IgG4 hinge on TCRab_VHH fusion proteins. Stable producer cell lines stably expressing the TCRab VHH in different architecture (PDGFR transmembrane domain + CD8a hinge or IgG4 hinge) were used to produce GFP-expressing lentiviruses pseudotyped with VSVgI331E envelope and targeted by the TCR VHH. 1.5x105 PBMC from 2 independent donors were then transduced with 100uL of unconcentrated lentivirus. The use of the CD8a hinge compared to the IgG4 hinge leads to higher total transduced T cells day 7 post-transduction. This result represents the geometric mean ± geometric SD of two independent PBMC donors and 2 independent lentiviral preparations (n = 4).

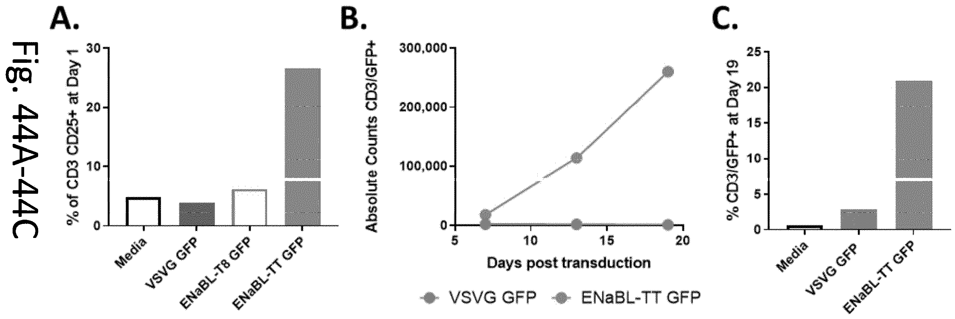

Fig. 44 Shows the quantification of the activation, expansion, and transduction of resting CD3 lymphocytes by flow cytometry after exposure to the LVGP1X-TCRab VHH or CD8 VHH vectors. Resting hPBMCs were transduced overnight with LVGP1x-TCR VHH, VSVG LV and LVGP1x-CD8a VHH eLV. This result shows that LVGP1X displaying the TCRab VHH binder induces T cell activation (A.), as shown by the increase in percentage of T cells expressing the activation markers CD25, as well as increased T cell expansion (B.) and T cell transduction (C.) compared to VSVG LV or LVGP1x-CD8a VHH eLV exposed resting PBMCs.

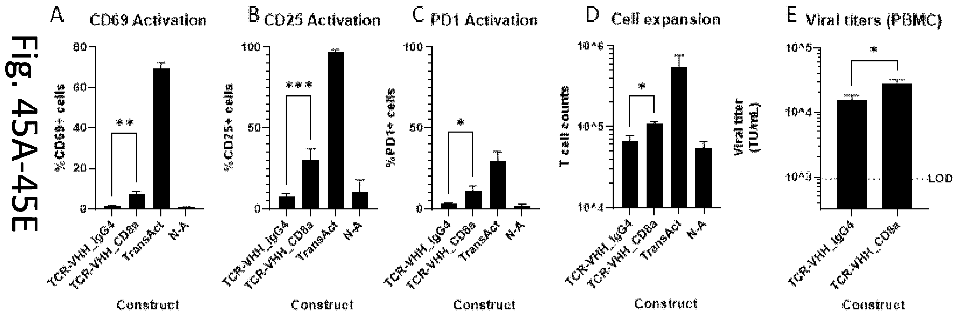

Fig. 45 shows graphs demonstrating increased transduction and stimulatory capacity of TCRab VHH-targeted lentiviruses using a CD8a hinge relative to IgG4 hinge on TCRab_VHH fusion proteins. In (A-E), where stable producer cell lines, transduced with a single lentiviral vector expressing TCRab_VHH were used to produce GFP-expressing lentiviruses pseudotyped with VSVgI331E envelope. 1.5x105 PBMC from 2 independent donors were then transduced with 100uL of unconcentrated lentivirus. Anti-CD3/CD28 (TransAct) was used as a positive control for T cell activation, and PBMC were also left untreated (N-A) to demonstrate background levels of T cell expansion and activation. The use of the CD8a hinge compared to the IgG4 hinge led to higher CD69 expression day 1 post-transduction (A), CD25 and PD1 expression day 4 post-transduction (B, C), as well as increased cell expansion (E), viral titers (F), and total transduced T cells (G) day 7 post-transduction. Results represent the mean±SEM for linear scaled graphs, or the geometric mean ± geometric SD for logarithmic-scaled graphs, of two independent PBMC donors and 2 independent lentiviral preparations (n = 4).

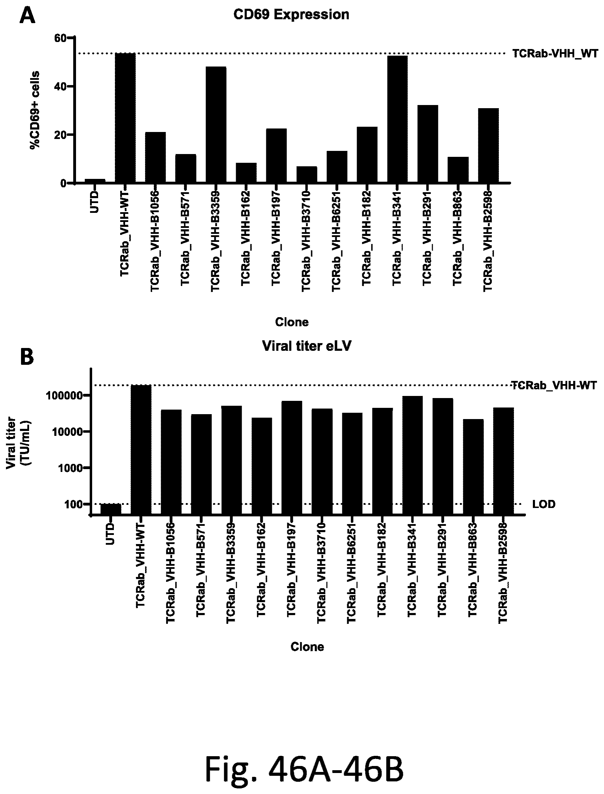

Fig. 46 shows graphs demonstrating the stimulatory and transduction-mediating capacity of lentiviral vectors targeted using various mutated TCRab_VHH binders. Producer cells were transfected with lentiviral plasmids expressing both the TCRab_VHH (wild-type or mutant), as well as TagBFP2, where the incorporation of the TCRab_VHH into the lentiviral vector was driven by transient expression of the TCRab_VHH from the lentiviral plasmid. Lentiviral vectors were pseudotyped with VSVgI331E envelope. Jurkat cells were transduced with 100uL of unconcentrated lentivirus. The stimulatory capacity of the TCRab_VHH clones was assessed by CD69 expression on Jurkat cells day 1 post-transduction. Viral titers were assessed by TagBFP2 expression day 3 post-transduction.

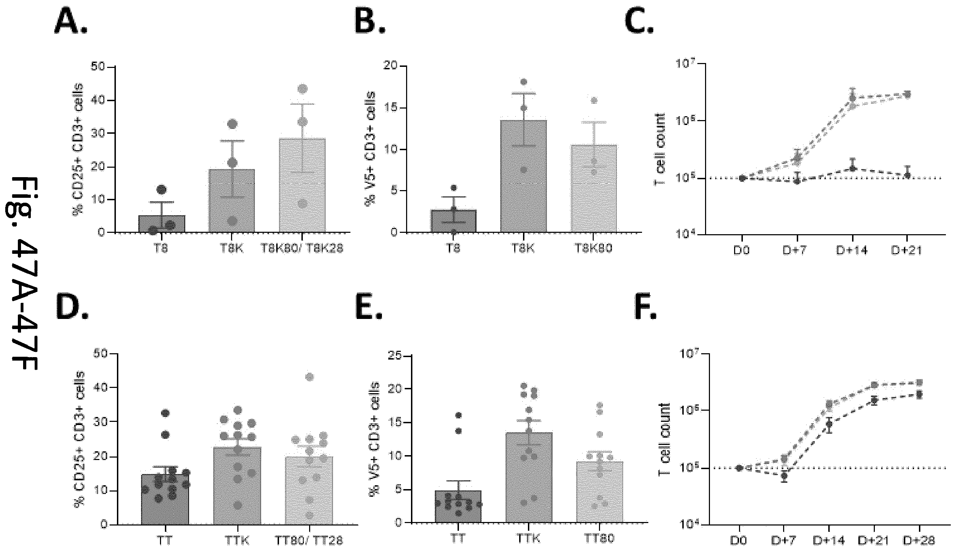

Fig. 47 Shows graphs demonstrating increased activation, transduction and T cell expansion induced by TCRab VHH or CD8a VHH targeted lentiviruses displaying stimulators or co-stimulators. Resting human PBMC freshly isolated by ficoll gradient from buffy coat were infected with 2500 viral particles per cells on day 0. In one instance viral particles areCD8a targeted VSV-G mutant (I331E) pseudotyped CD8a VHH G1x eLV lentivirus (T8), displaying the stimulatory anti-CD3 agonistic antibody OKT3 (T8K) in addition to either of two co-stimulators binding to CD28 (a truncated CD80 protein or an anti CD28 nanobody (VHH), T8K80 or T8K28 respectively). In another instance viral particles are TCRab targeted VSV-G mutant (I331E) pseudotyped TCRab VHH G1x eLV lentivirus (TT), displaying the stimulatory anti-CD3 agonistic antibody OKT3 (T8K) in addition to either of two co-stimulators binding to CD28 (a truncated CD80 protein or an anti CD28 nanobody (VHH), T8K80 or T8K28 respectively). CD3+ T lymphocytes were subsequently analyzed by flow cytometry at 24h post infection for activation (CD25 surface marker), 7, 14 and 21 days post infection for transduction (level of BCMA CAR expression at the cell surface, measured by V5-tag staining) and 7, 14, 21 and 28 days post infection for induced proliferation (expansion). This result shows that whereas T8 eLV do not activate resting T cells, TCRab VHH targeted eLV alone or with a co-stimulatory binder (TTK or TT80 or TT28) or CD8 VHH targeted eLVs in combination with a co-stimulatory binder induce T cell activation, expansion and a significant increase in T cell transduction. These data represent mean±SEM of 3 to 12 separate experiments using 3-5 different donors.

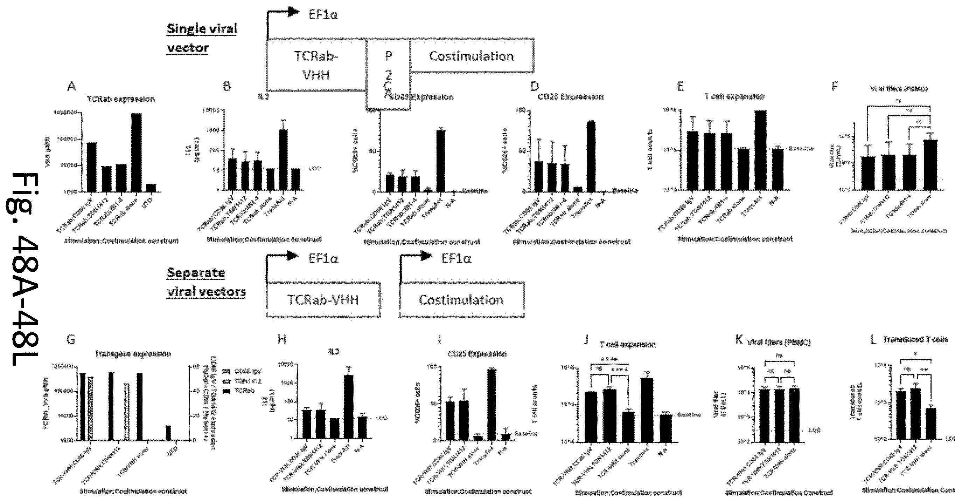

Fig. 48 shows graphs of primary T cell phenotypes following transduction with stimulatory/costimulatory vectors. A single-vector system was used in (A-F), where stable producer cell lines, transduced with a single lentiviral vector expressing stimulatory (TCRab_VHH) and various costimulatory molecules, were used to produce GFP-expressing lentiviruses pseudotyped with VSVgI331E envelope. The expression of the TCRab_VHH on the stable producer cells is shown in (A). 1.5x105 PBMC from 2 independent donors were then transduced with 100uL of unconcentrated lentivirus. Vectors carrying the CD28 costimulatory molecules CD86 IgV and TGN1412, as well as 4-1BB costimulatory molecule 4B1-4, lead to increased IL2 production (B) and CD69 expression (C) day 1 post-transduction, increased CD25 expression (D) day 4 post-transduction, and increased T cell expansion (E) day 7 post-transduction relative to TCRab_VHH alone. The overall transduction was reduced, however in vectors carrying costimulatory molecules, likely due to decreased TCRab_VHH expression in these cells. Results represent the mean±SEM for linear scaled graphs, or the geometric mean ± geometric SD for logarithmic-scaled graphs, of two independent PBMC donors (n = 2). In (G-L), a separate vector system was employed, whereby a clonal producer cell population transduced with the TCRab_VHH was retransduced with vectors expressing various costimulatory molecules (G-L). The expression of the TCRab_VHH and the costimulatory molecules CD86 IgV and TGN1412 is shown in (G). These producer cells were used to produce GFP-expressing lentiviruses pseudotyped with VSVgI331E envelope. 1.5x105 PBMC from 2 independent donors were then transduced with 2 independent preparations of 100uL of unconcentrated lentivirus. Vectors carrying the CD28 costimulatory molecules CD86 IgV and TGN1412, lead to increased IL2 production (H), increased CD25 expression (I) day 4 post-transduction, and increased T cell expansion (J) day 7 post-transduction relative to TCRab_VHH alone. Viral titers were unchanged (K), due to equivalent TCRab_VHH expression. As a consequence of increased T cell expansion, however, the costimulatory molecules lead to an increase in overall transduced cell counts (L). Results represent the mean±SEM for linear scaled graphs, or the geometric mean ± geometric SD for logarithmic-scaled graphs, of two independent PBMC donors and 2 independent lentiviral preparations (n = 4). In all cases, anti-CD3/CD28 (TransAct) was used as a positive control for T cell activation, and PBMC were also left untreated (N-A) to demonstrate background levels of T cell expansion and activation.

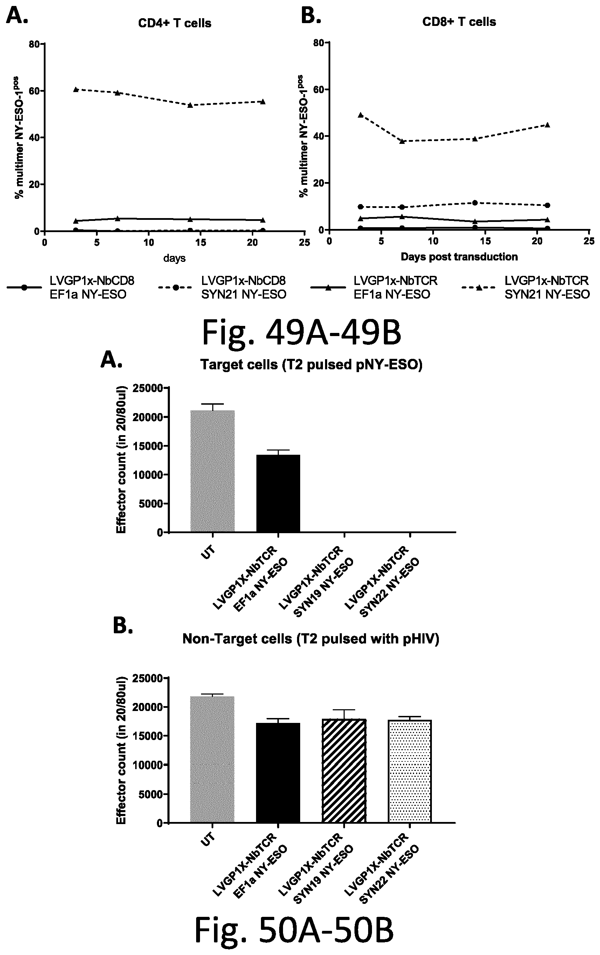

Fig. 49 shows graphs summarizing flow cytometry quantification of hCD3+ cells expressing NY-ESO TCR following transduction with 10ul of VHH CD8a (A.) or TCR targeted LVGP1X eLVs (B.) encoding the NY-ESO TCR transgene under the control of an EF1a or SYN21 or SYN22 promoter (not shown). Activated human PBMCs were transduced with LVGP1x-VHHCD8a or VHHTCR coding for NY-ESO TCR under the control of a EF1a, SYN21 or SYN22. Transduction efficiency in CD3+ T cells were assessed at day 3, 7, 14 and 21 by flow cytometry with a PE coupled A2/NY-ESO-1 multimer. This result demonstrates that LVGP1x-VHHCD8a or VHHTCR stably transduce primary T cells with an NY-ESO TCR construct. Supportive of the result presented in Fig. 31, this result shows that LVGP1X eLV vectors expressing NY-ESO under the control of the EF1a promoter have low functional titre, potentially due to the expression of the NY-ESO TCR on the viral envelope.
Fig. 50 shows graphs summarizing flow cytometry quantification of target and non-target cell numbers in NY-ESO TCR-T co-cultures. Activated human PBMCs were transduced with LVGP1x-VHHTCR coding for NY-ESO under the control of EF1a or a T cell specific synthetic promoters SYN19 and SYN22. The transduced cells were expanded and co-cultured with T2 cells pulsed NY-ESO peptide (target cells) or an HIV peptide (non-target cells) at ratio of 1:1 (ratio of T cells to target/non-target cell line). The number of Cell Trace Violet stained target/non-target cells was quantified by flow cytometry in a fixed volume of media and summarized in graphs A. and B. This figure shows that co-culture of NY-ESO TCR-T cells eradicated target cells (A.) but not of non-target cells (B.) compared to co-cultures with non-transduced T cells. Of note, despite the poor transduction efficiency of the LVGP1X-VHHTCR EF1a NY-ESO vector (see FIG. 35) and thus the low number of NY-ESO TCR-T cells in this co-culture, there was still a significant decrease in the number of target cells in those cultures. This result demonstrates that transduction of primary T cells with LVGP1x-VHH eLVs coding for NY-ESO under the control of the T cell specific promoter results in functional expression of the TCR construct and cytotoxicity of the transduced cells specific for target cells.

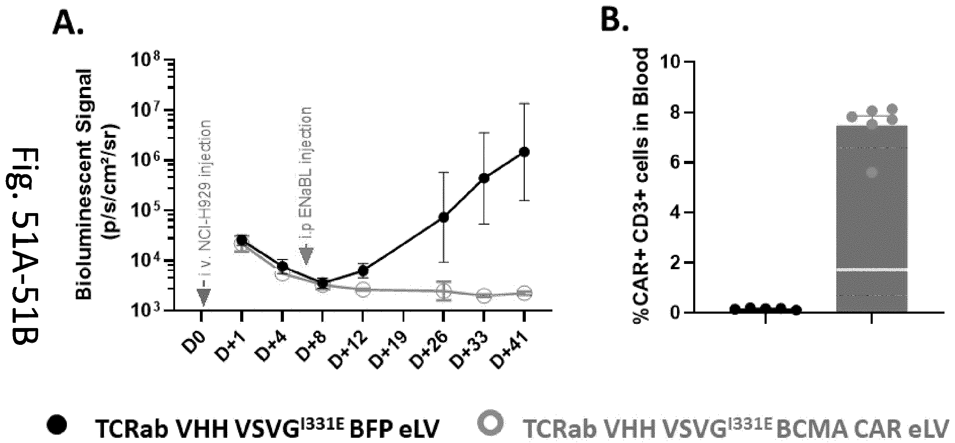

Fig. 51 shows graphs of tumor growth quantified by bioluminescence and flow cytometry quantification of transduced human CD3+ T cells in tumor bearing humanized MHCI/II KO NSG mice injected intraperitoneally with activated human PBMCs and TCRab VHH targeted VSVGI331E pseudotyped eLV vectors. NCI-H929 multiple myeloma cell line stably expressing luciferase was injected systemically (i.v.) in NSG mice deficient for the expression of the murine MHCI and MHCII complexes. Upon confirmation of tumor engraftment, the tumor bearing animals were injected intraperitoneally with activated human PBMCs on Day 0 and one day later with TCRab VHH targeted VSVGI331E pseudotyped eLV vectors expressing BFP or a BCMA CAR construct under the control of the T cell specific synthetic promoter #23. A. Tumor growth over time measured using in vivo bioluminescence signal from luciferase expressing NCI-H929 tumor cells. The data represent the mean BLI signal ± SD from 6 animals per condition. B. TCRab VHH VSVGI331E BCMA CAR in vivo transduction efficiency assessed by flow cytometric analysis of peripheral blood following immunostaining with a CAR specific antibody at Day 14 post vector injection. Data represent the mean± SEM from 6 animals per condition. These results demonstrate efficient transduction of T cells in vivo using eLV-G1X-TCRab targeted vector expressing a BCMA CAR and potent anti tumor activity of the in vivo reprogrammed BCMA CAR-T cells.

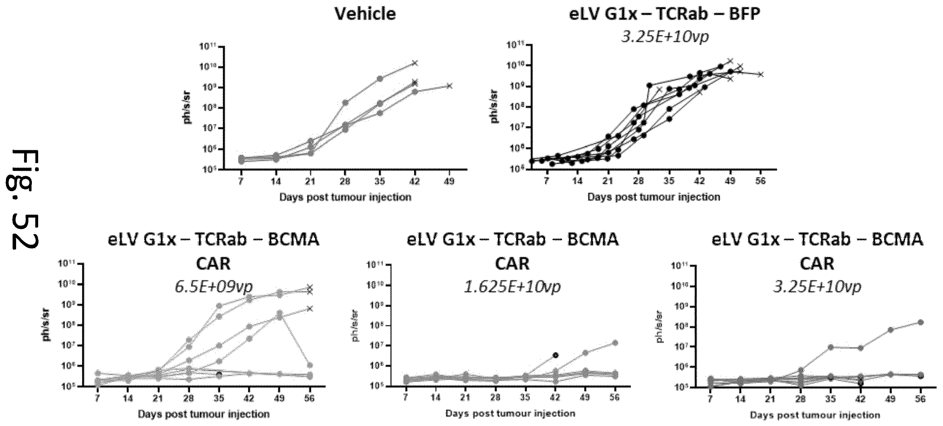

Fig. 52 shows graphs of tumor growth quantified by bioluminescence in tumor bearing hCD34+ humanized NCG mice injected intravenously with escalating doses of eLV-G1X-TCRab viral vectors expressing a BCMA CAR construct or a fluorescent reporter (BFP) under the control of a synthetic T cell promoter #23. NCI-H929 multiple myeloma cells stably expressing luciferase were injected systemically (i.v) in hCD34+ stably humanized NCG mice. Upon confirmation of tumor engraftment, the tumor bearing animals were injected intravenously with eLV-G1x-TCRab vectors expressing BFP or a BCMA CAR construct under the control of the T cell specific synthetic promoter #23. Tumor growth was measured over time using in vivo bioluminescence signal from luciferase expressing NCI-H929 tumor cells. The data represent the BLI signal for each animal. These results demonstrate potent anti tumor activity of the in vivo reprogrammed BCMA CAR-T cells using the eLV-G1X-TCRab vector with complete and persistent tumor clearance in animals treated with 3.25 or 6.5E+10 vp and modest tumor control in animals treated with 1.3E+10 vp.

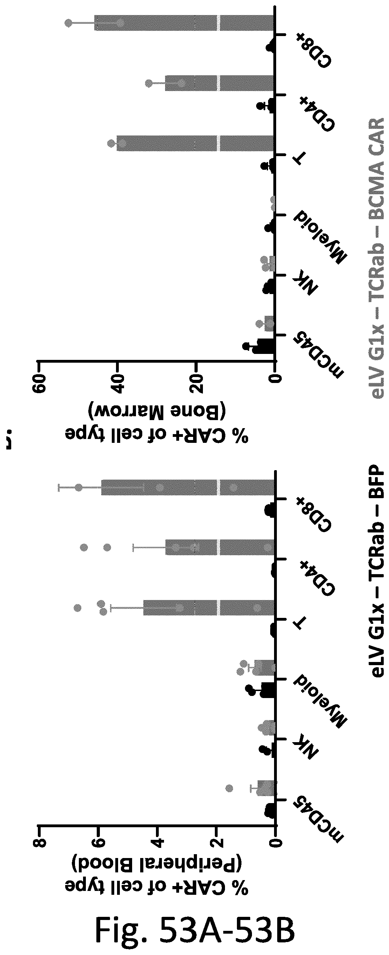

Fig. 53 shows graphs of flow cytometric analysis of tissue samples from tumor bearing hCD34 NCG mice injected intravenously with eLV-G1X-TCRab expressing either the BFP reporter or a BCMA CAR construct. Flow cytometric analysis of (A.) peripheral blood at Day 20 post vector infusion and (B.) bone marrow at euthanasia (up to 36 days post vector injection), for the percentage of CAR+ cells within different immune cell types (murine CD45+ cells, hCD45+/CD56+ human NK cells, hCD45+/CD14+ human myeloid cells, hCD45+/CD3+ T lymphocytes, hCD45+/CD3+/CD4+ and hCD45+/CD3+/CD8+ human T cells). Note, NKT cells were below level of detection and could not be assessed for CAR expression. The results represent the mean±SEM from 2-5 animals per group depending on the tissue and demonstrate the specific targeting and transduction of T cells by eLV-G1X-TCRab BCMA CAR lentiviral vectors.

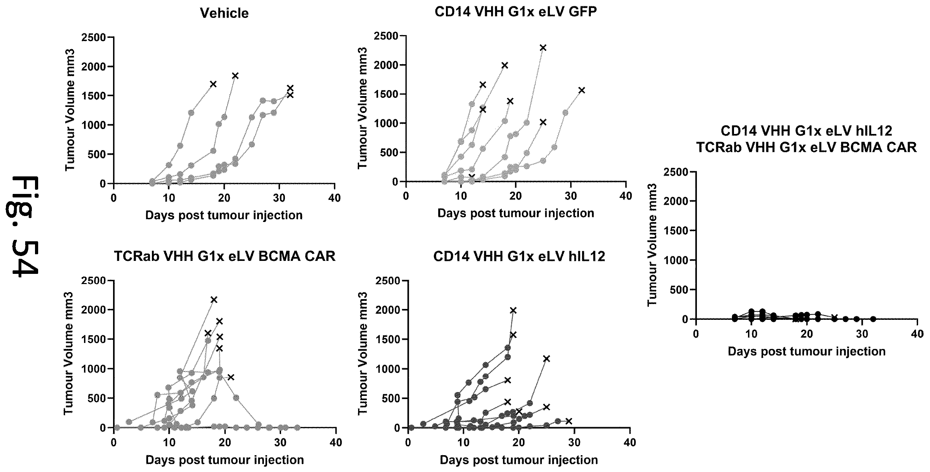

Fig. 54 shows graphs of tumor growth in tumor bearing hCD34+ humanized NCG mice injected intravenously with TCRab VHH targeted and/or CD14 VHH VSVGI331E pseudotyped eLV vectors. NCI-H929 multiple myeloma cell line was injected subcutaneously in NCG and upon confirmation of tumor engraftment, the tumor bearing animals were injected intravenously with vehicle, CD14 VHH VSVGI331E pseudotyped eLV expressing BFP or human IL12, TCRab VHH VSVGI331E pseudotyped eLV expressing BCMA CAR, or a combination of the same dose of both CD14 VHH VSVGI331E eLV human IL12 and TCRab VHH VSVGI331E pseudotyped eLV BCMA CAR in a single injection. Volume of the subcutaneous tumor was measured over time using a calliper. The data represent the tumor volume in each animal (n=4 or n=8) over time for each cohort. These results demonstrate synergistic anti-tumor activity between in vivo reprogrammed monocyte/macrophages and BCMA CAR T cells following a single injection of cell types specific VHH targeted eLV vectors.

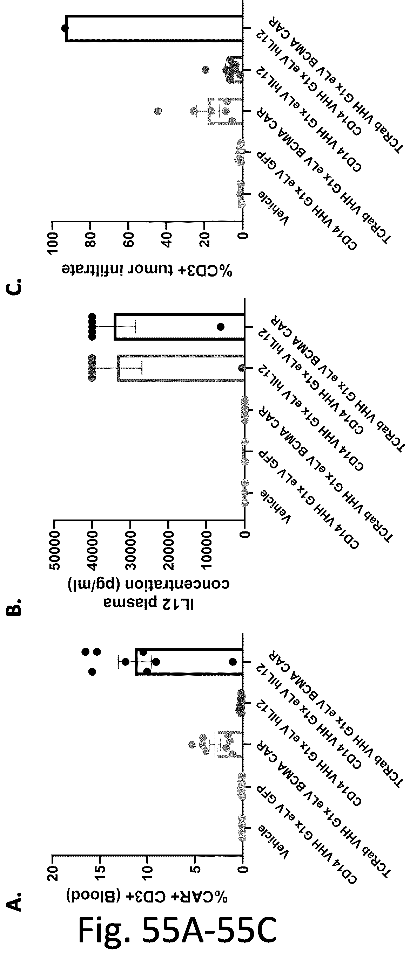

Fig. 55 shows graphs of flow cytometric analysis of tissue samples from tumor bearing hCD34 NCG mice tested in Figure 54. Flow cytometric analysis of (A.) peripheral blood at Day 11 post vector infusion and (C) tumor tissue at euthanasia, for the percentage of CAR+ cells and CD3+ cells, and analysis of the levels of secreted human IL12 in plasma (B) . The results represent the mean±SEM from 1-8 animals per group depending on the tissue and conditions. The data demonstrates that eLV-G1X-TCRab BCMA CAR lentiviral vectors and eLV-G1X-CD14 VHH hIL12 effectively reprogram cells in vivo and that (2) combining the reprogramming of T cells (with BCMA CAR) and monocyte/macrophage (to secrete human IL12) synergizes to increase anti-tumor activity of the individual vectors, at least in part by increasing the transduction/expansion of BCMA CAR-T cells and increasing tumor T cell infiltration.

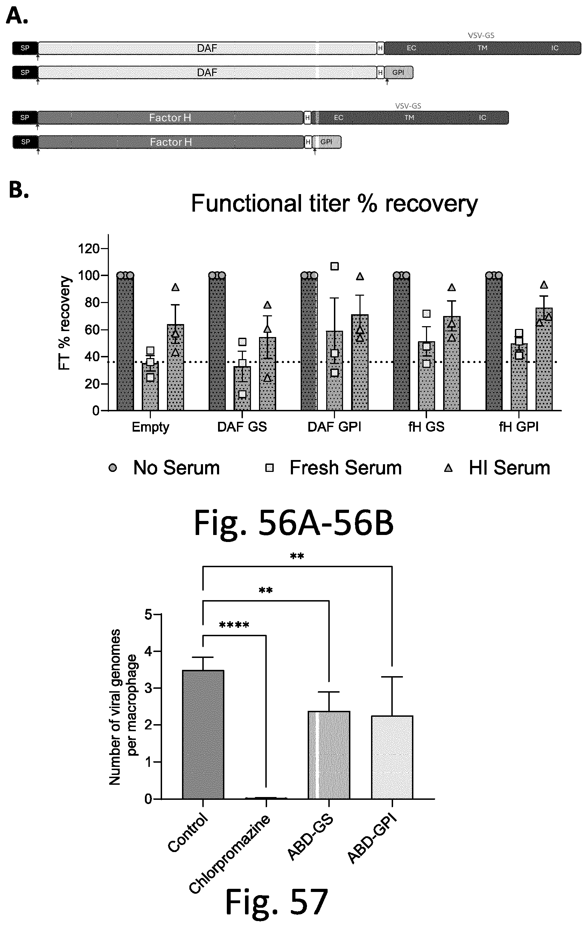

Fig. 56 shows data quantifying the recovery of TCRab VHH VSVGI331E eLVs displaying envelope anchored regulators of the complement system (RCA). (A) Schematic representation of RCA constructs that were tested for their ability to prevent complement-mediated inactivation of VHH display lentiviruses; DAF and factor H were tethered to the lentiviral envelope through a VSV-GS anchor or a GPI anchor. SP : signal peptide, H : 6xHIS-Tag, EC : extracellular domain, TM : transmembrane domain, IC : intracellular domain, GPI : phosphatidylinositol anchor signal. Arrows indicate cleavage sites. (B) A complement inactivation assay was performed where the different lentiviral vectors, encoding a GFP reporter transgene, were incubated for one hour in serum free, media supplemented with fresh human serum or medium complement with heat-inactivated human serum. The vector preparations were then incubated for 2 hours with Jurkat cells and 72hrs later the percentage transduction was assessed by flow cytometry. The results represent the mean±SEM from 3 separate experiments and indicate that DAF and factor H-displaying LVs are partly shielded from complement-mediated inactivation with up to 24,1% and 16,3% increased mean recovery, respectively. In conclusion, displaying RCA on the surface of LVs shields the lentiviral particles against complement-mediated inactivation.
Fig. 57 Show a graph quantifying the phagocytosis by macrophages of T cell targeted TCRab VHH G1x eLVs displaying an Albumin Binding Domain. The Albumin Binding domain of streptococcal G418 was fused to the CD59-GPI anchor or the VSV-GS anchor and tested for its ability to prevent phagocytosis of VHH display lentiviruses by macrophages. A macrophage phagocytosis assay was performed in which human monocyte-derived macrophages were incubated for 2 hours with TCRab VHH G1x lentiviral suspensions then washed and incubated in cRPMI for 3 days. Chlorpromazine was used as a control of inhibiting macrophages activity. 72h postinfection, gDNA was extracted from the macrophages and vector copy number (VCN) was determined. Results show the protective effect of the ABD in the envelope of an eLV displaying a membrane hTCR nanobody (VHH) and the I331E VSVG glycoprotein. The data represent the mean±SEM from 3 separate experiments using 2 distinct PBMC donors and demonstrated that displaying the Albumin Binding Domain in the lentiviral envelope shields the lentiviral particles against phagocytosis by macrophages.

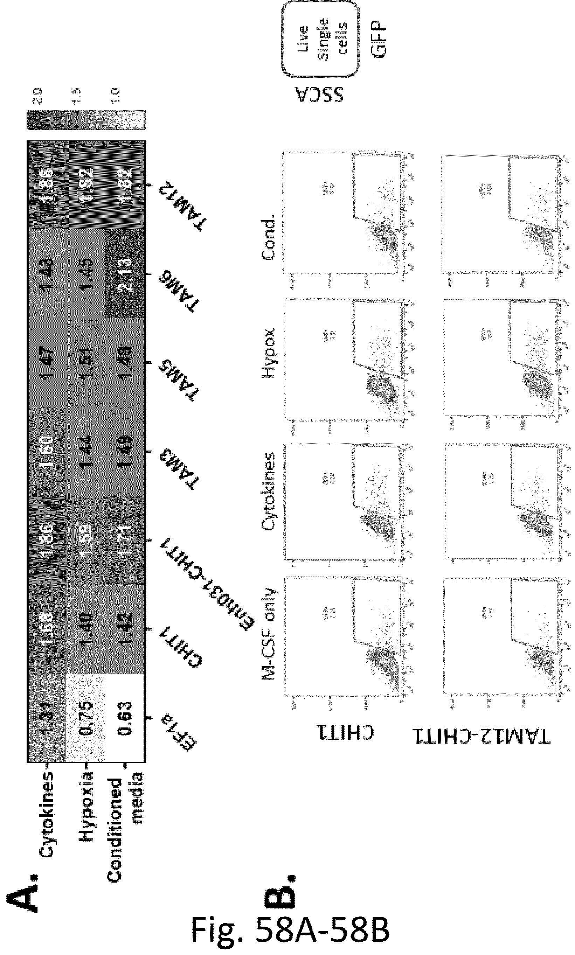

Fig. 58 shows the level of GFP expression, assessed by flow cytometry as the geometric mean fluorescent intensity, in macrophages cultured under conditions mimicking the tumor microenvironment and expressing the reporter under the control of 4 different synthetic promoters (assembled as in Fig. 27) derived from genes specifically upregulated in macrophages of the tumor microenvironment. Human primary monocytes were transduced with VSVG broad tropism lentivirus containing a luciferase-eGFP transgene under the control of EF1a, no promoter (empty condition) or 12 TAM enhancers with CHIT1 proximal promoters (4 of which are represented in this figure). The human primary monocytes were transduced and differentiated in MO-like macrophages in presence of 50ng/ml of M-CSF for 7 days then treated with TME condition for 48h. For "cytokines" condition, MO-like macrophage were treated with 20ng/ml of IL-4, 20ng/ml of IL-10, 10ng/ml of TGFβ and 50ng/ml of M-CSF. For "hypoxia" condition, MO-like macrophage were treated with 100µM of Cobalt Chloride (CoCl2). For "Conditioned medium" condition, M0-like macrophage were treated with the supernatant of NUG-C4 cell culture. The results in A. show the fold change of GFP gMFI of each TME condition normalised to the level of reporter expression in macrophages prior to TME treatment. The results in B show flow cytometry plot data of each tested TME condition for CHIT1, TAM12-CHIT1 and Enh031-CHIT1 synthetic promoters. The experiment was performed with 2 batches of lentivirus per conditions.

### Examples

### Example 1: Production of stable producer cell lines expressing membrane anchored single domain antibodies

This example describes the generation of stable producer cell lines for the production of single domain antibodies (VHH) targeted engineered lentivirus (VHH-eLV). For this purpose, 293T cells stably expressing human CD47 and deficient for B2 microglobulin expression (e293T) were engineered to stably express a membrane anchored VHH targeting human CD3e (SEQ ID NO. 3) or human CD8a (SEQ ID NO. 6) or TCRab (SEQ ID NO 9).

e293T were transduced 24h after seeding with a self-inactivating Moloney murine leukemia virus (SIN gRV) derived retrovirus or self-inactivating lentiviral vectors (LV) encoding either CD3e, CD8a or TCRab VHH. Post-infection, medium was replaced, and cells were grown for 3 days. At harvest, the cells were stained in PBS using a VHH specific antibody (Jackson Immunoresearch) and sorted based on VHH expression level on a fluorescence activated cell sorter (BD FACS Aria^{™}) or cloned by limiting dilution. Selected cells were expanded and banked as polyclonal cell lines (CD3eVHH-e293T and CD8aVHH-e293T) or monoclonal cell lines (e.g. e293T B619 clone 38) stably expressing human CD47 and membrane anchored CD3e or CD8a or TCRab VHH and deficient for MHCl expression.

As shown in Fig. 2A, a subpopulation of e293T cells transduced with membrane anchored CD3e or CD8a nanobodies encoding gRVs expresses high levels of either CD3e (left panel) or CD8a (right panel) membrane anchored nanobodies. After cell sorting the cells expressing the highest levels of VHH (P3 gate in Fig. 2A) and expansion, Fig. 2B shows that the sorted polyclonal cells lines (CD3eVHH-e293T and CD8aVHH-e293T) express stable and homogeneous levels of each VHH. In a different iteration of this example, elevated expression of the membrane anchored TCRab VHH and CD47 and, as well as the decreased MHCl expression, were confirmed by flow cytometry in the e293T B619 clone 38 cell line compared to wildtype cell line HEK 293T (Fig. 2C).

### Example 2: Characterisation of lymphocyte cell lines and primary peripheral blood mononuclear cells for cell surface expression of VHH targets

This example describes the characterization of cell surface markers/receptors expression (e.g. CD3, CD4, CD8 and T Cell Receptor or TCR) on human primary cells (i.e. human Peripheral Blood Mononuclear Cells or hPBMC) or human T cell lines (Jurkat WT, Clone E6-1; SupT1 ) in order to select the cell types for testing and titration of specific embodiments of the VHH-eLVs.

Activated human PBMC (hPBMCs) from a healthy donor (cultured in IL2 containing growth media supplemented with anti-CD3 and anti-CD28 antibodies), Jurkat WT and SupT1 were stained with the following antibodies: anti human CD3 BV510 (BioLegend), anti-human CD4 APC-Cyanine7 (BioLegend), anti-human CD8a PerCP (BioLegend) and anti-human TCRa/b BV421 (BioLegend) and DAPI. Flow cytometric analysis was performed using a CytoFLEX LX^{™} (Beckman Coulter).

As shown in Fig. 3A, activated hPBMCs positively stain for all four markers (top panels), with two main cells populations: CD3+ CD4+ TCR+ and CD3+ CD8+ TCR+. Jurkat WT cells are CD3+ TCR+ but do not stain for CD8a and a small subpopulation express low level of CD4 receptors (middle panels). SupT1 cells on are both CD4+ CD8+ but do not stain for CD3 or TCR (bottom panels). In-house engineered Jurkat cells stably expressing hCD8a (see example 4) following lentiviral transduction with a vector encoding the hCD8a are CD3+ CD8+ TCR+ (Fig. 3B).

In conclusion, primary human PBMCs, Jurkat WT, SUP-T1 and Jurkat CD8 cells provide different targeting cell populations that can serve to select and characterise VHH-eLV vectors targeted to a range of T cell surface markers, including CD3, CD4, CD8 or TCR).

### Example 3: The combination the wild type vesicular stomatitis virus envelope glycoprotein G and nanobodies targeting either CD3e or CD8a fails to redirect VHH targeted lentiviral vectors to CD3+ or CD8+ T cells.

Viral cell entry is an early and critical step in the process of cell transduction by lentiviruses. This occurs through the fusion of the viral envelope with the cell membrane of targeted cell. A range of viral proteins have evolved to catalyse this step in viral transduction, one of which is widely used to pseudotype lentiviral vector: the vesicular stomatitis virus glycoprotein G (VSVG). However, viral envelope glycoproteins also ensure targeting of the virus, which in the case of VSVG is to a broad range of cell types through binding to cell surface receptors including the LDLR.

In this example, we tested whether envelope anchored nanobodies can be used in combination with VSVG to redirect the broad tropism of VSVG pseudotyped lentivirus towards a single cell type while still benefiting from the membrane fusion properties of VSVG. For this purpose, CD3e or CD8a envelope anchored nanobodies were used in combination with the wild type VSVG protein and the tropism of the combination vectors was tested on target and non-target cells.

VSVG pseudotyped CD3e or CD8aVHH-eLVs or VSVG eLVs were generated by transient transfection of producer cells with a lentiviral transfer plasmid, encoding the destabilized GFP reporter gene, and an envelope construct expressing the wild type VSVG. VHHCD3e, VHHCD8a or wild-type e293T cells (see Example 1) were transiently transfected 24hr after seeding in culture media. Transient transfection was performed using the PElpro transfection reagent (58ug; Invitrogen) and a mixture of the lentiviral packaging plasmids (12ug of the transfer plasmid, 5.5ug of the VSVG envelope plasmid, 5.5ug of Gag/Pol packaging plasmid and 6ug of Rev-expressing plasmid).The PElpro/plasmid DNA mixture was added to the cells and 72h post-transfection (cells cultured at 37°C in 5%CO2), the supernatant containing lentiviruses was harvested, clarified for 10 minutes at 500 g and filtered through a 0.2mm polyethersulfone filter (Millipore). 5X Lenticoncentrator (Origene) was added to the clarified supernatant and the mixture stored at 4°C for 1.5hr before centrifugation (3500 g, 25 minutes, 4°C). The pellet, containing lentiviruses, was resuspended in 150uL of FBS free TexMACS media (Miltenyi).

To determine the functional titre, Jurkat WT or SupT1 cells transduced with serial dilutions of concentrated viral preparations (final dilution 100x - 1000x - 10000x - 100000x) were assessed for GFP expression by flow cytometry 72h post-infection. The functional titre (transducing units or TU/mL) is calculated on at least two linear dilutions using the following formula: (number of seeded cells x % of GFP+ cells x dilution factor)/volume in millilitres (mL).

For transduction assay of Jurkat and SupT1 cells, a functional MOI of 2 was used growth media and 72h post-infection the cells were harvested and assessed for GFP+ expression by flow cytometry as described above.

As shown in Fig.4 wild type VSVG pseudotyped eLV vectors (VSVG) transduce both Jurkat WT and SupT1 cells, in agreement with the broad cellular tropism of the VSVG envelope protein. However, the co-expression of the wild type VSVG envelope protein and either the VHHCD3e or the VHHCD8 (CD3e VSVG and CD8a VSVG, respectively) failed to re-direct the targeting of the vectors to CD3+ (Jurkat WT) or CD8+ (SupT1) cells respectively. Indeed, the CD3e VSVG vector infected SupT1 cells (which do not express CD3e, see Fig. 3) to a level similar to that of the VSVG vectors. Similarly, the CD8a VSVG eLV infected Jurkat cells (which do not express CD8, see Fig. 3) to a level similar to the VSVG vectors. Taken together, these results demonstrated that the envelope anchored VHHCD3e or VHHCD8a fail to re-target wild type VSVG pseudotyped eLV to cells expressing the VHH target, but instead maintain the broad tropism targeting associated with the wild type VSVG protein.

### Example 4: The combination of a VHH targeting the CD8a receptor and the Y150A Lassa Virus glycoprotein mutant or the wild type Le Dantec virus envelope protein targets the lentiviral particles specifically to CD8+ T cells

The aim of this study was to determine whether association a cell surface targeting VHH and viral envelope glycoproteins other than VSVG can direct the tropism of the lentiviral vectors to the cell type expressing the cell surface protein targeted by the VHH. For this purpose, lentiviral vectors displaying a combination of a CD8a targeting VHH and different wild type/mutant viral envelope proteins on the envelope were tested for specificity of transduction on CD8 positive and negative cell lines. In the results presented in this example, we identified two viral envelope proteins which in combination with a CD8a VHH induced specific targeting of CD8+ cells.

To test the specificity of CD8a directed lentiviral particles, we first established a CD8a expressing Jurkat cell line (Jurkat CD8+). Jurkat WT cells (Clone E6-1) were transduced with a lentivirus coding for the human CD8a receptor, expanded and the population stably expressing hCD8a was sorted and banked as the Jurkat CD8+ polyclonal cell line.

CD8a VHH eLVs were generated by transient transfection of the VHHCD8a e293T producer cell line (see Fig. 2) using a transfer plasmid coding for the dsGFP reporter and viral envelope construct encoding either Y150A Lassa Virus glycoprotein mutant (LVGP1x; SEQ ID NO. 32) or the wild type Le Dantec virus envelope protein (LDV; SEQ ID NO. 33). 72h post-transfection, the supernatant containing lentiviruses was harvested, concentrated and the vectors tittered as outlined in Example 3.

To test the transduction specificity of the LVGP1X- or LDV-VHHCD8a eLVs, Jurkat WT and Jurkat CD8+ cells were transduced as in Example 3 with an MOI of 2. 72h post-infection, cells were harvested and assessed for GFP+ expression by flow cytometry.

As shown in Fig.5, LVGP1X- or LDV-VHHCD8a eLVs specifically transduced CD8a expressing Jurkat cells (Jurkat CD8+), contrary to the broad tropism VSVG pseudotyped eLV which transduced both Jurkat WT and Jurkat CD8+ T cells. Taken together, our results show that the LVGP1X and the LDVG pseudotyped lentiviral vectors can be targeted specifically to CD8+ T cells by an envelope anchored CD8a directed VHH.

### Example 5: The combination of a VHH targeting the CD3e T cell surface protein and LVGP1x targets the lentiviral particles specifically to CD3+ T cells

The aim of the study is to extend the findings in Example 4 to other T cell surface markers and confirm that association a cell surface targeting VHH with LVGP1x can direct the tropism of the lentiviral vectors to the target cell. For this purpose, lentiviral vectors displaying a combination of a CD3e targeting nanobodies and LVGP1X were tested for specificity of transduction on CD3e positive and negative cell lines.

hCD3e VHH display vectors were generated by transient transfection VHHCD3e H6 (SEQ ID NO. 4) or VHHCD3e G7 (SEQ ID NO. 3) stably expressing e293T producer cell line (similar to those presented in Fig. 2) as described previously.

To test the transduction specificity of the LVGP1X-VHHCD3e eLVs, SupT1 (which do not express CD3e) and Jurkat WT cells (which express CD3e) were transduced as in described previously with an MOI of 2. 72h post-infection, cells were harvested and assessed for GFP+ expression by flow cytometry.

As shown in Fig.6, LVGP1X-VHHCD3e eLVs specifically transduced CD3e expressing Jurkat WT cells, contrary to the broad tropism VSVG pseudotyped eLVs which transduced both Jurkat WT and CD3e negative SupT1 cells. Taken together, our results show that the LVGP1X pseudotyped lentiviral vectors can be targeted specifically to CD3e T cells by an envelope anchored CD3e directed VHH.

### Example 6: The combination of a binder targeting the TCR receptor and the Y150A Lassa Virus glycoprotein mutant virus envelope protein targets the lentiviral particles specifically to TCR+ cells

The aim of the study is to extend the VHH-eLV technology to other cell surface markers and confirm that association a cell surface targeting VHH with the selected envelope proteins directs the tropism of the lentiviral vectors to the VHH target cell. For this purpose, lentiviral vectors displaying a combination of a TCR targeting VHH and LVGP1X pr LDV were tested for specificity of transduction on TCR positive and negative cell lines.

hTCR VHH display vectors were generated by transient transfection of a VHH TCR 2 (SEQ ID NO. 34) stably expressing e293T producer cell line (similar to those presented in Fig. 2) as outlined previously.

To test the transduction specificity of the VHHTCR2 eLVs, SupT1 (which do not express TCR) and Jurkat WT cells (which express TCR) were transduced as in Example 4. 72h post-infection, cells were harvested and assessed for transgene expression (a V5 tagged BCMA CAR construct) by flow cytometry.

As shown in Fig.7, LVGP1X-VHHTCR2 and LDV-VHHTCR2 eLVs specifically transduced TCR expressing Jurkat WT cells, contrary to the broad tropism VSVG pseudotyped eLV which transduced both Jurkat WT and TCR negative SupT1 cells equally, albeit with low efficiency in this study (potentially due to excessive dilution of the highly concentrated vector). Taken together, our results show that the LVGP1X and LDV pseudotyped lentiviral vectors can be targeted to surface expressed TCR through the use of an envelope anchored TCR VHH.

### Example 7: Lentiviral vectors targeted using envelope anchored CD8a VHH and LVGP1x or LDV envelope proteins induce persistent transduction of CD8+ Jurkat cells

The aim of the study is to determine whether LVGP1X- or LDV-VHHCD8 eLVs stably transduce target cells. For this purpose, lentiviral vectors displaying a combination of a CD8 targeting VHH and the LVGP1X- or LDV envelope proteins were tested for persistence of transduction on CD8 positive Jurkat cell line.

LVGP1X- or LDV-VHHCD8a eLVs were prepared as before and used to transduce Jurkat CD8+ cells. 2, 7 and 14 days post-infection, cells were harvested and assessed for GFP+ expression by flow cytometry.

As shown in Fig.8, transduction of Jurkat CD8+ cells with LVGP1X- or LDV-VHHCD8a eLVs persists over 14 days, similar to transduction by the broad tropism VSVG pseudotyped eLV. This result demonstrates that LVGP1X- and LDV-CD8a eLVs induce stable transduction of target cells.

### Example 8: Lentiviral vectors targeted using envelope anchored CD3e VHH and the LVGP1x envelope protein induce persistence transduction of Jurkat cells

The aim of the study is to determine whether LVGP1X-VHHCD3e eLVs stably induce persistent transduction of target cells.

hCD3e VHH eLVs were generated as before and used to transduce Jurkat WT cells at an MOI of 2. 2, 7, 14 and 21 days post-infection, cells were harvested and assessed for GFP+ expression by flow cytometry. In addition, vector insertion was quantified by qPCR on genomic DNA extracted at day 21 post transduction. Briefly, genomic DNA (gDNA) from infected cells was extracted and purified using the DNeasy Blood and Tissue Kit (from QIAgen). 50ng of gDNA was assessed by quantitative PCR using the Luna Universal qPCR master mix (New England Biolabs) using primers specific for human Albumin and reverse-transcribed Long Terminal Repeat (LTR). Standard curves for Albumin (3 to 30.000 cells) and transcribed LTRs (40 to 400.000 viral copies) were used respectively to quantify the number of cells and the number of vector copies in the test samples. The quantitative PCR was performed on a QuantStudio 3 (Life Technologies).

As shown in Fig.9, expression of the reporter GFP transgene persists over 21 days following transduction of Jurkat WT cells with LVGP1X-VHHCD3e eLVs, similar to transduction by the broad tropism VSVG pseudotyped eLV. In addition, vector copy number assessment at 21 days demonstrates LVGP1X-VHHCD3e eLVs integrates into the host genome at an average of 1.6 copies per cell at an MOI of 2. This result demonstrates that LVGP1X-VHHCD3e eLVs induce stable and persistent transduction of Jurkat cells through integration into the host genome.

### Example 9: The combination of a VHH targeting the CD8a receptor and the LVGP1x envelope protein targets the lentiviral particles specifically to cell lines endogenously expressing CD8a

The aim of the study is to extend the findings in Example 4 using a target cell line that expresses CD8a endogenously (Sup-T1) rather than exogenously (Jurkat stably expressing CD8 from viral transduction), to avoid potential confounding effects associated with transgenic overexpression of CD8a in the Jurkat CD8+ cell line. In addition, persistence of transduction of the LVGP1X-VHHCD8a eLV was evaluated in this example. For this purpose, lentiviral vectors displaying a combination of a CD8a targeting nanobodies and LVGP1X were tested for specificity of transduction on CD8a positive and negative cell lines.

hCD8a VHH display vectors were generated by transient transfection VHHCD8a (SEQ ID NO. 6) stably expressing e293T producer cell line as before.

To test the transduction specificity of the LVGP1X-VHHCD8 eLVs, SupT1 (which express CD8a) and Jurkat WT cells (which do not express CD8a) were transduced as at an MOI of 2. 3, 7, 14 and 21 days post-infection, cells were harvested and assessed for GFP+ expression by flow cytometry.

As shown in Fig.10, LVGP1X-VHHCD8a eLV specifically transduced the SupT1 endogenously expressing CD8a but not Jurkat WT cells (A) in which CD8a is below the level of detection (see Fig 2). Moreover, transduction of SupT1 cells with LVGP1X-VHHCD8a eLV persists over 21 days (B) and a vector copy number assessment at 21 days demonstrates that LVGP1X-VHHCD8a eLV integrate into the host genome with an average of 3.1 copies per cell in this experiment (C). This result demonstrates that LVGP1X-VHHCD8a eLVs induce stable transduction of target cells through integration into the host genome.

### Example 10: The combination of a VHH targeting the TCR receptor and the LVGP1x or LDV envelope protein induce persistence transduction of Jurkat WT cells

The aim of the study is to extend previous findings to demonstrate persistent transduction of TCR+ Jurkat WT cells using LVGP1x-VHHTCR and LDV-VHHTCR vectors.

TCR VHH display vectors were generated by transient transfection VHHTCR2 (SEQ ID NO. 34) stably expressing e293T producer cell line (similar to those presented in Fig. 2) as previously described.

To test the transduction efficiency of the LVGP1X-VHHTCR2 and LDV-VHHTCR2 eLVs, Jurkat WT cells were transduced as before with an a 1:40 dilution of the vector preparation. 4, 7, 12, 16 days post-infection, cells were harvested and assessed for GFP+ expression by flow cytometry.

As shown in Fig.11, LVGP1X-VHHTCR2 and LDV-VHHTCR2 eLVs efficiently transduced TCR expressing Jurkat WT cells, and persistence of transduction was observed for both types of vector similar to the persistence of broad tropism VSVG pseudotyped vectors.

### Example 11: The combination of a VHH targeting the CD8a receptor and the LVGP1x envelope protein targets the lentiviral particles specifically to primary CD8+ T lymphocytes

This example describes the specificity of the LVGP1X-VHHCD8a on primary T cells. For this purpose, lentiviral vectors displaying a combination of a CD8a targeting nanobodies and LVGP1X were tested for specificity of transduction on activated hPBMCs which contain both CD4+ and CD8+ T lymphocytes.

hCD8a VHH display vectors were generated by transient transfection VHHCD8a (SEQ ID NO. 6) stably expressing e293T producer cell line as described previously. hPBMC isolated from healthy donors were stimulated by anti-CD3 and anti-CD28 antibodies for 2 days and were transduced at an MOI of 20 with LVGP1x-VHHCD8a eLV or broad tropism VSVG eLV. Four days after transduction, half of the cells were collected, washed, and stained for flow cytometry with anti-CD4 and anti-CD8. The same analysis was performed overtime at day 7, 11, 14 and 20 to assess the persistence of GFP expression.

As shown in Fig.12, LVGP1X-VHHCD8a eLV specifically transduced the primary CD8+ T cells and not primary CD4+ T lymphocytes, contrary to broad tropism VSVG eLVs which transduced T lymphocytes non-specifically. This result demonstrates that LVGP1x-VHHCD8a eLVs discriminate between subpopulations of primary T cells and targets the vector only to CD8+ lymphocytes.

### Example 12: The combination of a VHH targeting TCR and the LVGP1x envelope protein on eLVs effectively transduces primary T lymphocytes

This example describes the transduction efficiency and persistence of the LVGP1X-VHHTCR2 eLV on primary T cells. For this purpose, lentiviral vectors displaying a combination of a TCR targeting VHH and LVGP1X were tested on activated hPBMCs.

TCR VHH display vectors expressing the GFP reporter were generated by transient transfection VHHTCR2 (SEQ ID NO. 34) stably expressing e293T producer cell line as previously described and the vector was titrated on Jurkat WT cells. hPBMC isolated from healthy donors stimulated by anti-CD3 and anti-CD28 antibodies for 2 days were transduced at an MOI of 5. Four days after transduction, half of the cells were collected and stained as previously described with anti-CD3, CD4 and CD8 antibodies before analysis by flow cytometry for the percentage of GFP expressing cells. The same analysis was performed overtime at day 7, 11, 14 and 20 to assess the persistence of vector transduction monitored by GFP expression.

As show in Fig. 13, LVGP1X-VHHTCR2 transduces both CD4+ and CD8+ primary T cells persistently over the duration of the study (20 days) similar to the VSVG vector, albeit with a higher transduction efficiency. In conclusion, these results demonstrate that a lentiviral vector associating of a TCR targeting VHH with the LVGP1X envelope protein effectively targets and stably transduces activated primary T cells.

### Example 13: Transduction of Jurkat CD8+ cells with LVGP1X-VHHCD8a or LDV-VHHCD8a eLVs encoding a BCMA CAR results in functional expression of the CAR construct as demonstrated by the activation of the transduced cells after exposure to target cells.

In this example, CAR-Jurkat cells targeting the BCMA surface antigen were generated using LVGP1X-VHHCD8a or LDV-VHHCD8 eLVs to demonstrate functional delivery of a CAR construct using the VHH targeted eLVs. Functional delivery of the CAR construct was assessed by upregulation of the T cell activation marker CD69 after exposure to target cells expressing the CAR antigen.

LVGP1X-VHHCD8a or LDV-VHHCD8a vectors encoding a BCMA CAR construct (SEQ ID NO. 35) were generated from stably expressing e293T producer cell as previously described. Jurkat cells were transduced with the VHH-eLVs at an MOI of 2 and were passaged for 7 days prior to use in the co-culture assay. For co-culture assay, BCMA antigen negative (K562) and BCMA antigen positive (NCI-H929) target cells were labelled with CellTrace Violet (Thermo Fisher) to differentiate them from CAR-transduced Jurkat (CAR-Jurkat). CAR-Jurkat cells were cocultured overnight at an effector : target ratio of 1:1. The level of CAR-Jurkat cell activation was assessed 18 hours post-coculture by flow cytometry following CD69 antibody staining (BioLegend), DAPI staining was used to differentiate living cells. The binder domain of BCMA-CAR used in this study is a VHH and expression of the CAR construct in live cells was detected by staining with an anti-VHH antibody (Jackson Immunoresearch).

As shown in Fig. 14A, BCMA CAR-Jurkat cells upregulated CD69 expression only in the presence BCMA+ NCI-H929 target cells, to a similar extent whether VHHCD8a eLV or broad-tropism VSVG eLV were used to transduce the BCMA CAR. Fig. 14B represents a quantification of the results in Fig. 14A.

### Example 14: Transduction of primary T lymphocytes with LVGP1X-VHHCD3e vectors encoding a BCMA CAR results in functional expression of the CAR construct as demonstrated by the activation of the transduced cells after exposure to target cells.

This example describes the functional delivery to primary T cells of a CAR construct using LVGP1X-VHHCD3e and the assessment of the upregulation of the T cell activation marker CD137 after exposure to target cells expressing the CAR antigen.

LVGP1X-VHHCD3e eLVs encoding a BCMA CAR construct (SEQ ID NO. 35) were generated from stably expressing e293T producer cell as previously described. hPBMC were activated with anti-CD3/CD28 T Cell TransAct reagent on day 0 (Miltenyi) and transduced on day 2 with the eLVs encoding BCMA CAR constructs overnight, and media exchanged on day 3. Supplementation with cytokines (30 ng/mL IL-2; Miltenyi) was performed during primary cell culture. Cultures were propagated until harvest on day 14 for coculture assays. For co-culture assay, BCMA antigen negative (K562) and BCMA antigen positive (NCI-H929) target cells were labelled with CellTrace Violet (Thermo Fisher) to differentiate them from primary human T cells (CAR-T). CAR-T cells were cocultured overnight at an effector : target ratio of 1:1. The level of CAR-Jurkat cell activation by assessed 18 hours post-coculture by flow cytometry following CD137 antibody staining (BioLegend), 7-AAD staining was used to differentiate living cells. CAR expression in live cells was determined by staining with an anti-VHH antibody (Jackson Immunoresearch).

As shown in Fig. 15A, BCMA CAR-T cells upregulated CD137 expression only in the presence BCMA+ NCI-H929 target cells, to a similar extent whether CD3e VHH-targeted eLV or broad-tropism VSVG LV were used to transduce the BCMA CAR. Fig. 15B represents a quantification of the results in Fig. 15A.

### Example 15: Transduction of primary T lymphocytes with LVGP1X-VHHCD8a vectors encoding a BCMA CAR results in functional expression of the CAR construct and cytotoxicity of the CAR-T against BCMA expressing target cells.

This example describes the in vitro functionality of BCMA CAR-T cells generated using the LVGP1X-VHHCD8a as measured by target cancer cell killing.

LVGP1X-VHHCD8a vectors encoding a BCMACAR construct under the control of a T cell specific promoter SYN19 (SEQ ID NO. 12) were generated from stably expressing CD8aVHH-e293T producer cell as previously described. hPBMCs were activated in vitro for 48h in the presence of anti-CD3 and anti-CD28 antibodies. 2 days post activation the cells were transduced at an MOI of 5 in complete IMDM media supplemented with 30 ng/ml of human IL-2 and expanded 7 days. Transduction of PBMCs resulted in approximately 12 % CD3+ CAR+ cells with the VSVG vector (expressing the BCMA CAR construct under the control of the ubiquitous EF1a promoter) and 6 % with the LVGP1X-VHHCD8a vector, confirming expression of the CAR. For co-culture assay, BCMA antigen negative (K562) and BCMA antigen positive (NCI-H929) target cells were labelled with CellTrace Violet (Thermo Fisher) to differentiate them from the CAR-T cells. 40,000 target cells were co-cultured with CD3+ T cells adjusted to obtain a 1:1 or a 1:2 effector to target ratio. Untransduced or GFP expressing T cells were used as controls to determine background alloreactivity. After five days of co-cultured, the number of target/non-target cells was quantified by flow cytometry after staining the cell suspension with anti-CD3 PercP.

As shown in Fig. 16A, there was >75% decrease in the number of NCI-H929 target cells in the co-cultures with CAR-T cells transduced with LVGP1X-VHHCD8 or VSVG eLVs, compared to untransduced or GFP transduced controls. In contrast, the K562 cell number remained relatively constant across all groups (Fig. 16B), confirming that NCI-H929 depletion was CAR dependent and BCMA specific.

Altogether, these results demonstrate that primary BCMA CAR-T cells from transduction with LVGP1X-VHHCD8a eLVs are functional and cytotoxic against a BCMA+ target cancer cell line.

### Example 16: Transduction of primary T lymphocytes with LVGP1X-VHHCD8a vectors encoding a CD19 directed CAR results in functional expression of the CAR construct and cytotoxicity of the CAR-T against CD19 expressing target cells.

The aim of this study is to extend the functional characterisation of primary CAR-T cells generated in vitro using LVGP1X-VHHCD8a to other CAR-T constructs.

LVGP1X-VHHCD8a vectors encoding a CD19 CAR construct (SEQ ID NO. 36) under the control of a T cell specific promoter SYN19 (SEQ ID NO. 12) were generated from stably expressing CD8aVHH-e293T producer cell as previously described. hPBMCs were activated in vitro for 48h in the presence of anti-CD3 and anti-CD28 antibodies. 2 days post activation the cells were transduced at an MOI of 10 in complete IMDM media supplemented with 30 ng/ml of human IL-2 and expanded 7 days. Transduction of PBMCs resulted in approximately 4% CD3+ CAR+ cells with the VSVG vector (expressing the CD19 CAR construct under the control of the ubiquitous EF1a promoter) and 12 % with the LVGP1X-VHHCD8a, as assessed by flow cytometry after staining cells for CD3 and V5 tag (fused to the CD19 CAR).

For co-culture assay, CD19 antigen negative myelogenous leukaemia cell line (K562) and CD19 expressing B cell precursor leukaemia cell line (Nalm6) target cells were labelled with CellTrace Violet (Thermo Fisher) to differentiate them from the CAR-T cells. Target and non-target cells were co-cultured with CD3+ T cells adjusted to obtain a 1:1 or a 1:2 effector to target ratio. Untransduced T cells were used as a control for non-specific alloreactivity. After three days, the number of target/non-target cells was quantified by flow cytometry after staining the cell suspension with anti-CD3 A700.

As shown in Fig. 17A, there was >75% decrease in the number of Nalm6 target cells in the co-cultures with CAR-T cells transduced with LVGP1X-VHHCD8a or VSVG eLVs at 1:2 and 1:5 co-culture ratios, compared to untransduced controls. In contrast, the K562 cell number remained relatively constant across all groups (Fig. 17B), confirming that NCI-H929 depletion was CAR dependent and CD19 specific. Altogether, these results demonstrate that primary CD19 CAR-T cells from transduction with LVGP1X-VHHCD8a eLVs are functional and cytotoxic against a CD19+ target cancer cell line.

### Example 17: Transduction of primary T lymphocytes with a LVGP1X-VHHTCR2 vector encoding a BCMA CAR results in functional expression of the CAR construct and cytotoxicity of the CAR-T against BCMA expressing target cells.

This example describes the in vitro functionality of BCMA CAR-T cells generated using the LVGP1X-VHHTCR2 as measured by target cancer cell killing.

LVGP1X-VHHTCR2 vectors encoding a BCMA CAR construct under the control of a T cell specific promoter SYN19 were generated from stably expressing VHHTCR2-e293T producer cell as previously described. hPBMCs were activated and transduced at an MOI of 10 as previously described. Transduction of hPBMCs resulted in approximately 80% CD3+ CAR+ cells with the LVGP1X-VHHTCR2 vector, confirming expression of the CAR. For co-culture assay, BCMA antigen negative chronic myelogenous leukaemia (leukemia) cell line (K562) and the multiple myeloma BCMA positive NCI-H929 cell line were labelled with CellTrace Violet (Thermo Fisher) to differentiate them from the CAR-T cells. Target and non-target cells were co-cultured with CD3+ T cells adjusted to obtain a 1:1, 1:2 or a 1:5 effector to target ratio. Untransduced T cells were used as controls to determine background alloreactivity. After five days of co-cultured, the number of target/non-target cells was quantified by flow cytometry after staining the cell suspension with anti-CD3 PercP.

As shown in Fig. 18A, There was an >80-90% decrease in the number of NCI-H929 target cells in the co-cultures with CAR-T cells transduced with LVGP1X-VHHTCR2, compared to untransduced controls across effector : target ratios. In contrast, the K562 cell number remained relatively constant across all groups (Fig. 18B), confirming that NCI-H929 depletion was CAR dependent and BCMA specific.

Altogether, these results demonstrate that LVGP1X-VHHTCR2 BCMA CAR eLV vectors reprogram T cells into BCMA CAR T cells which are functional and cytotoxic against a BCMA+ target cancer cell line.

### Example 18: LVGP1X-VHHCD8a dsGFP eLV specifically and stably transduces CD8+ human T cells in vivo in a humanized mouse model.

This example describes the transduction specificity and persistence of LVGP1X-VHHCD8a in humanized mice. Of note, the CD8a VHH targeting the LVGP1X eLV does not cross react with the mouse CD8a homologue, thus the need to assess in vivo transduction of LVGP1X-VHHCD8a in an immunodeficient mouse model (NOD SCID gamma mouse; NSG) engrafted with human PBMCs providing the necessary targets for eLV transduction.

NSG mice were humanised by injection of 10 million activated hPBMCs (2 day activation with anti-CD3 and anti-CD28 antibodies) intraperitoneally. The following day, 25 million TU (transducing units) or 100 million TU of LVGP1X-VHHCD8a eLV dsGFP were injected intraperitoneally. A control group injected with the vehicle (TexMACS) was included. Study treatment groups are described in Table 1. LVGP1X-VHHCD8a eLV dsGFP vectors encoding GFP under the control of an EF1a ubiquitous promoter were produced from stably expressing CD8VHH-e293T producer cell as previously described. In vivo transduction by LVGP1X-VHHCD8a eLV dsGFP was quantified by the percentage of cells expressing GFP measured by flow cytometry on peripheral blood collected from weekly bleeds over 27 days. hPBMCs were stained for CD3, CD4, CD8, CD19 and CD56 to determine the cell types expressing the GFP transgene and thus transduction specificity of LVGP1X-VHHCD8a eLVs.

**Table 1 . Study Treatment Groups**

| Group | Treatment | Dose (TU) | No. of animals |
|---|---|---|---|
| 1 | Vehicle | NA | 3 |
| 2 | LVGP1x-VHHCD8a EF1a dsGFP eLV | 25 Million | 5 |
| 3 | LVGP1x-VHHCD8a EF1a dsGFP | 100 Million | 5 |

GFP expression was observed in both LVGP1X-VHHCD8a eLV dsGFP groups at day 4 and was specific to CD8+ T cells (Fig. 19A), with ≤0.1% GPF+ CD4+ cells in the high dose group 3. Expression of the GFP transgene was stable over the 27 days study and ranged between 2-3% for the 25 million TU dose and 3-4% for the 100 million TU dose (Fig. 19B). These results demonstrate that LVGP1X-VHHCD8a eLV can selectively transduce human CD8 positive T cells in vivo and that expression of the transgene is persistence for at least 27 days.

### Example 19: LVGP1X-VHHCD8a in vivo reprogramming of T cells with a CD19 CAR results in B cell aplasia in a humanized mouse model.

This example describes the functionality of CD8+ CAR-T cells transduced in vivo using the LVGP1X-VHHCD8a eLV encoding for a CD19 CAR transgene.

10 million activated hPBMCs were injected intraperitoneally into NSG mice on day-1 (as described in Example 19) followed by 100 million TU of LVGP1X-VHHCD8a CD19 CAR eLV the next day (day 0). Study treatment groups are described in Table 2: VSVG EF1a dsGFP eLV is a broad tropism VSVG pseudotyped eLV encoding the destabilised GFP reporter under the control of the EF1a promoter; LVGP1X-VHHCD8a CD19 CAR eLV encodes for a CD19 CAR construct (SEQ ID NO. 36) under the control of the synthetic T cell-specific promoter SYN19 (SEQ ID NO. 12) and is fused with a V5 tag to facilitate detection of CAR expressing cells; VSVG CD19 CAR eLV is a broad tropism VSVG pseudotyped eLV encoding the same CD19 CAR under the control of SYN19. B cells, isolated from the donor used for the day-1 injection, were injected intravenously on 1 day following vector administration to provide additional targets for the in vivo re-programmed CD19 CAR-T cells. At day 7, half of the animals were collected, and flow cytometry performed on peripheral blood and freshly dissociated spleen and bone marrow, to assess transduction efficiency (% V5 expressing cells) and anti-CD19 CAR activity (percentage of B cells). The remaining mice were collected for the same analysis on day 14 (data not shown).

**Table 2 . Study Treatment Groups**

| Group | Treatment | Dose (TU) | No. of animals |
|---|---|---|---|
| 1 | VSVG EF1a dsGFP eLV | 100 Million | 8 |
| 2 | LVGP1X-VHHCD8a CD19 CAR eLV | 100 Million | 8 |
| 3 | VSVG CD19 CAR eLV | 100 Million | 8 |

Circulating CAR T cells were detected at day 7 (Fig. 20A) and day 14 (data not shown) in both VSVG and LVGP1X-VHHCD8a eLV CD19 CAR groups. Whereas VSVG eLV transduced both human CD4 and CD8 T cells, CAR-T expression was only observed in human CD8+ T cell population in the LVGP1X-VHHCD8a eLV CD19 CAR group, corroborating the specificity of the LVGP1X-VHHCD8a eLV vector observed in Fig. 19. Expression of CAR in group 2 and 3 was accompanied by depletion of human B cells in peripheral blood, bone marrow and spleen at day 7 (Fig. 20B) and day 14 (data not shown), with approximately a 1-2 log decrease in the percentage of B cells compared to the control group treated with the VSVG eLV EF1a dsGFP vector. B cells were defined as hCD45+hCD20+ cells. B cell aplasia in groups 2 and 3 also confirms that the T cell synthetic promoter supports sufficient expression of the CD19 CAR to generate functional CAR-T cells.

In conclusion, LVGP1X-VHHCD8a eLV CD19 CAR vector in vivo transduced CD8+ CAR-T cells are functional and effectively eradicate B cell in vivo.

### Example 20: CD8a VHH LVGP1X eLV in vivo delivery of a CD19 CAR construct in stably humanized mice results in B cell aplasia.

This example describes the functionality of CD8+ CAR-T cells delivered using the CD8a VHH LVGP1x eLV encoding for a CD19 CAR transgene in a model of stably humanized mice (hCD34 NSG) which supports the targeting of CD8a VHH LVGP1x eLV to resting/naïve human T cells in the context of an endogenously reconstituted multilineage human immune system using the intended clinical route of administration.

hCD34+ NCG humanized mice were generated by transplantation of human CD34+ hematopoietic progenitors in chemoablated NCG mice. At ~18 weeks of age, hCD34+ NCG mice with human blood chimerism >25% were injected with 70E+6 TU of CD8a VHH LVGP1x eLV encoding a CD19 CAR or a GFP reporter transgene under the control of the T cell specific synthetic promoter SYN19 (SEQ ID NO 12). Cytokines IL-7, IL-15/IL-15Ra were injected subcutaneously four and one day prior to vector injection with the goal of supporting endogenous T cell expansion and potentially T cell transduction. As shown in Fig. 20C, significant B cell aplasia was observed in animals injected with CD8a VHH LVGP1x CD19 CAR eLV compared to vehicle or CD8a VHH LVGP1x GFP eLV injected animals. B cell aplasia was persistent until the end of the study at Day 53 in the CD8a VHH LVGP1x CD19 CAR eLV group. VCN analysis confirmed presence of the transgene in liver, spleen, and bone marrow in decreasing order of prevalence with levels below 0.3 VCN per cell in liver and below 0.1 VCN per cell in spleen. No VCN was detected above background in lung, kidney, heart, brain, and ovaries (data not shown). No adverse health effects were reported in any of the treatment groups.

Conclusion - This study demonstrates that injection of ENaBL-T8 vector using the intended clinical route of administration was well tolerated in a murine model with endogenously circulating resting T cells, generating sufficient CD19 CAR T cells to observe elimination of the target B cell population. Biodistribution of the transgene was limited to liver, spleen, and bone marrow.

### Example 21: CD8a or TCRab VHH LVGP1X eLV specifically delivers CD19 CAR construct to T cell in vivo and generate functional CD19 CAR T cells inducing rapid B cell aplasia in a humanized mouse model.

This example describes in vivo transduction efficiency and specificity of CD8a or TCRab VHH LVGP1X eLV in humanized mice, resulting in the in vivo production of functional CAR-T cells and the elimination of the target cell population.

NSG mice were humanised by injection of 10 million activated hPBMCs (2 day activation with anti-CD3 and anti-CD28 antibodies) intraperitoneally. The following day, 10 million TU (transducing units) of CD8a or TCRab VHH LVGP1x eLV encoding for a CD19 CAR transgene or 50 million TU of TCRab VHH LVGP1x eLV dsGFP were injected intraperitoneally. A control group injected with 50 million TU of VSVG LV CD19 CAR was included.

As shown in Fig. 20D, CD19 CAR T cells were detected in CD8a or TCRab VHH LVGP1x CD19 CAR eLV or VSVG LV CD19 CAR injected animals as early as Day 7 post vector injection. TCRab VHH LVGP1x CD19 CAR eLV vector transduction efficiency of CD3+ T cells was higher compared to CD8a VHH LVGP1x CD19 CAR eLV vectors (9.0% ±1.5 and 1.0% ±0.15 respectively at the 10e6 TU dose in peripheral blood at Day 7) and induced higher expansion of the T cell population (10e5-10e7 T cells/ml vs 10e4-10e5 T cells/ml, respectively at Day 7), hence higher overall numbers of circulating CAR T cells (10e5-10e6 CAR T cells/ml vs 10e2-10e4 CAR T cells/ml, respectively). As shown in Fig. 20F, CD8a VHH LVGP1x CD19 CAR eLV vector transduction was specific for CD8+ T cells (1.9%+0.3 CD4+CAR+ cells vs 22.4%+2.2 CD8+CAR+ cells at the 10e6 TU dose at Day 11), whereas TCRab VHH LVGP1x CD19 CAR eLV transduced both CD4 and CD8 T cells with a slight preference for CD4 T cells (14.5%+2.3 CD4+CAR+ cells vs 7.6%+1.4 CD8+CAR+ cells at the 10e6 TU dose at Day 11). Detectable levels of transduction and expression of CD19 CAR was observed for both vectors in the NKT population but not in NK, CD14 or mouse CD45 cells. Despite different levels of in vivo reprogrammed CD19 CAR T cells in the different groups, there was near complete or complete B cell aplasia in blood and spleen in all CD19 CAR groups by Day 7 and Day 11 respectively.

This study demonstrates that CD19 CAR T cells generated by CD8a or TCRab VHH LVGP1x CD19 CAR eLV vectors in vivo have anti-target cytotoxic activity. TCRab targeting of TCRab VHH LVGP1x CD19 CAR eLV results in higher levels of transduction compared to the CD8 targeting vector and an overall higher expansion of the T cell population. Expression of the CD19 CAR by the T cell specific synthetic promoter 23 was specific to T and NKT cells and resulted in potent anti-target cytotoxic activity of the CAR.

### Example 22: The combination of a VHH targeting the CD8 receptor and a triple mutant of the Lassa Virus glycoprotein targets the lentiviral particles specifically to CD8 expressing cells

This example describes cell specific transduction achieved using an eLV displaying a membrane anchored VHH and a mutant version of the LVGP envelope protein. For this purpose, multiple mutants of the Lassa Virus envelope glycoprotein were tested and Fig. 21 presents the finding on one such mutant envelope protein which in association with a CD8a targeting VHH specifically transduces CD8 positive cell line.

hCD8a VHH display vectors were generated as previously described by co-transfection of the VHHCD8a e293T producer cell line with the packaging and transfer plasmids and a viral envelope construct expressing the either Y150A Lassa Virus glycoprotein mutant (as a positive control) or a Y150A H141A F147A Lassa Virus glycoprotein mutant (LVGP3x; SEQ ID NO. 37).

To test the transduction specificity of the LVGP1X- or LVGP3X -VHHCD8a eLVs, Jurkat WT, 293T and Sup-T1 cells were transduced as previously with an MOI of 2. 72h post-infection, cells were harvested and assessed for GFP+ expression by flow cytometry.

As shown in Fig. 21, LVGP3X-VHHCD8a eLVs specifically transduced CD8 expressing cells (Sup-T1) similar to the LVGP1X-VHHCD8 vector and contrary to the broad tropism VSVG pseudotyped eLV which transduced both CD8 negative cell lines (Jurkat WT and 293T) and CD8+ SupT1 cells. Taken together, these results show that the LVGP3X pseudotyped lentiviral vectors, similar to LVGP1X eLVs, are targeted by the envelope anchored VHH and that effective transduction is mediated by the mutant Lassa envelope protein.

### Example 23: Pseudotyping CD8 VHH displaying vectors with wild type or H141A/F147A, but not Y150A/F446L Lassa Virus glycoprotein targets the lentiviral particles specifically to CD8+ primary T cells

This example describes cell specific transduction achieved using an eLV displaying a membrane anchored CD8 VHH and pseudotyped with either WT or H141A/F147A or Y150A/F446L Lassa Virus glycoprotein.

CD8a VHH displaying vectors were generated as previously described by co-transfection of the CD8a VHH e293T producer cell line with the packaging and transfer plasmids and a viral envelope construct expressing Wild type (SEQ ID NO 196), Y150A/F446L (SEQ ID NO 195) or H141A/F147A (SEQ ID NO 194) Lassa Virus glycoprotein or the Y150A mutant Lassa Virus glycoprotein (used as a positive control). To test the transduction specificity of the differentially pseudotyped CD8a VHH eLVs, activated PBMC's were transduced as previously described with an MOI of 10 and expression of the GFP cargo transgene was assessed overtime by flow cytometry.

As shown in Fig. 22, CD8 VHH targeted eLVs pseudotyped with wild type or H141A/F147A Lassa Virus glycoprotein transduced CD8+ primary T cells only, albeit at a lower level of transduction compared to Y150A Lassa Virus glycoprotein pseudotyped vectors. On the contrary, pseudotyping CD8 VHH eLVs with the Y150A/F446L mutant Lassa Virus glycoprotein abrogated transduction efficiency of the vectors while pseudotyping with VSVG induced nonspecific transduction of both CD8+ and CD8- (CD4+) primary T cells. Taken together, these results show that the wild type and H141A/F147A Lassa Virus glycoprotein pseudotyped VHH displaying eLVs, similar to Y150A VHH eLVs, are targeted by the envelope anchored VHH and that effective transduction is mediated by both the wild type and select mutants of Lassa envelope protein.

### Example 24 Cell specific transduction is achieved by combining VHH mediated targeting and select mutants of the VSVG envelope glycoprotein.

This example describes cell specific transduction achieved using an eLV displaying a membrane anchored VHH and newly identified mutants of the VSVG glycoprotein. Based on the crystal structure of the VSVG glycoprotein we generated specific mutants in key residues located around the binding pocket or in the path binding of the VSVG protein to its native receptor: LDLR. These mutations were designed to prevent (steric hindrance) or block (polarity change) the interactions with the LDL-R with the goal of preserving the viral entry activity of VSVG while abrogating the targeting function of the protein.

Fig. 23 presents the findings supporting the selection of mutants of VSVG which lack broad tropism targeting of wild type VSVG and which, in association with a CD8a targeting VHH, specifically transduce CD8 positive cell line.

hCD8a VHH display vectors were generated as previously described by co-transfection of the CD8a VHH e293T producer cell line with the packaging and transfer plasmids as well as viral envelope constructs expressing mutants of VSVG listed in Fig 23A (SEQ ID NO. 38 to 40 and SEQ ID NO. 153 to 182) respectively. To test the transduction specificity of the VSVG mutant VHHCD8 eLVs, Jurkat WT, Jurkat cells expressing CD8 and Sup-T1 cells (endogenously expressing CD8a) were transduced with an MOI of 2. 72h post-infection, cells were harvested and assessed for GFP+ expression by flow cytometry.

As shown in Fig. 23B and C, a number of VSVG mutant CD8 VHH eLVs, including single mutant A51E, I182E, I331W, I331E, T352W, T352Q or double/triple/quadruple mutants "I52R, I331E, E355L", "N9G, K47G, I331E, T352S", "H8F, A51P, R329V, T352V", "N9Q, K47T, T328S, E355"L, "N9G, K50S, V333I, T351R", "H8F, S48L, R322,5K", "H8Y, K47G", "N9H, S48R, R329T, T352E", "Q53A, I331E, T352I", "N9T, I52T, R329K, T352E", "H8S, H49A, R329H, T352Q", "K11A, S48L, I331L, R354K", "N9R, Q53I, I331Q, R354M", "H8S, I52V, V333Y, T351S", "I331E, E353K", "Q10V, K50H, I331M, T352D", "N9A, I331E, E353S", "H8V, K47W, Y330M, T351D" specifically transduced CD8 expressing cells (Jk-CD8 or SupT1 cells) contrary to the broad tropism VSVG pseudotyped eLV which transduced both the CD8 negative cell line (Jurkat WT) and CD8+ cells (Jk-CD8 or SupT1 cells).

As shown in Fig. 24A and B, CD8 VHH eLVs pseudotyped with mutants of VSVG identified in Fig. 23B on cell lines endogenously expressing CD8 (Sup T1 or CD8+ primary T cells), confirmed target specific transduction of A51E, I182E and I331E mutant VSVG pseudotyped vectors.

In Fig. 24C, transduction specificity of I331E mutant VSVG pseudotyped vectors was also tested with a TCRab VHH targeted eLV. These data confirm that I331E pseudotyping preserves targeting specificity of eLV displaying a VHH other than the CD8a VHH and demonstrate that without VHH targeting, I331E pseudotyped eLVs are transduction deficient.

As shown in Fig 25, lack of LDL-R binding of a TCRab VHH eLV pseudotyped with VSVG mutant I331E was confirmed on HEK 293 T cells over-expressing LDL-R, whereas binding of the vector was observed on HEK 293T cells over-expressing SIRPa (as expected since CD47, the ligand of the SIRPa receptor, is displayed at the surface of the TCRab VHH eLV).

Taken together, our results show that VSVG mutants designed to abrogate binding to LDLR, in particular VSVG mutants containing a mutation at position I331, can be used to pseudotype lentiviral vectors to achieve cell targeted transduction through the use of an envelope anchored target directed VHH.

### Example 25 Transduction of primary T lymphocytes with TCRab VHH targeted eLVs pseudotyped with VSVG targeting deficient mutants encoding a CAR constructs results in functional expression of the CAR construct and cytotoxicity of the CAR-T against target expressing cells.

This example describes the in vitro functionality of CAR-T cells generated using the TCRab VHH eLV pseudotyped with the targeting deficient VSVG^{I331E} mutant as measured by target cancer cell killing.

TCRab VHH G1x eLV vectors encoding a BCMA CAR construct (SEQ ID NO 35) under the control of a T cell specific promoter SYN23 (SEQ ID NO 15) were generated from stably expressing TCRab VHH e293T producer cells as previously described. Unstimulated hPBMCs were transduced with BCMA CAR encoding TCRab VHH G1x eLVs and after confirmation of CAR expression, the cells were co-cultured at different ratios with BCMA antigen negative chronic myelogenous leukaemia (leukemia) cell line (K562), the multiple myeloma BCMA positive NCI-H929 cell line and the acute lymphoblastic leukemia. lymphocytic cell line Nalm6 expressing low levels of BCMA. at different effector to target ratios. As shown in Fig. 26A-I there was an >90% decrease in the number of NCI-H929 target cells in the co-cultures with CAR-T cells transduced with TCRab VHH G1x eLVs, compared to untransduced controls across effector : target ratios. In contrast, the K562 cell number remained relatively constant across all groups, confirming that NCI-H929 depletion was CAR dependent and BCMA specific. CAR-T mediated cytotoxicity correlated expansion and with increased IL2 production, indicating polyfunctionality of the CAR-T cells generated using the TCRab VHH G1x eLVs vector.

TCRab VHH G1x eLVs mediated delivery of functional CARs is applicable to other constructs. CAR-T cells targeting Claudin 18.2 were generated as described above using TCRab VHH G1x eLV vectors encoding a Claudin 18.2 CAR (SEQ ID NO 53) under the control of a T cell specific promoter SYN23. The CAR-T cells were co-cultured at different ratios with Claudin 18.2 antigen negative chronic myelogenous leukaemia (leukemia) cell line (K562), the Claudin 18.2 positive gastric cancer cell line (NUGC4) and the acute lymphoblastic leukemia lymphocytic cell line Nalm6 stably expressing either Claudin 18.2 or Claudin 18.1. As shown in Fig. 26K-N, cytotoxic activity of TCRab VHH G1x eLV transduced Claudin 18.2 CAR-T cells was detected as early as 18 hours of co-culture with NUGC4 cells and was specific to Claudin 18.2 expressing cells, with no observed toxicity to K562 cells and Nalm6 cells stably expressing Claudin 18.1.

Altogether, these results demonstrate that TCRab VHH G1x eLV vectors effectively deliver CAR constructs to resting T cells and produce CAR-T cells with potent anti-tumor activity.

### Example 26: Reporter construct for testing novel cell type specific synthetic promoters

Synthetic cell specific promoters are assembled from genome sequences upstream of selected cell specific genes. These genome sequences encompass the core and proximal promoters of the selected cell specific genes, as well as enhancer sequences identified upstream of those genes. The selected sequences are assembled in a synthetic promoter under 1Kb in size and tested using the reporter construct shown in Fig. 27. This dual reporter construct allows for the qualitative and quantitative assessment of the specificity and potency (level of expression) of the upstream promoter. The fluorescent reporter is used for the qualitative assessment of promoter specificity using flow cytometry and the luminescence reporter is used for quantitative assessment of promoter potency.

### Examples 27: Specificity and potency of T cell synthetic promoters driven by specific combinations of core, proximal and enhancer sequences.

This example describes the specificity and potency of 10 synthetic T cell specific promoters assembled as described in Example 23. For this purpose, multiple candidate synthetic promoters were tested for specify (using a flow cytometry readout of GFP expression in specific cell types) and potency (using a luminescence readout from the expression of the luciferase reporter) in a range of cell types, using the EF1a ubiquitous promoter as comparator. The panel of cell types was selected based on the predicted vector exposure after systemic administration and include lymphocytes (Jurkat and SupT-1cell lines and primary T lymphocytes CD4+ and CD8+ from human PBMC) as well as non-T lymphocytes from the myeloid (THP1) and B cell (NCI-H929) compartment and originating from liver (Huh7) and kidney (293T). Fig. 28-31 present the results obtained on 10 candidates.

VSVG eLVs expressing the reporter construct under the control of EF1a or the different synthetic promoter, were generated in e293T producer cell line by transient transfection as previously described. Activated hPBMC and cell lines were transduced (MOI of 5 and 2 respectively) with the different VSVG eLVs and 7 days post-transduction, the cells were evaluated for eGFP expression by flow cytometry, viability using Cell-Titer Fluor (Promega) and luciferase expression by luminescence using a Steady-Glo assay (Promega). For flow cytometry on hPBMCs, cells were stained with anti-CD3 BV510 anti-CD4 APC-Cy7 and anti-CD8 PerCP to identify T cell subtypes.

As shown in Fig 28-31, the luminescence signal from several of the synthetic promoter driven luciferase expression (notably SYN22) is equivalent or above that of EF1a reporter expression in Sup-T1 (Fig. 28), Jurkat (Fig. 29) and primary T cells (Fig. 30). Addition of the enhancer sequences in the SYN constructs significantly increases the expression of the luciferase reporter compared to the proximal promoter alone. Furthermore, as shown in Fig. 30D, expression level (measured as the geometric mean of the fluorescent signal) of the GFP reporter is equivalent in CD4+ and CD8+ primary T cells cell types. Conversely, when reporter expression was tested in non-T cell lines, luminescence was 4-628x fold lower in SYN constructs compared to EF1a driven reporter expression depending on the cell type (Fig. 31).

Altogether, these results demonstrate that purpose-built T cell specific synthetic promoters with an optimized size for inclusion into lentiviral constructs effectively restrict the expression of reporter transgenes to T lymphocytes in culture.

### Example 28: Use of a T cell synthetic promoter to express a CAR transgene using a VHH directed eLV significantly reduces expression of the CAR at the surface of the producer cell line during LV manufacturing and increases functional titre (titer) of the vector

This example describes the effect of using the T cell specific synthetic promoters on VHH targeted lentiviral vectors manufacturing. We assessed the production yield (assessed using functional titre) of a LVGP1x-VHHCD8a vectors encoding for a BCMA or CD19 CAR under the control of the SYN or EF1a promoters.

LVGP1x-VHHCD8a SYN19 BCMA/CD19 CAR and LVGP1x-VHHCD8a EF1a BCMA/CD19 CAR eLVs were produced under the same conditions as previously described from the VHHCD8 e293T producer cell line. As shown in Fig. 29A, and consistent with Fig. 32A, expression of the transgene (BCMA CAR stained with an Anti-V5 A647 and assessed by flow cytometry) was significantly reduced on the surface of end of process cells (producer cells collected after the final viral harvest) from LVGP1x-VHHCD8 SYN19 BCMA CAR production compared to end of process cells from LVGP1x-VHHCD8 EF1a BCMA CAR production. To assess the functional titre of the vector, SupT1 cells were transduced with each viral production at a range of dilutions (1:50 to 1:3125) and transduction was assessed 3 days post transduction following anti-V5 A647 staining and quantification of percentage BCMA or CD19 CAR expressing cells by flow cytometry. Functional titre (transducing Units/ml) was calculated from a minimum of two dilutions of vector yielding a percentage of transduced cells between 5 and 50%.

As shown in Fig. 32B, functional titre for LVGP1x-VHHCD8 encoding either CAR constructs under the control of a SYN promoter was 10x higher compared the same construct expressed under the EF1a promoter. In conclusion, replacing EF1a by a synthetic T cell specific promoter to control expression of the CAR cargo of LVGP1x-VHHCD8 vectors significantly enhances functional titre of the vector production.

### Example 29: T cell specific synthetic promoters drive functional CAR expression and hight CAR-T cell potency compared to a constitutive promoter

This example describes the assessment of the potency of CAR-T cells expressing the CAR construct under the control of the T cell specific synthetic promoters selected in Examples 26-28. For this purpose, BCMA CAR cytotoxic activity was assessed in primary T cells transduced with eLVs for the expression of a BCMA CAR under the control of an EF1a or a SYN promoter.

In Fig. 33, BCMA CAR-T cells produced by transduction of activated human PBMCs with VSVG pseudotyped eLV vectors (SYN19 BCMA CAR expressing a BCMA CAR transgene under the control of the Synthetic promoter 19 (SYN19); or EF1a BCMA CAR expressing a BCMA CAR transgene under the control of the Elongation factor 1a (EF1a) promoter; or EF1a GFP expressing a BCMA CAR transgene under the control of the EF1a promoter) were cultured in the presence of multiple myeloma BCMA expressing NCI-H929 cells (stably expressing a luciferase reporter) at various effector to target ratios. Untransduced or GFP expressing T cells were used as a control for non-specific alloreactivity. After five days of co-culture, 100 µl (100 microlitres) of culture was washed and luciferase expression (correlating to the number of live NCI-H929 target cells) was assessed using a Steady-Glo assay (Promega). Compared to co-cultures with untransduced or EF1a-GFP vector transduced T cells, there is >4x decrease in the luminescence (correlating with the levels of viable NCI-H929 target cells) in the co-cultures with the SYN19-BCMA CAR or EF1a-BCMA CAR transduced CAR-T cells at both E:T ratios. This result demonstrates that the cytotoxicity of SYN19-BCMA CAR-T cells is enhanced compared to that of EF1a-BCMA CAR-T cells..

In order to further characterise the activity of T cell synthetic promoter regulated BCMA CAR-T cells, BCMA CAR-T cells produced from human PBMCs from 3 different donors by transduction with VSVG pseudotyped eLV vectors (SYN23 BCMA CAR expressing a BCMA CAR transgene under the control of the Synthetic promoter 23, SEQ ID NO 15); or EF1a BCMA CAR expressing a BCMA CAR transgene under the control of the Elongation factor 1a (EF1a) promoter) were cultured at different effector (CAR) to target (cell line) ratios with target (NCI-H929) and non-target (K562) cells. Fresh target cells were added at various time points to test for the persistence of CAR-T anti-tumor activity. The results presented in Fig. 34 show BCMA CAR-T cells expressing the CAR construct under the control of the SYN23 promoter, contrary to the EF1a promoter regulated CAR, demonstrated (1) enhanced expansion, (2) enhance anti-tumor activity in the presence of high levels of target cells (1:12) and (3) lower background activation in the absence of BCMA antigen and increased BCMA specific activation. Together, these results demonstrate that BCMA CAR-T cells expressing the CAR construct under the control of the T cell specific synthetic promoter #23 have superior and more persistent anti-tumor activity.

### Example 30: CD14 binder displaying LVGP1x eLVs specifically transduce monocytes

Targeting of the eLVs through combination of an envelope anchored binder and a mutant envelope protein (e.g. LVGP1x, G1x, LDV) is designed to target cell types beyond T lymphocytes by switching the envelope anchored binder. This example describes the specific transduction of myeloid cells using an LVGP1x eLV displaying a membrane anchored scFv binder targeting CD14 (a protein expressed on the surface of myeloid cells which functions as part of the LPS receptor complex). Of note, monocytes express multiple viral restriction factors (e.g. SAMHD1) and to achieve effective transduction of monocytes using lentiviral vectors it is necessary to package the Viral protein X (Vpx) within the viral particle. Incorporation of Vpx is achieved during viral production by adding a Vpx expression plasmid during the transient transfection of the producer cell line. As shown in Fig. 35A, incorporation of Vpx within VSVG LV or LVGP1x scFV CD14 eLV vectors particles (see below for details on vector production and transduction conditions) significantly increases transduction of primary monocytes in culture.

Specificity of LVGP1x-scFvCD14 eLV vectors was assessed by comparing transduction of human PBMCs to that of primary monocytes isolated from the same donor PBMCs. LVGP1x-scFVCD14 scFvCD14 eLV vectors expressing GFP were produced by transient transfection of a e293T cell line stably expressing the membrane anchored anti-CD14 scFv (SEQ ID NO. 41) with packaging (using a Gag/pol construct containing a modified gag sequence, SEQ ID NO. 42, to allow packaging of Vpx), transfer and envelope expression plasmids (LVGP1X) supplemented with a Vpx expression construct (SEQ ID NO. 43). The physical titre of the vector suspension was determined using an RT-qPCR viral genome quantification kit (Takara) and non-stimulated hPBMCs (cultured in 30ng/ml IL2) or isolated human monocytes (isolated from hPBMCs using a CD14 MACS isolation kit from Miltenyi and cultured in 50ng/ml GM-CSF (130-093-865) + 50ng/ml IL-4) were transduced with 100, 200, 500, 1000 vector particles per cell. 7 days after transduction the percentage of cells expressing GFP was assessed was assessed by flow cytometry.

As shown in Fig. 35B, both CD14 targeted LVGP1 Vpx eLVs transduced primary monocytes with high efficacy, whereas transduction of non-stimulated PBMCs remained below 2% even when the cells were exposed to 1000 viral particles per cell. In conclusion, redirecting LVGP1x eLVs using an envelope anchored anti-CD14 binder induces effective and specific transduction of primary monocytes.

### Example 31: Selection of CD14 VHH for targeted transduction of monocytes by CD14 targeted eLVs

Targeting of primary monocytes was successfully achieved with eLVs targeted using a CD14 scFv. To demonstrate that this is also applicable to VHH targeted eLVs, de novo VHH targeting CD14 or CCR2 (SEQ I NO 66 to 116 and SEQ ID NO 117 to 120, respectively) were generated by immunization and tested as membrane anchored binders to target transduction of eLVs to monocytes. This example describes the selection of the CD14 VHH for optimal transduction of primary monocytes by eLVs (containing Vpx).

As shown in Fig. 36A. several of the newly generated VHHs (SEQ ID NO 66 to 76, 101, 102, 104, 106 to 117) targeted transduction of G1x eLVs specifically to CD14 expressing cells (Jurkat stably expressing CD14). For this early stage of the screening process, eLVs were produced from e293T cells and the VHH binder was encoded in the transfer vector. Thus, transduction efficiency of the G1x CD14 VHH eLVs produced with this method was assessed by expression of the VHH in the transduced cell population.

A subset of the most specific CD14 VHH (SEQ ID NO 70, 72, 76, 107, 111) were selected from the results in Fig.35A and G1x CD14 VHH eLVs were produced from e293T cells stably expressing to test for transduction efficiency on primary monocytes in culture. As shown in Fig. 35B, G1x CD14 VHH eLVs effectively transduced monocytes, G1x eLVs targeted with the 3RMB1 VHH (SEQ ID NO 76) achieving the highest level of transduction, and transduction was proportional to the number of vector particles per cell.

The 3RMB1 VHH was selected for further optimisation by in vitro maturation. As shown in Fig 36C, transduction efficiency of matured 3RMB1 (SEQ ID NO 77 to 100) targeted G1x eLVs varied compared to the original 3RMB1 VHH targeted vector, with the matured 3RMB1-13 VHH (SEQ ID NO 94) targeted G1x eLV achieving the highest level of transduction.

Specificity of G1x eLVs targeted with 3RMB1 VHH was assessed in additional cell types. As shown in Fig. 37, 3RMB1 VHH G1x eLV effectively transduced CD14+ cells (monocytes/macrophages and Jurkat CD14 cell line) but not CD14 negative cells (stimulated PBMCs, Jurkat or NCI-H929 cell lines). Importantly, there was no detectable transduction of non-targeted (3RMB1 VHH deficient) G1x eLV, underscoring the key function of the targeting VHH in promoting specific and effective transduction of target cells.

Together these results demonstrate that G1x eLVs (incorporating Vpx) can be effectively and specifically targeted to CD14+ cells (including primary monocytes) using membrane anchored CD14 VHHs.

### Examples 32: Specificity and potency of monocyte/macrophage cell synthetic promoters driven by specific combinations of core, proximal and enhancer sequences.

This example describes the specificity and potency of 11 of the synthetic myeloid specific promoters assembled as described in Example 23 (SEQ ID NO 130 to 140 and SEQ ID 197). For this purpose, multiple candidate synthetic promoters were tested for specify (using a flow cytometry readout of GFP expression in specific cell types) and potency (using a luminescence readout from the expression of the luciferase reporter) in a range of cell types, using the EF1a ubiquitous promoter as comparator. The panel of cell types was selected based on the predicted vector exposure after systemic administration and include lymphocytes (SupT-1cell line and human PBMC) as well as non-T lymphocytes from the myeloid (THP1) and B cell (NCI-H929) compartment and originating from liver (Huh7) and kidney (293T). Fig. 38-40 present the results obtained on 7 candidates.

VSVG eLVs expressing the reporter construct under the control of EF1a or the different core promoters or full synthetic promoters, were generated in e293T producer cell line by transient transfection as previously described. Primary cells and cell lines were transduced with the purified vectors and expression level of the reporter eGFP transgene was evaluated in transduced cells.

6 proximal promoters derived from macrophage-specific genes were selected for testing. As shown in Fig. 38, CCL13, CCL18, CHIT1, HLA-DRA, LYZ, MRC1 (SEQ ID NO 130, 131, 132, 133, 134 and 197) proximal promoters induce higher expression eGFP in primary monocytes compared to EF1a promoter driven reporter vector (excluding MRC1) and ~5-20x lower expression in cell lines and activated PBMCs (predominantly T cells). These data indicate that HLA-DRA, LYZ, CHIT1, CCL8 and CCL13 induce reporter expression is highly enriched in primary myeloid cells compared to expression under the control of EF1a.

The CHIT1 sequence was used as the core for the myeloid specific synthetic promoter tested in Fig. 39 and assembled using enhancer sequences isolated from myeloid specific genes (SEQ ID NO 135 to 140). As shown in Fig. 39, addition of the enhancer sequences to the CHIT1 core promoter significantly increases the expression of the eGFP reporter compared to the proximal promoter alone or EF1a, while preserving the specificity to primary monocyte/macrophages.

Confirmation that the myeloid synthetic promoters tested in Fig. 39 induce functional levels of expression of transgenes other than the eGFP reporter in monocyte/macrophages, we tested secretion of IL12 from macrophages transduced with VSVG LV vectors expressing human IL12 under the control of the EF1a promoters or the myeloid specific synthetic promoter Enh31 CHIT1 (SEQ ID NO 139). As shown in Fig. 40, Levels of IL12 secretion from Enh31 CHIT1 transduced macrophages (M0 or M2) was equivalent or higher compared to EF1a and significantly higher compared to macrophages transduced with IL12 under the control of the core CHIT1 promoter only.

Altogether, these results demonstrate that purpose-built monocyte/macrophage cell specific synthetic promoters with an optimized size for inclusion into lentiviral constructs effectively restrict the expression of reporter transgenes to monocyte/macrophages in culture.

### Example 33: Optimising the structure of the VHH envelope anchoring scaffold increases transduction efficiency of CD8a and CD3e VHH targeted vectors

In these examples different hinge regions and transmembrane domains were used to determine the optimal envelope anchoring scaffold for the CD3e VHH (SEQ ID NO. 3) or CD8 VHH (SEQ ID NO 6) to achieve high-level transduction of target cells.

VHHCD3e eLV and VHHCD8a eLV vectors expressing were produced by transient transfection of e293T cells with packaging plasmids encoding the Gag/Pol, Rev and LVGP1x constructs, as well as the transfer vector for the expression of the VHH-targeting constructs. Each VHH was embedded in a viral envelope anchoring construct composed of a transmembrane domain (PDGFR or B7-1; SEQ ID NO. 27 and SEQ ID NO. 28, respectively) and fused to different linker region of varying flexibility, length and multimerization potential (see SEQ ID NO. 21-26). After harvest, Jurkat WT cells were transduced with the same volume of lentiviral preparation. Functional titre of each vector was assessed by anti-VHH staining of transduced cells by flow cytometry 3 days after transduction and expressed as transducing units per millilitre.

As shown in Fig. 41 and Fig. 42, the transmembrane domain variants PDGFR or B7-1 did not greatly impact the degree of targeting with the CD3e or CD8a VHHs, with PDGFR transmembrane performing slightly better. The hinge domain had a greater degree of impact. The two hinge regions that performed best in both LVGP1X-VHHCD3e eLV and LVGP1X-VHHCD8a eLV were the PDGFR short stalk and CD8a hinge, which have an intermediate degree of flexibility and less tendency to multimerize compared to short more rigid hinges (e.g. GAPGAS) or to multimerising hinges (e.g. Tetramer Coil Coil).

### Example 34: Optimising the structure of the VHH envelope anchoring scaffold increases transduction efficiency of TCR VHH targeted vectors

In this example, different hinge regions and transmembrane domains were used to determine the optimal envelope anchoring scaffold for the TCR VHH (SEQ ID NO. 9) to achieve high- level transduction of target cells.

VHHTCR eLV vectors expressing dsGFP were produced as previously described from e293T cells stably expressing a membrane anchored TCR VHH anchored to the viral envelope through 4 different scaffolds of varying flexibility, length and multimerization potential: TCR1: PDGFR transmembrane domain and the membrane proximal region of PDGFR (SEQ ID NO. 44); TCR2: PDGFR transmembrane domain and the membrane proximal region of IgG4 (SEQ ID NO. 34); TCR3: PDGFR transmembrane domain and a CD8a stalk (SEQ ID NO. 45), TCR4: PDGFR transmembrane domain and a Tetramer coiled coil hinge (SEQ ID NO. 46). Of note, a VSVG mutant (K47A, R354; SEQ ID NO. 47), previously shown to abrogate the broad tropism of wild-type VSVG, was used to pseudotype the VHHTCR eLVs in this study. After concentration, Jurkat WT cells were transduced with the same volume of lentiviral preparation and transduction efficiency (GFP+ cells) was assessed at day 3 by flow cytometry.

As shown in Fig. 43A, different hinge sequences within the TCR VHH scaffold impacted the transduction efficiency. In the case of the TCR VHH, the anchoring scaffold containing the PDGFR transmembrane domain and membrane proximal region of IgG4 provided the architecture which achieved the highest transduction efficiency in association with the VSVG (K47A, R354) pseudotype.

The architecture of VHH scaffold was further optimized for the TCRab VHH when targeting eLV vectors were pseudotyped with G1x. TCRab VHH G1x eLVs were produced by transient transfection from e293T cells stably expressing TCRab VHH in the PDGFR transmembrane domain and the membrane proximal region of IgG4 scaffold (SEQ ID NO. 34) or a scaffold composed of the PDGFR transmembrane domain and the membrane proximal region of CD8a s (SEQ ID NO. 52). Primary human PBMCs exposed to the same volume of unconcentrated vectors were characterised for T cell activation expansion and transduction. As shown in Fig. 43B, TCRab VHH G1x eLVs using the PDGFR/CD8a hinge scaffold to present the VHH compared to the IgG4 hinge led to total transduced T cells at day 7 post-transduction.

### Example 35: Targeting LVGP1x or G1x eLV to T cells using a TCRab VHH binders induces activation, expansion, and enhanced transduction of resting T cells

Upon binding to their cognate antigen, TCR complexes aggregate and trigger a signalling cascade resulting in T-cell activation. In this example we demonstrate that TCRab VHH binders on the surface of eLVs, in different scaffolds, induce T cells activation, expansion and increased transduction compared to VSVG LV.

LVGP1x-TCRabVHH (SEQ ID NO. 34) expressing dsGFP were generated as previously described. Resting human PBMCs were transduced with 10ul of the LVGP1x-VHHTCR eLVs in presence of low concentration rhIL-2 (30ng/ml) and T cell activation was assessed 24h post-transduction by staining with anti-CD25 or anti-CD69 and CD3. As shown in Fig. 44, LVGP1x-TCRab VHH eLV induced significant expression of both CD25 in resting T cells compared to media alone, VSVG LV and LVGP1x-CD8a VHH eLV. Correlating with increased T cell activation, LVGP1x-TCRab VHH successfully transduced resting CD3+ T cells, as measured by GFP expression, and more effectively than a VSVG GFP eLV at Day 19 (Fig 44C). Activation and transduction of resting T cells by LVGP1x-TCRab VHH eLVs also correlated with significant expansion of the GFP expressing cells over 20 days, compared to VSVG GFP transduced cells (Fig. 44B).

Activation and expansion of resting T cells observed using TCRab VHH eLV was also modulated by the VHH scaffold in G1x pseudotyped LVs. As shown in Fig. 45, TCRab VHH G1x eLVs using the PDGFR/CD8a hinge scaffold (SEQ ID NO 52) to present the VHH compared to the IgG4 hinge (SEQ ID NO 34) lead to higher CD69, CD25 and PD1 expression, as well as increased cell expansion and functional viral titres.

Similarly, T cell activation was observed with alternative TCRab VHH G1x eLVs displaying alternative TCRab VHH binders (SEQ ID NO 54 to 65) optimized by in vitro maturation on the original TCRab VHH (SEQ ID NO 9). As shown in Fig. 46A, T cell activation (assessed by increased expression of CD69) was induced in Jurkat cells by exposure of multiple different TCRab VHH G1x eLVs, each with a similar transduction efficiency (Fig 46B.).

In conclusion, LVGP1x or G1x pseudotyped eLVs targeted to T cells using a TCR specific VHH in different scaffolds induce activation and effective transduction of resting lymphocytes resulting in significant expansion of the transduced cells.

### Example 36: Displaying co-stimulatory ligands on the surface of VHH targeted eLVs increases activation expansion and transduction of resting T cells

Exposure of resting T lymphocytes to CD8aVHH eLV induces low levels of T cell activation (indicated by CD25 expression) and transduction (indicated by expression of the V5 tagged BCMA CAR transgene) and minimal T cell expansion (Fig. 47A-C). To test whether display of co-stimulatory ligands on the surface of CD8a VHH eLV vectors increased T cell activation/expansion and transduction, CD8a VHH G1x eLVs were produced as described previously from CD8a VHH e293T cell lines displaying the OKT3 (CD3 agonistic antibody; SEQ ID NO 29) alone or in combination with CD28 co-stimulatory receptor activators, namely CD80 or a CD28 VHH (SEQ ID NO. 198 and 199, respectively, and SEQ ID NO. 200 for the scaffold of the CD8 scFV). Resting human PBMCs were transduced with CD8a VHH G1x eLV, CD8a VHH/OKT3 G1x eLV, CD8a VHH/OKT3/CD80 G1x eLV or CD8a VHH/OKT3/CD28 scFv G1x eLV in the presence of low levels of rhIL-2 (30ng/ml) and T cell activation, expansion and transduction was by flow cytometry. As shown in Fig. 47 A-C, CD8a VHH/OKT3/ G1x eLV, CD8a VHH/OKT3/CD80 G1x eLV or CD8a VHH/OKT3/CD28 scFv G1x eLV induced a significant increase in T cell activation (CD25), T cell expansion (>10 fold compared to CD8a VHH G1x eLV.

Similar testing was performed with TCRab VHH G1x eLV vectors, which already induce T cell activation and expansion, to determine whether co-stimulatory ligands further increase T cell activation. As shown in Fig. 47 D-F, TCRab VHH/ displaying the CD3 agonistic binder OKT3 (TCRab VHH/OKT3scFv G1x eLV) or the CD28 ligand CD80 (TCRab VHH/CD80 G1x eLV) or the CD28 agonistic VHH (TCRab VHH/CD28scFv G1x eLV) increased activation and transduction of resting T cells, with a minimal increase in the level of T cell expansion. As shown in Fig. 48 TCRab VHH G1x eLV displaying CD28 costimulatory molecules CD86 IgV or TGN1412, or the 4-1BB costimulatory molecule 4B1-4 (SEQ ID NO. 50, 51 and 201, respectively), induced increased IL2 production and CD69 and CD25 expression, indicating increased T cell activation, which was associated with increased expansion and transduction of resting PBMCs compared to TCRab VHH G1x eLV vectors.

### Example 37: Transduction of primary T lymphocytes with CD8a or TCR VHH targeted eLV encoding an engineered TCR results in specific, persistent, and functional expression of the transgene, as demonstrated by the cytotoxicity of the TCR engineered cells.

Adoptive T-cell therapy using T-cell receptor-engineered T cells (TCR-T cells) has shown promising results in the treatment of certain types of cancers and in vivo reprogramming of TCR-T cells using VHH directed eLVs would overcome the challenges associated with the current ex vivo manufactured TCRT-T products. To assess the capacity of VHH directed eLVs to deliver an engineered TCR construct, we tested CD8a and the TCR VHH targeted LVGP1x eLVs encoding a clinically validated and widely used engineered TCR (NY-ESO) on human PBMCs in culture.

LVGP1X-VHHCD8a and LVGP1X-VHHTCR encoding a TCR transgene against the HLA-A*02:01 bound NY-ESO-1157-165 peptide (i.e. 1G4-LY, TCR alpha chain SEQ ID NO. 30 and TCR beta chain SEQ ID NO. 31) were generated from stably expressing e293T producer cell as previously described. Human PBMCs (activated with anti-CD3/CD28) were transduced with 10ul of the concentrated eLVs and transduction efficiency in CD3+ T cells were assessed at day 3, 7, 14 and 21 by flow cytometry with a PE coupled A2/NY-ESO-1 multimer. On day 17 post infection, transduced lymphocytes were counted and co-cultured at a 1 to 1 ratio with lymphoblastic BFP positive HLA-A*02:01 positive T2 cells pulsed with 1µg/ml of a control HIV peptide (HIV476-484 amino acids sequence: ILKEPVHGV) or the NY-ESO-1157-165 peptide (SLLMWITQC). After 72 hours the cells were collected and analysed by flow cytometry for viability and count.

As shown in Fig. 49A and B., transduction of activated hPBMCs with CD8a or TCR VHH targeted LVGP1x eLVs coding for NY-ESO resulted in specific and stable transduction of CD8+ T cells or CD3 T cells respectively. As previously demonstrated for CAR expressing eLVs, restricting the expression of the NY-ESO1 transgene during viral production using a T cell synthetic promoter (SYN21; SEQ ID NO. 12) significantly increases functional titre of the vectors compared to EF1a driven transgene expression. Transduction efficiency of the EF1a NY-ESO constructs is approximately 1 log lower compared to SYN NY-ESO constructs.

After 72h, T2 cells pulsed with the control HIV peptide were recovered from co-culture with either untransduced (UT) or NY-ESO-1 transduced cells (Fig. 50B). However, T2 cells pulsed with the NY-ESO-1 peptide cells were recovered only in the UT group, and to some extent in the EF1a promoter group (where there was less than 5% of total CD3+ T cells expressing NY-ESO TCR). No target NY-ESO pulsed T2 cells were recovered from the co-cultures with LVGP1x-VHHTCR SYN NY-ESO transduced cells (Fig. 50A). This result demonstrates that T cells transduced with VHH targeted eLVs coding for an engineered TCR construct are functional and highly cytotoxic to target cells.

### Example 38: TCRab VHH G1x eLV specifically deliver BCMA CAR constructs to T cell in vivo and generate functional BCMA CAR T cells which eliminate a systemically engrafted multiple myeloma cell line in a humanized mouse model.

This example describes in vivo transduction efficiency of TCRab VHH G1X eLV in humanized mice, resulting in the in vivo production of functional BCMA CAR-T cells and the elimination of the target cancer cell population.

5e6 NCI-H929 cells were injected i.v. and allowed to engraft for 7 days prior to i.p. injection of activated human PBMCs and one day later i.p. injection of TCRab VHH G1X eLV encoding for a BCMA CAR construct under the control of the T cell specific synthetic promoter SYN23 vectors in NSG MHCl/II KO mice. In vivo reprogrammed BCMA CAR T cells were analysed weekly in peripheral blood and in the bone marrow at euthanasia on day 42 post vector infusion. Anti-tumor activity of BCMA CAR-T cells was assessed weekly by bioluminescence imaging of the luciferase expressing NCH-H929 cells, body weight and clinical observations.

As shown in Fig. 51A, intraperitoneal injection of ENaBL-GTT vectors was well tolerated with no adverse events observed during the course of the study. While tumor growth was exponential starting from day 8 in animals injected with the control TCRab VHH G1X eLV BFP vector, TCRab VHH G1X eLV BCMA CAR injection resulted in elimination of the tumour cells and prevented re-growth until the end of the study at day 42. BCMA CAR T cells were detected only in the TCRab VHH G1X eLV BCMA CAR injected animals (7.9%±1.2 % CD3+CAR+ cells; Fig. 51B). Persistence of BCMA CAR T cells was observed throughout the study.

This study demonstrates that TCRab VHH G1x eLV BCMA CAR vector reprograms BCMA CAR T cells in vivo which results in complete elimination of a systemically engrafted multiple myeloma cell line (NCI-H929) in a hPBMC humanized mouse.

### Example 39: TCRab VHH G1x eLV specifically deliver BCMA CAR constructs to T cell in vivo and generate functional BCMA CAR T cells which eliminate a systemically engrafted multiple myeloma cell line in a humanized mouse model.

This example describes *in vivo* T cell transduction specificity and efficacy of TCRab VHH G1X eLV in stably humanized mice bearing systemic multiple myeloma tumors, and the resulting in the in vivo production of functional BCMA CAR-T cells and the elimination of the target cancer cell population.

TCRab VHH G1X eLV vectors encoding a BCMA CAR transgene (or a BFP reporter) under the control of the T cell specific synthetic promoter SYN23 were administered in single i.v. injections in hCD34+ NCG mice bearing multiple myeloma tumors from the injection of 5e6 NCI-H929 stably expressing luciferase. Anti-tumor efficacy was evaluated by monitoring NCI-H929 cell growth (in vivo bioluminescence imaging), animal body weight, clinical state, survival. In addition, transduction kinetics of TCRab VHH G1X eLV BCMA CAR and the cellular kinetics of the transduced cells were analyzed by flow cytometry on peripheral blood. Flow cytometry of spleen, bone marrow and tumor were also performed to identify the presence and state of CAR T cells in these tissues.

Whereas tumor growth was not controlled in animals that received TCRab VHH G1X eLV BFP or vehicle, mice injected with TCRab VHH G1X eLV BCMA CAR eradicated the tumor in 2/8, 8/8 and 7/8 mice in low, medium, and high dose groups respectively by Day 35 post tumor injection (Fig. 52). As shown in Fig. 53, transduction of TCRab VHH G1X eLV BCMA CAR was specific to human T cells (both CD4 and CD8 lymphocytes) in peripheral blood (Fig. 53A) and bone marrow (Fig. 53B), with less than 0.5% of hCD14, hCD20, hCD56 and mouse CD45 cells expressing CAR levels above background.

This study demonstrates (1) the effective anti-tumor activity of TCRab VHH G1X eLV BCMA CAR reprogrammed T cells in vivo and (2) the specificity TCRab VHH G1X eLV vectors for T lymphocytes in vivo.

### Example 40: Combination of T lymphocyte and monocyte reprogramming synergizes to eliminate solid tumors in humanized mice.

This example describes *in vivo* administration of the T cell specific TCRab VHH G1x eLV BCMA CAR vector in combination with the monocyte specific CD14 VHH G1x eLV expressing human IL12 and the synergistic anti-tumor activity of this vector combination on NCI-H929 cells implanted subcutaneously to simulate a solid tumor.

hCD34+ NCG mice bearing multiple myeloma tumors in a solid tumor form from the subcutaneous injection of 5e6 NCI-H929 were injected intravenously with (1) the monocyte specific CD14 VHH G1x eLV vector expressing human IL12 or a GFP reporter under the control of the constitutive EF1a promoter; or (2) TCRab VHH G1X eLV vectors encoding a BCMA CAR transgene under the control of the T cell specific synthetic promoter 23; or (3) the same dose of the CD14 VHH G1x eLV hIL12 and TCRab VHH G1X eLV BCMA CAR vectors in a single combined injection. Anti-tumor efficacy was evaluated by monitoring NCI-H929 cell growth (calliper measurement of the subcutaneous solid tumor mass) and animal survival. In addition, the levels of BCMA CAR-T cells and human IL12 in peripheral blood transduced cells were analyzed by flow cytometry.

As shown in Fig. 54, whereas tumor growth was not controlled or poorly controlled in animals injected with the TCRab VHH G1x eLV BCMA CAR, CD14 VHH G1x eLV hIL12, CD14 VHH G1x eLV GFP vectors alone, combining TCRab VHH G1x eLV BCMA CAR and CD14 VHH G1x eLV hIL12 in a single dose eliminated the NCI-H929 tumor cells. As shown in Fig. 55A, transduction of T lymphocytes was observed only in the TCRab VHH G1X eLV BCMA CAR and combination TCRab VHH G1x eLV BCMA CAR and CD14 VHH G1x eLV hIL12 group, with a significant increase in the percentage of BCMA CAR-T cells in the combination group. As shown in Fig. 55B, human IL12 was detected only in animals injected with CD14 VHH G1x eLV hIL12. IL12 levels in circulation were still ≥40000pg/ml at the end of the study (data not shown). The effect of the combination treatment was notable in the level of T cell infiltration within the tumor. As shown in Fig. 55B, combining reprogramming of T cells with a BCMA CAR and of monocytes with secretable IL12 induced a significant increase in T cell tumor infiltration.

This study demonstrates (1) intravenous administration of CD14 VHH G1x eLV hIL12 results in the persistent secretion of high levels of hIL12, suggesting persistence and stable transduction of monocyte/macrophages using CD14 VHH G1x eLV hIL12 and (2) in vivo reprogramming of multiple cell types can be achieved within the same organism with a single intravenous injection of our VHH targeted vectors and (3) in vivo reprogramming of multiple cell types with enhanced anti-tumor activity synergizes to eliminate tumors which are otherwise resistant to a single engineered cell type.

### Example 41: Displaying regulators of complement activity on the viral envelope of VHH targeted eLVs decreases complement mediated inactivation of the vector particles.

Transduction efficiency of systemically injected lentiviral vectors is limited by host viral inactivation mechanisms, including complement mediated inactivation. This example describes the engineering TCRab VHH G1x eLV vector envelope to display exogenous inhibitors or resistance factors that decrease inactivation of the viral vectors by the complement.

TCRab VHH targeted G1x eLVs displaying regulators of Complement activity (RCA) were produced as previously described using transient transfection of e293T producer cells stably expressing TCRab VHH and either of the following RCAs: (1) DAF (CD55) or (2) factor H, both RCAs were tethered to the envelope through fusion to a GPI anchor or the transmembrane domain of VSVG (SEQ ID NO 183 to 186; Fig. 56A).

Complement mediated inactivation of the vector particles was assessed by for one hour with (1) serum-free (complement-free) medium or (2) medium supplemented with fresh human serum (active complement) or (3) medium complement with heat-inactivated human serum (inactivated complement). Jurkat cells were then incubated for 2 hours with the lentiviral preparations and transduction efficiency of the vectors was assessed 72hrs later to quantify the percentage functional titre recovery of viral particles incubated in the presence of serum. As shown in Fig. 56B, DAF-GPI and factor H (GPI or GS) displaying TCRab VHH G1x eLV were partly protected against complement-mediated inactivation with up to 24,1% and 16,3% increased mean recovery, respectively. This result shows the protective effect of the RCA on vector complement mediated inactivation.

### Example 42: Displaying an Albumin binding domain on the viral envelope of VHH targeted eLVs decreases complement mediated inactivation of the vector particles.

Albumin binding domains have been used previously to extend the pharmacokinetics of therapeutic agents injected intravenously and reduce phagocytosis. This example describes the engineering of TCRab VHH G1x eLV vector envelope to display an albumin binding domain to reduce vector phagocytosis.

TCRab VHH targeted G1x eLVs displaying the albumin binding domain for protein G of Streptococcus strain G148 (ABD) were produced as previously described using transient transfection of e293T producer cells stably expressing TCRab VHH and ABD tethered to the envelope through fusion to a GPI anchor or the transmembrane domain of VSVG (SEQ ID NO 192 and 191, respectively).

Phagocytosis of the vector particles was assessed by incubating the vector preparations with fresh human monocyte derived macrophages for 2 hours and quantifying vector insertion 3 days post exposure. The inhibitor of phagocytosis Chlorpromazine was used as a negative control. As shown in Fig. 57, TCRab VHH G1x eLV vectors are phagocytosed by primary macrophages, resulting in vector integration into the host cell genomic DNA which is inhibited by Chloropromazine. Displaying ABD on the envelope of envelope of TCRab G1x eLVs decreased phagocytosis of the lentiviral articles, resulting in a significant decrease in the number of viral genomes per macrophages. This result shows the protective effect of the ABD on vector phagocytosis.

### Example 43: Synthetic promoters assembled using enhancers of genes expressed preferentially in tumor associated macrophages compared to tissue resident macrophages, are inducible by signals specific to tumor microenvironments.

This example describes the expression of a reporter transgene from synthetic promoters inducible by signals associated with the tumor microenvironment. 12 tumor associated macrophage (TAM) specific promoters were assembled as described in Example 23 using enhancers from genes expressed preferentially in TAMs (see SEQ ID NO 141-152).

VSVG eLVs expressing the GFP/luciferase reporter construct under the control of EF1a or the different synthetic promoter, were generated in e293T producer cell line by transient transfection as previously described. Primary human monocytes were transduced with the different VSVG eLVs and cultured for 7 days followed by culture for 2 days in media supplemented with TME associated signals: (1) IL4, IL10 and TGFb; or (2) CoCl2 (to induce hypoxic signaling); or (3) conditioned media from gastric cancer cell line NUGC4. After 2 days of culture, the level of reporter (GFP) expression was assessed by flow cytometry.

As shown in Fig 58, whereas expression of the reporter from the EF1a promoter decreases when the cells are exposed to hypoxia or conditioned media, expression driven by TAM synthetic promoters is increased, in particular for the TAM 12 promoter.

Altogether, these results demonstrate that purpose-built TAM synthetic promoters with an optimized size for inclusion into lentiviral constructs are inducible by signals associated with the TME, thus providing a strategy to restrict expression of transgenes to the tumor microenvironment.

## Claims

1. A retroviral vector having a lipid bilayer envelope comprising:
(a) a single-domain antibody (sdAb) binding domain displayed on the exterior of the envelope that is cell-type specific;
(b) a Lassa virus envelope protein displayed on the exterior of the envelope that is able to facilitate infection of the same cell type as in (a); and
(c) a nucleic acid molecule comprising a promoter expressible in the same cell type as in (a).

2. A retroviral vector according to claim 1, wherein the promoter is cell-type specific.

3. A retroviral vector according to claim 1 or claim 2, wherein the sdAb binding domain and the promoter are specific for use in immune cells, e.g., T cells, NK cells or macrophages.

4. A retroviral vector according to any preceding claim, wherein the retroviral vector is a lentiviral vector.

5. A retroviral vector according to any preceding claim, wherein retroviral envelope is substantially free from MHC class I or is devoid of MHC class I.

6. A retroviral vector according to any preceding claim, wherein the retroviral vector displays a high level of CD47, an agonistic CD3 scFv, 41BBL and/or CD80 on the surface of the envelope.

7. A retroviral vector according to any preceding claim, wherein the sdAb binding domain is specific for CD3ε, CD8, or the T cell receptor (TCR).

8. A retroviral vector according to any of claims 1-6, wherein the sdAb binding domain is specific for CD14, CD16, CCR1, CCR2, CXCR4, or CD64.

9. A retroviral vector according to any preceding claim, wherein the nucleic acid molecule encodes a transgene, preferably for a chimeric antigen receptor (CAR).

10. A retroviral vector according to any preceding claim for use in diagnosis, prevention, or treatment of disease.

11. A method of making the retroviral vector of any of claims 1 to 9, comprising the steps of:
(a) engineering a cell to express a membrane-anchored sdAb binding domain that is cell-type specific;
(b) engineering the cell to express a Lassa virus envelope protein that is able to facilitate infection of the same cell type as in (a);
(c) replicating in the cell a nucleic acid vector comprising a promoter expressible the same cell type as in (a); and
(d) harvesting the retroviral vector in the form of vesicles from the cell.

12. A method according to claim 11, wherein the cell is a 293T-derived cell line.

13. A method according to claim 11 or 12, wherein cell has been modified to express CD47 and to knock down MHC class I expression.

14. A retroviral vector according to any of claims 1 to 9, a retroviral vector for use according to claim 10, or a method according to any of claims 11 to 13, wherein the Lassa virus envelope protein comprises a tyrosine to alanine substitution at amino acid position 150, i.e., mutation Y150A.

15. An ex vivo method of modifying a cell, comprising exposing the cell to a retroviral vector according to any of claims 1 to 9 or 14.

## Patentansprüche

1. Retroviraler Vektor, der eine Lipiddoppelschichthülle aufweist, umfassend:
(a) eine Einzeldomänenantikörper(sdAb-)Bindungsdomäne, die auf der Außenseite der Hülle angezeigt ist, die zelltypspezifisch ist;
(b) ein Lassa-Virus-Hüllprotein, das auf der Außenseite der Hülle angezeigt ist, das in der Lage ist, Infektion des gleichen Zelltyps wie in (a) zu erleichtern; und
(c) ein Nukleinsäuremolekül, das einen Promotor umfasst, der in dem gleichen Zelltyp wie in (a) exprimierbar ist.

2. Retroviraler Vektor nach Anspruch 1, wobei der Promotor zelltypspezifisch ist.

3. Retroviraler Vektor nach Anspruch 1 oder Anspruch 2, wobei die sdAb-Bindungsdomäne und der Promotor spezifisch für Verwendung in Immunzellen, z. B. T-Zellen, NK-Zellen oder Makrophagen sind.

4. Retroviraler Vektor nach einem vorhergehenden Anspruch, wobei der retrovirale Vektor ein lentiviraler Vektor ist.

5. Retroviraler Vektor nach einem vorhergehenden Anspruch, wobei retrovirale Hülle im Wesentlichen frei von MHC-Klasse I ist oder ohne MHC-Klasse I ist.

6. Retroviraler Vektor nach einem vorhergehenden Anspruch, wobei der retrovirale Vektor ein hohes Maß an CD47, einem agonistischen CD3 scFv, 41BBL und/oder CD80 auf der Oberfläche der Hülle anzeigt.

7. Retroviraler Vektor nach einem vorhergehenden Anspruch, wobei die sdAb-Bindungsdomäne spezifisch für CD3ε, CD8 oder den T-Zellrezeptor (TCR) ist.

8. Retroviraler Vektor nach einem der Ansprüche 1-6, wobei die sdAb-Bindungsdomäne spezifisch für CD14, CD16, CCR1, CCR2, CXCR4 oder CD64 ist.

9. Retroviraler Vektor nach einem vorhergehenden Anspruch, wobei das Nukleinsäuremolekül ein Transgen kodiert, bevorzugt für einen chimären Antigenrezeptor (CAR).

10. Retroviraler Vektor nach einem vorhergehenden Anspruch zur Verwendung bei Diagnose, Prävention oder Behandlung von Krankheit.

11. Verfahren zur Herstellung des retroviralen Vektors nach einem der Ansprüche 1 bis 9, umfassend die folgenden Schritte:
(a) Entwickeln einer Zelle, um eine membranverankerte sdAb-Bindungsdomäne zu exprimieren, die zelltypspezifisch ist;
(b) Entwickeln der Zelle, um ein Lassa-Virus-Hüllprotein zu exprimieren, das in der Lage ist, Infektion des gleichen Zelltyps wie in (a) zu erleichtern;
(c) Replizieren, in der Zelle, eines Nukleinsäurevektors, der einen Promotor umfasst, der den gleichen Zelltyp wie in (a) exprimierbar ist; und
(d) Ernten des retroviralen Vektors in der Form von Vesikeln aus der Zelle.

12. Verfahren nach Anspruch 11, wobei die Zelle eine von 293T abgeleitete Zelllinie ist.

13. Verfahren nach Anspruch 11 oder 12, wobei die Zelle modifiziert worden ist, um CD47 zu exprimieren und um MHC-Klasse-I-Expression abzubauen.

14. Retroviraler Vektor nach einem der Ansprüche 1 bis 9, retroviraler Vektor zur Verwendung nach Anspruch 10 oder Verfahren nach einem der Ansprüche 11 bis 13, wobei das Lassa-Virus-Hüllprotein eine Tyrosin-zu-Alanin-Substitution an Aminosäureposition 150, d. h.Mutation Y150A, umfasst.

15. Ex-vivo-Verfahren zum Modifizieren einer Zelle, umfassend Exponieren der Zelle gegenüber einem retroviralen Vektor nach einem der Ansprüche 1 bis 9 oder 14.

## Revendications

1. Vecteur rétroviral possédant une enveloppe bicouche lipidique comprenant :
(a) un domaine de liaison d'anticorps à domaine unique (sdAb) affiché à l'extérieur de l'enveloppe qui est spécifique d'un type de cellule ;
(b) une protéine d'enveloppe du virus Lassa affichée à l'extérieur de l'enveloppe qui est capable de faciliter l'infection du même type de cellule qu'en (a) ; et
(c) une molécule d'acide nucléique comprenant un promoteur exprimable dans le même type de cellule qu'en (a).

2. Vecteur rétroviral selon la revendication 1, dans lequel le promoteur est spécifique d'un type de cellule.

3. Vecteur rétroviral selon la revendication 1 ou la revendication 2, dans lequel le domaine de liaison sdAb et le promoteur sont spécifiques pour une utilisation dans des cellules immunitaires, par exemple, des lymphocytes T, des cellules NK ou des macrophages.

4. Vecteur rétroviral selon une quelconque revendication précédente, dans lequel le vecteur rétroviral est un vecteur lentiviral.

5. Vecteur rétroviral selon une quelconque revendication précédente, dans lequel l'enveloppe rétrovirale est sensiblement exempte de CMH de classe I ou est dépourvue de CMH de classe I.

6. Vecteur rétroviral selon une quelconque revendication précédente, ledit vecteur rétroviral présentant un niveau élevé de CD47, un scFv CD3 agoniste, 41BBL et/ou CD80 sur la surface de l'enveloppe.

7. Vecteur rétroviral selon une quelconque revendication précédente, ledit domaine de liaison sdAb étant spécifique de CD3ε, de CD8 ou du récepteur des lymphocytes T (TCR).

8. Vecteur rétroviral selon l'une quelconque des revendications 1 à 6, dans lequel le domaine de liaison sdAb est spécifique de CD14, CD16, CCR1, CCR2, CXCR4 ou CD64.

9. Vecteur rétroviral selon une quelconque revendication précédente, dans lequel la molécule d'acide nucléique code pour un transgène, de préférence pour un récepteur d'antigène chimérique (CAR).

10. Vecteur rétroviral selon une quelconque revendication précédente destiné à être utilisé dans le diagnostic, la prévention ou le traitement d'une maladie.

11. Procédé de préparation du vecteur rétroviral de l'une quelconque des revendications 1 à 9, comprenant les étapes de :
(a) conception d'une cellule pour exprimer un domaine de liaison sdAb ancré à la membrane qui est spécifique d'un type de cellule ;
(b) conception de la cellule pour exprimer une protéine d'enveloppe du virus Lassa qui est capable de faciliter l'infection du même type de cellule qu'en (a) ;
(c) réplication dans la cellule d'un vecteur d'acide nucléique comprenant un promoteur exprimable dans le même type de cellule qu'en (a) ; et
(d) récolte du vecteur rétroviral sous forme de vésicules de la cellule.

12. Procédé selon la revendication 11, dans lequel la cellule est une lignée cellulaire dérivée de 293T.

13. Procédé selon l'une des revendications 11 ou 12, dans lequel la cellule a été modifiée pour exprimer CD47 et réduire l'expression de CMH de classe I.

14. Vecteur rétroviral selon l'une quelconque des revendications 1 à 9, vecteur rétroviral destiné à être utilisé selon la revendication 10, ou procédé selon l'une quelconque des revendications 11 à 13, ladite protéine d'enveloppe du virus Lassa comprenant une substitution de tyrosine par alanine à la position 150 d'acide aminé, c'est-à-dire la mutation Y150A.

15. Procédé ex vivo de modification d'une cellule, comprenant l'exposition de la cellule à un vecteur rétroviral selon l'une quelconque des revendications 1 à 9 ou 14.
